# EUROPEAN PATENT APPLICATION

(11) **EP 2 246 329 A1**
(43) Date of publication of application: **03.11.2010**
(21) Application number: 09382005.8
(22) Date of filing: 19.01.2009
(51) Int. Cl.: C07D 213/68, C07D 401/10, C07D 401/14, C07D 405/12, C07D 409/10, C07D 409/12, C07D 417/12, A61K 31/4412, A61K 31/443, A61K 31/4436, A61K 31/444, A61K 31/4427, A61P 33/06

(54) **4(1H)-pyridinone derivatives and their use as antimalaria agents**

(71) Applicant: Glaxo Group Limited, Greenford Middlesex UB6 0NN (GB)
(72) Inventor: The designation of the inventor has not yet been filed
(74) Representative: Elzaburu Marquez, Alberto

(57) **Abstract**

4-pyridone (4-pyridinone) derivatives of Formula I 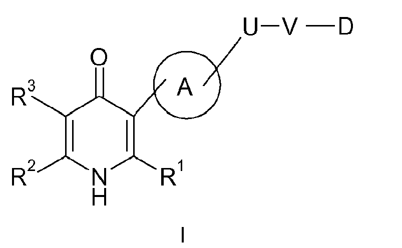 and pharmaceutically acceptable derivatives thereof, processes for their preparation, pharmaceutical formulations thereof and their use in chemotherapy of certain parasitic infections such as malaria, are provided.

## Description

### FIELD OF THE INVENTION

The invention relates to heterocyclic compounds and their use in chemotherapy. More specifically, this invention is concerned with certain 4-pyridone (4-pyridinone) derivatives, processes for their preparation, pharmaceutical formulations thereof and their use in chemotherapy of certain parasitic infections such as malaria, and in particular infection by *Plasmodium falciparum.*

### BACKGROUND OF THE INVENTION

Parasitic protozoal infections are responsible for a wide variety of diseases of medical and veterinary importance, including malaria in man and various coccidioses in birds, fish and mammals. Many of the diseases are life-threatening to the host and cause considerable economic loss in animal husbandry, such as species of *Eimeria, Theileria, Babesia, Cryptosporidium, Toxoplasma* (such as *Toxoplasma brucei,* African sleeping sickness and *Toxoplasma cruzi,* Chagas disease) and *Plasmodium* (such as *Plasmodium falciparum),* and the Mastigophora such as species of *Leishmania* (such as *Leishmania donovani*). Another parasitic organism of increasing concern is *Pneumocytis carinii,* which can cause an often fatal pneumonia in immunodeficient or immunocompromised hosts, including those infected with HIV.

Malaria is one of the major disease problems of the developing world. The most virulent malaria-causing parasite in humans is the parasite *Plasmodium falciparum,* which is the cause of hundreds of millions of cases of malaria per annum, and is thought to cause over 1 million deaths each year, Breman, J. G., et al., (2001) Am. Trop. Med. Hyg. 64, 1-11. One problem encountered in the treatment of malaria is the build-up of resistance by the parasite to available drugs. Thus, there is a need to develop new antimalarial drugs.

A group of 3,5-dihalo-2,6-dialkyl-4-pyridinol derivatives (the tautomeric form of 4-pyridones) is described in US patent No. 3,206,358 as having anticoccidial activity.

European Patent Publication EP123239 discloses combinations of the aforementioned 4-pyridinol derivatives with antiprotozoal naphthoquinones, e.g. antimalarial naphthoquinones, in a potentiating ratio.

PCT Patent Publication WO 91/13873 A1 discloses a class of 4-pyridone derivatives which exhibit activity against protozoa, in particular against the malarial parasite *Plasmodium falciparum,* and species of *Eimeria* as well as the parasitic organism *Pneumocytis carinii.*

PCT Patent Publication WO 2006/094799 A2 discloses certain 4-pyridone (4-pyridinone) derivatives and their use in chemotherapy of certain parasitic infections such as malaria, and in particular infection by *Plasmodium falciparum.*

PCT Patent Application No. PCT/EP2007/055188 discloses certain 4-pyridone (4-pyridinone) derivatives and their use in chemotherapy of certain parasitic infections such as malaria, and in particular infection by *Plasmodium falciparum.*

### SUMMARY OF THE INVENTION

This invention is directed to certain 4-pyridone (4-pyridinone) derivatives, processes for their preparation, pharmaceutical compositions comprising such compounds and use of the compounds in the chemotherapy of certain parasitic infections such as malaria, and in particular infection by *Plasmodium falciparum.*

### DETAILED DESCRIPTION OF THE INVENTION

The present invention provides a compound of Formula I: 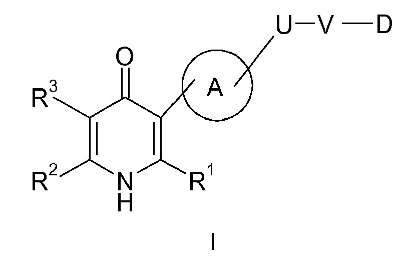 wherein:
R¹ and R² independently represent C₁₋₄alkyl or C₁₋₄alkylOH;
R³ represents halo;
A represents phenyl; pyridinyl; pyrimidinyl; or benzothienyl; wherein A is optionally substituted with halo;
U represents a bond; -C₂alkynyl-; phenyl; furanyl; or thienyl; wherein U is optionally substituted with halo or C₁₋₆alkyl;
V represents C₀₋₄alkylene or a C₁₋₄alkoxy linker group, either of which is optionally substituted with halo;
Either
i) D represents an 8-12-membered fused bicyclic ring system XY, wherein one fused ring X is linked to V and is either saturated or partially saturated and the other ring Y is either aromatic, saturated or partially saturated and wherein ring X contains one or two nitrogen atoms; and wherein D is optionally substituted with halo, CF₃, OCF₃, C₁₋₄alkyl or -SO₂C₁₋₄alkyl;
   or
ii) D represents -E-W-Cy, wherein
   E represents -N(R⁴)- or a 5-7-membered saturated or partially saturated heterocyclic ring containing one or two nitrogen atoms, wherein any nitrogen atom in the heterocyclic ring is a tertiary nitrogen, and wherein the heterocyclic ring is optionally substituted with with halo, or with one or more C₁₋₄alkyl groups;
   R⁴ represents hydrogen, optionally substituted C₃₋₇cycloalkyl wherein C₃₋₇cycloalkyl is optionally substituted with one or more C₁₋₄alkyl groups, or optionally substituted C₁₋₆alkyl, wherein C₁₋₆alkyl is optionally substituted with a group selected from C₃-₇cycloalkyl; phenyl and C₁₋₄alkoxy;
   W represents C₀₋₄alkylene; and
   Cy represents phenyl; furanyl; thienyl; thiazolyl; pyrrolyl; pyridinyl or C₁₋₆alkyl; each of which is optionally substituted with halo, CF₃, OCF₃, C₁₋₄alkyl or -SO₂C₁₋₄alkyl, or Cy represents halo;
   or a pharmaceutically acceptable salt, solvate, ester, carbamate or ether thereof; provided that when Cy represents C₁₋₆alkyl, U represents -C₂alkynyl- and V represents C₁₋₄alkylene or a C₁₋₄alkoxy linker group, either of which is optionally substituted with halo.

### Terms and Definitions

As used herein, the term "alkyl" as a group or a part of a group refers to a linear or branched saturated hydrocarbon group, containing the number of carbon atoms as specified; examples of such groups include methyl, ethyl, propyl, isopropyl, n-butyl, sec-butyl, isobutyl and tert-butyl and the like.

As used herein, the term "alkylene" refers to a linear or branched saturated hydrocarbon linker group, containing the number of carbon atoms as specified. Examples of such groups include methylene and ethylene and the like.

As used herein, the term "alkynyl" refers to a linear or branched hydrocarbon group containing one or more carbon-carbon triple bonds. In one aspect the alkynyl group has from 2 to 6 carbon atoms. Examples of such groups include ethynyl, propynyl, butynyl, pentynyl or hexynyl and the like.

As used herein, the term "alkoxy" refers to an -O-alkyl group wherein alkyl is as defined herein. Examples of such groups include methoxy, ethoxy, propoxy, butoxy, pentoxy or hexoxy and the like.

As used herein, the term "cycloalkyl" as used herein refers to a saturated monocyclic hydrocarbon ring, containing the number of carbon atoms as specified. Examples of such groups include cyclopropyl, cyclobutyl, cyclopentyl, cyclohexyl, cycloheptyl or cyclooctyl and the like. In one aspect, the cycloalkyl group has from 3 to 7 carbon atoms.

As used herein, "halogen" or "halo" refer to a fluorine, chlorine, bromine or iodine atom. In one embodiment, "halogen" or "halo" refer to a fluorine, chlorine or bromine atom. References to "fluoro", "chloro", "bromo" or "iodo" should be construed accordingly.

Unless otherwise specified, "heterocyclic ring" refers to a 5-7 membered monocyclic ring which may be saturated or partially unsaturated containing 1 or 2 nitrogen atoms, wherein the remainder of the atoms are carbon atoms. Examples of such monocyclic rings include pyrrolidinyl, pyrazolidinyl, oxazolidinyl, piperidinyl, piperazinyl, morpholinyl, thiomorpholinyl, thiazolidinyl, hydantoinyl, valerolactamyl, oxiranyl, oxetanyl, dioxolanyl, dioxanyl, oxathiolanyl, oxathianyl, dithianyl, dihydrofuranyl, tetrahydrofuranyl, dihydropyranyl, tetrahydropyranyl, tetrahydropyridinyl, tetrahydropyrimidinyl, tetrahydrothiophenyl, tetrahydrothiopyranyl, diazepanyl, azepanyl and the like.

As used herein, "fused bicyclic ring system" refers to a fused bicyclic ring system which may be saturated or partially unsaturated containing 1 to 4 heteroatoms selected from oxygen, nitrogen or sulphur. Examples of such bicyclic rings include indolinyl, isoindolinyl, benzopyranyl, quinuclidinyl, 2,3,4,5-tetrahydro-1*H*-3-benzazepine, tetrahydroisoquinolinyl and the like. In one aspect, "fused bicyclic ring system" moieties contain 8-12 carbon atoms.

All aspects and embodiments of the invention described herein are in respect of compounds of Formula I, unless otherwise specified.

In one aspect of the invention R¹ represents C₁₋₄alkyl. In a further aspect of the invention, R¹ represents methyl.

In one aspect of the invention, R² represents C₁₋₄alkyl. In a further aspect of the invention, R² represents methyl.

In one aspect of the invention, R³ represents chloro.

In one aspect of the invention, A represents phenyl, pyridinyl or benzothienyl. In a further aspect of the invention, A represents phenyl, pyridinyl or benzothienyl wherein A is optionally substituted with halo. In a yet further aspect of the invention, A represents unsubstituted phenyl (for example, phenyl attached to the pyridone ring and the group U in a para- or meta-orientation), or A represents unsubstituted pyridinyl (for example, pyridinyl attached to the pyridone ring and the group U), or unsubstituted benzothienyl (for example 1-benzothien-5-yl).

In one aspect of the invention U represents a bond; -C₂alkynyl-; phenyl (for example phenyl attached to the pyridine ring and the group U in a para- or meta-orientation); -furanyl-; or -thienyl-; wherein U is optionally substituted with halo. In a further aspect of the invention, U represents a bond; -C₂alkynyl-; phenyl (for example phenyl attached to the pyridine ring and the group U in a para- or meta-orientation, e.g. 1,4-phenyl); -furanyl-(e.g. 2,5-furanyl); or-thienyl- (e.g. 2,5-thienyl).

In one aspect of the invention, V represents C₁₋₄alkylene or a C₁₋₄alkoxy linker group, either of which is optionally substituted with halo. In another aspect, V represents C₀alkylene, -CH₂-, -CH₂CH₂-, -CH₂CH₂CH₂-, -C(CH₃)₂-, -CF₂-CH₂- or -O-CH₂-CH₂-.

In one aspect of the invention, when D represents a 8-12 membered fused bicyclic ring system XY, wherein one fused ring X is linked to V and is either saturated or partially saturated and the other ring Y is either aromatic, saturated or partially saturated and wherein ring X contains one or two nitrogen atoms; and wherein D is optionally substituted with halo, CF₃, OCF₃, C₁₋₄alkyl or -SO₂C₁₋₄alkyl; and U represents -C₂alkynyl-; phenyl; furanyl; or thienyl; wherein U is optionally substituted with halo or C₁₋₆alkyl. In a further aspect of the invention, when D represents a 8-12 membered fused bicyclic ring system XY, wherein one fused ring X is linked to V and is either saturated or partially saturated and the other ring Y is either aromatic, saturated or partially saturated and wherein ring X contains one or two nitrogen atoms; and wherein D is optionally substituted with halo, CF₃, OCF₃, C₁₋₄alkyl or -SO₂C₁₋₄alkyl, U represents -C₂alkynyl-.

In one aspect of the invention, D represents a 10-membered fused bicyclic ring system XY, wherein one fused ring X is linked to V and is either saturated and the other ring Y is either aromatic and wherein ring X contains one nitrogen atom; and wherein D is optionally substituted with CF₃. In a further aspect of the invention, D represents 6-(trifluoromethyl)-3,4-dihydro-2(1H)-isoquinolinyl or 8-(trifluoromethyl)-3,4-dihydro-2(1H)-isoquinolinyl.

In one aspect of the invention, D represents -E-W-Cy. In another aspect, -E-C₀₋₄alkylene-Cy.

In one aspect of the invention, E represents -N(R⁴)- wherein R⁴ represents hydrogen, methyl, propyl, 2-methylpropyl, cyclopropyl, phenylmethyl, cyclopentylmethyl or 2-(methyloxy)ethyl.

In one aspect of the invention, E represents a 5- or 6-membered saturated or partially saturated heterocyclic ring containing one nitrogen atom, wherein the heterocyclic ring is optionally substituted with with halo (for example fluoro), or with one or more C₁₋₄alkyl groups. In a further aspect of the invention, E represents a 6-membered saturated or partially saturated heterocyclic ring containing one nitrogen atom wherein the heterocyclic ring is optionally substituted with with halo (for example fluoro), or with one or more C₁₋₄alkyl groups. In a further aspect of the invention, E represents a 5-membered saturated or partially saturated heterocyclic ring containing one nitrogen atom wherein the heterocyclic ring is optionally substituted with with halo (for example fluoro), or with one or more C₁₋₄alkyl groups. In a further aspect of the invention, E represents a 6-membered saturated or partially saturated heterocyclic ring containing one nitrogen atom wherein the heterocyclic ring is optionally substituted with with halo (for example fluoro), or with one or more C₁₋₄alkyl groups, wherein E is linked to either V or W via the nitrogen atom. In a further aspect of the invention, E represents a 5-membered saturated or partially saturated heterocyclic ring containing one nitrogen atom and wherein the heterocyclic ring is optionally substituted with with halo (for example fluoro), or with one or more C₁₋₄alkyl groups, wherein E is linked to either V or W via the nitrogen atom. In a further aspect of the invention, E represents piperidinyl, 1,2,3,6-tetrahydro-4-pyridinyl or pyrrolidinyl.

In one aspect of the invention, W represents C₀alkylene, -CH₂- or -CH₂CH₂-.

In one aspect of the invention, Cy represents phenyl; furanyl; thienyl; thiazolyl; pyrrolyl; or C₁₋₆alkyl; each of which is optionally substituted with one or more substituents selected from halo, CF₃, OCF₃, C₁₋₄alkyl and -SO₂C₁₋₄alkyl, or Cy represents halo, for example fluoro, provided that when Cy represents C₁₋₆alkyl, U represents -C₂alkynyl- and V represents C₁₋₄alkylene or a C₁₋₄alkoxy linker group, either of which is optionally substituted with halo. In one aspect of the invention, Cy represents C₁₋₆alkyl substituted with halo, for example CF₃. In one aspect of the invention, Cy represents unsubstituted phenyl, 4-(methylsulfonyl)phenyl, fluorophenyl [for example 4-fluorophenyl or 2,4-difluorophenyl], chlorophenyl [for example 2,4-dichlorophenyl], (4-trifluoromethyl)oxyphenyl, (trifluoromethyl)phenyl [for example, 2-(trifluoromethyl)phenyl, 4-(trifluoromethyl)phenyl or 2,4-bis(trifluoromethyl)phenyl], furanyl [for example, unsubstituted furan-2-yl], thienyl [for example unsubstituted thien-2-yl, unsubstituted thien-3-yl or 5-methyl-thien-2-yl], thiazolyl [for example, 1,3-thiazol-2-yl], pyrrolyl [for example 1-methyl-1H-pyrrol-2-yl], C₁₋₆alkyl [for example -C(CH₃)₃ or -CF₃] or halo (for example fluoro).

In one aspect of the invention, R¹ represents C₁₋₄alkyl; R² represents C₁₋₄alkyl; R³ represents halo; A represents phenyl or benzothienyl wherein A is optionally substituted with halo; U represents bond; -C₂alkynyl-; phenyl (for example phenyl attached to the pyridine ring and the group U in a para- or meta-orientation); furanyl; or thienyl; wherein U is optionally substituted with halo; V represents C₀alkylene, -CH₂-, -CH₂CH₂-, - CH₂CH₂CH₂-, -C(CH₃)₂-, -CF₂-CH₂- or -O-CH₂-CH₂-; D represents a 10-membered fused bicyclic ring system XY, wherein one fused ring X is linked to V and is is either saturated and the other ring Y is either aromatic and wherein ring X contains one nitrogen atom; and wherein D is optionally substituted with CF₃ or D represents -E-W-Cy; E represents a 5 or 6-membered saturated or partially saturated heterocyclic ring containing one nitrogen atom, wherein the nitrogen atom in the heterocyclic ring is a tertiary nitrogen, and wherein the heterocyclic ring is optionally substituted with with halo, or with one or more C₁₋₄alkyl groups; W represents C₀alkylene, -CH₂- or -CH₂CH₂-; and Cy represents phenyl; furanyl; thienyl; thiazolyl; pyrrolyl; or C₁₋₆alkyl; each of which is optionally substituted with one or more substituents selected from halo, CF₃, OCF₃, C₁₋₄alkyl and -SO₂C₁₋₄alkyl, or Cy represents halo, for example fluoro, provided that when Cy represents C₁₋₆alkyl, U represents -C₂alkynyl- and V represents C₁₋₄alkylene or a C₁₋₄alkoxy linker group, either of which is optionally substituted with halo.

In a further aspect of the invention, R¹ represents methyl; R² represents methyl; R³ represents chloro; A represents unsubstituted phenyl (for example, phenyl attached to the pyridine ring and the group U in a para- or meta-orientation) or benzothienyl (for example 1-benzothien-5-yl); U represents bond; -C₂alkynyl-; phenyl (for example phenyl attached to the pyridine ring and the group U in a para- or meta-orientation, e.g. 1,4-phenyl); furanyl (e.g. 2,5-furanyl); or thienyl (e.g. 2,5-thienyl); V represents C₀alkylene, -CH₂-, -CH₂CH₂-, - CH₂CH₂CH₂-, -C(CH₃)₂-, -CF₂-CH₂- or -O-CH₂-CH₂-; D represents 6-(trifluoromethyl)-3,4-dihydro-2(1H)-isoquinolinyl or 8-(trifluoromethyl)-3,4-dihydro-2(1H)-isoquinolinyl, or D represents -E-C₀₋₄alkylene-Cy; E represents -N(R⁴)- and R⁴ represents hydrogen, methyl, propyl, 2-methylpropyl, cyclopropyl, phenylmethyl, cyclopentylmethyl or 2-(methyloxy)ethyl, or E represents piperidinyl or 1,2,3,6-tetrahydro-4-pyridinyl; W represents C₀alkylene, -CH₂- or -CH₂CH₂-; Cy represents unsubstituted phenyl, 4-(methylsulfonyl)phenyl, fluorophenyl [for example 4-fluorophenyl or 2,4-difluorophenyl], chlorophenyl [for example 2,4-dichlorophenyl], (4-trifluoromethyl)oxyphenyl, (trifluoromethyl)phenyl [for example, 2-(trifluoromethyl)phenyl, 4-(trifluoromethyl)phenyl or 2,4-bis(trifluoromethyl)phenyl], furanyl [for example, unsubstituted furan-2-yl], thienyl [for example unsubstituted thien-2-yl, unsubstituted thien-3-yl or 5-methyl-thien-2-yl], thiazolyl [for example, 1,3-thiazol-2-yl], pyrrolyl [for example 1-methyl-1H-pyrrol-2-yl] or C₁₋₆alkyl [for example -C(CH₃)₃ or -CF₃].

The meaning of any functional group or substituent thereon at any one occurrence in Formula I, or any subformula thereof, is independent of its meaning, or any other functional group's or substituent's meaning, at any other occurrence, unless stated otherwise. It is to be understood that the present invention covers all combinations of the groups according to different aspects of the invention as described hereinabove.

In one aspect of the invention, there is provided a compound, or a pharmaceutically acceptable derivative thereof, wherein the compound is selected from the group consisting of:
3-chloro-2,6-dimethyl-5-(4-{3-[methyl({4-[(trifluoromethyl)oxy]phenyl}methyl)amino]-1-propyn-1-yl}phenyl)-4(1H)-pyridinone;
3-chloro-2,6-dimethyl-5-{4-[3-(methyl{[4-(trifluoromethyl)phenyl]methyl}amino)-1-propyn-1-yl]phenyl}-4(1H)-pyridinone;
3-chloro-2,6-dimethyl-5-{4-[3-(methyl{[2-(trifluoromethyl)phenyl]methyl}amino)-1-propyn-1-yl]phenyl}-4(1H)-pyridinone;
3-(4-{3-[{[2,4-bis(trifluoromethyl)phenyl]methyl}(methyl)amino]-1-propyn-1-yl}phenyl)-5-chloro-2,6-dimethyl-4(1H)-pyridinone;
3-chloro-2,6-dimethyl-5-(4-{3-[methyl(phenylmethyl)amino]-1-propyn-1-yl}phenyl)-4(H)-pyridinone;
3-chloro-2,6-dimethyl-5-(4-{3-[propyl({4-[(trifluoromethyl)oxy]phenyl}methyl)amino]-1-propyn-1-yl}phenyl)-4(1H)-pyridinone trifluoroacetate;
3-chloro-5-(4-{3-[cyciopropyl({4-[(trifiuoromethyl)oxy]phenyl}methyl)amino]-1-propyn-1-yl}phenyl)-2,6-dimethyl-4(1H)-pyridinone;
3-chloro-2,6-dimethyl-5-(4-{3-[({4-[(trifluoromethyl)oxy]phenyl}methyl)amino]-1-propyn-1-yl}phenyl)-4(1H)-pyridinone;
3-chloro-2,6-dimethyl-5-(4-{3-[[2-(methyloxy)ethyl]({4-[(trifluoromethyl)oxy]phenyl}methyl)amino]-1-propyn-1-yl}phenyl)-4(1H)-pyridinone;
3-chloro-2,6-dimethyl-5-{4-[3-(4-phenyl-1-piperidinyl)-1-propyn-1-yl]phenyl}-4(1H)-pyridinone;
3-chloro-2,6-dimethyl-5-{4-[3-(4-{4-[(trifluoromethyl)oxy]phenyl}-1-piperidinyl)-1-propyn-1-yl]phenyl}-4(1H)-pyridinone hydrochloride;
3-(4-{3-[bis(phenylmethyl)amino]-1-propyn-1-yl}phenyl)-5-chloro-2,6-dimethyl-4(1H)-pyridinone hydrochloride;
3-chloro-2,6-dimethyl-5-{4-[3-(2-phenyl-1-piperidinyl)-l-propyn-1-yl]phenyl}-4(1H)-pyridinone hydrochloride;
3-chloro-2,6-dimethyl-5-(4-{3-[6-(trifluoromethyl)-3,4-dihydro-2(1H)-isoquinolinyl]-1-propyn-1-yl}phenyl)-4(1H)-pyridinone hydrochloride;
3-chloro-2,6-dimethyl-5-(4-{3-[8-(trifluoromethyl)-3,4-dihydro-2(1H)-isoquinolinyl]-1-propyn-1-yl}phenyl)-4(1H)-pyridinone hydrochloride;
3-chloro-2,6-dimethyl-5-{4-[3-methyl-3-(4-phenyl-1-piperidinyl)-1-butyn-1-yl]phenyl}-4(1H)-pyridinone;
3-chloro-2,6-dimethyl-5-(4-{3-[methyl(2-{4-[(trifluoromethyl)oxy]phenyl}ethyl)amino]-1-propyn-1-yl}phenyl)-4(1H)-pyridinone hydrochloride;
3-chloro-2,6-dimethyl-5-(4-{4-[methyl({4-[(trifluoromethyl)oxy]phenyl}methyl)amino]-1-butyn-1-yl}phenyl)-4(1H)-pyridinone hydrochloride;
3-chloro-2,6-dimethyl-5-(4-{3-[methyl({4-[(trifluoromethyl)oxy]phenyl}methyl)amino]-1-propyn-1-yl}phenyl)-4(1H)-pyridinone hydrochloride;
3-chloro-2,6-dimethyl-5-(4-{3-[propyl({4-[(trifluoromethyl)oxy]phenyl}methyl)amino]-1-propyn-1-yl}phenyl)-4(1H)-pyridinone;
3-chloro-2,6-dimethyl-5-(4-{3-[propyl({4-[(trifluoromethyl)oxy]phenyl}methyl)amino]-1-propyn-1-yl}phenyl)-4(1H)-pyridinonehydrochloride;
3-chloro-2,6-dimethyl-5-{4-[3-(4-{4-[(trifluoromethyl)oxy]phenyl}-1-piperidinyl)-1-propyn-1-yl]phenyl}-4(1H)-pyridinone trifluoroacetate;
3-chloro-2,6-dimethyl-5-(4-{[methyl({4-[(trifluoromethyl)oxy]phenyl}methyl)amino]methyl}phenyl)-4(1H)-pyridinonehydrochloride;
3-chloro-2,6-dimethyl-5-{4-[(4-{4-[(trifluoromethyl)oxy]phenyl}-1-piperidinyl)methyl]phenyl}-4(1H)-pyridinone hydrochloride;
3-chloro-2,6-dimethyl-5-(3-{[methyl({4-[(trifluoromethyl)oxy]phenyl}methyl)amino]methyl}phenyl)-4(1H)-pyridinonehydrochloride;
3-chloro-2,6-dimethyl-5-[4-(5-{[methyl({4-[(trifluoromethyl)oxy]phenyl}methyl)amino]methyl}-2-thienyl)phenyl]-4(1H)-pyridinone trifluoroacetate;
3-chloro-2,6-dimethyl-5-[4-(5-{[methyl({4-[(trifluoromethyl)oxy]phenyl}methyl)amino]methyl}-2-thienyl)phenyl]-4(1H)-pyridinone hydrochloride;
3-chloro-2,6-dimethyl-5-[4-(5-{[methyl({4-[(trifluoromethyl)oxy]phenyl}methyl)amino]methyl}-2-furanyl)phenyl]-4(1H)-pyridinone trifluoroacetate;
3-chloro-2,6-dimethyl-5-[4-(5-{[methyl({4-[(trifluoromethyl)oxy]phenyl}methyl)amino]methyl}-2-furanyl)phenyl]-4(1H)-pyridinone hydrochloride;
3-chloro-2,6-dimethyl-5-{3-[3-(4-{4-[(trifluoromethyl)oxy]phenyl}-1-piperidinyl)-1-propyn-1-yl]phenyl}-4(1H)-pyridinone trifluoroacetate;
3-chloro-2,6-dimethyl-5-(2-{[methyl({4-[(trifluoromethyl)oxy]phenyl}methyl)amino]methyl}-1-benzothien-5-yl)-4(1H)-pyridinone hydrochloride;
3-chloro-2,6-dimethyl-5-{4-[1-({4-[(trifluoromethyl)oxy]phenyl}methyl)-1,2,3,6-tetrahydro-4-pyridinyl]phenyl}-4(1H)-pyridinone hydrochloride;
3-chloro-2,6-dimethyl-5-{4'-[(4-{4-[(trifluoromethyl)oxy]phenyl}-1-piperidinyl)methyl]-4-biphenylyl}-4(1H)-pyridinone hydrochloride;
3-chloro-2,6-dimethyl-5-(3'-{[methyl({4-[(trifluoromethyl)oxy]phenyl}methyl)amino]methyl}-4-biphenylyl)-4(1H)-pyridinone hydrochloride;
3-chloro-2,6-dimethyl-5-(4'-{[methyl({4-[(trifluoromethyl)oxy]phenyl}methyl)amino]methyl}-4-biphenylyl)-4(1H)-pyridinone hydrochloride;
3-chloro-2,6-dimethyl-5-(4'-{[methyl({4-[(trifluoromethyl)oxy]phenyl}methyl)amino]methyl}-4-biphenylyl)-4(1H)-pyridinone trifluoroacetate;
3-chloro-2,6-dimethyl-5-(4-{3-[(2-methylpropyl)({4-[(trifluoromethyl)oxy]phenyl}methyl)amino]-1-propyn-1-yl}phenyl)-4(1H)-pyridinone hydrochloride;
3-chloro-5-(4-{3-[(cyclopentylmethyl)({4-[(trifluoromethyl)oxy]phenyl}methyl)amino]-1-propyn-1-yl}phenyl)-2,6-dimethyl-4(1H)-pyridinone hydrochloride;
3-chloro-2,6-dimethyl-5-(4-{3-[methyl(2-thienylmethyl)amino]-1-propyn-1-yl}phenyl)-4(H)-pyridinone hydrochloride;
3-chloro-5-(4-{3-[[(2,4-difluorophenyl)methyl](methyl)amino]-1-propyn-1-yl}phenyl)-2,6-dimethyl-4(1H)-pyridinone hydrochloride;
3-chloro-5-(4-{3-[(2-furanylmethyl)(methyl)amino]-1-propyn-1-yl}phenyl)-2,6-dimethyl-4(1H)-pyridinone hydrochloride;
3-chloro-2,6-dimethyl-5-[4-(3-{methyl[(5-methyl-2-thienyl)methyl]amino}-1-propyn-1-yl)phenyl]-4(1H)-pyridinone hydrochloride;
3-chloro-2,6-dimethyl-5-(4-{3-[methyl(1,3-thiazol-2-ylmethyl)amino]-1-propyn-1-yl}phenyl)-4(1H)-pyridinone hydrochloride;
3-chloro-2,6-dimethyl-5-[4-(3-{methyl[(1-methyl-1H-pyrrol-2-yl)methyl]aminol-1-propyn-1-yl)phenyl]-4(1H)-pyridinone hydrochloride;
3-chloro-2,6-dimethyl-5-(4-{3-[methyl(3-thienylmethyl)amino]-1-propyn-1-yl}phenyl)-4(H)-pyridinone hydrochloride;
3-chloro-5-(4-{3-[[(2,4-dichlorophenyl)methyl](methyl)amino]-1-propyn-1-yl}phenyl)-2,6-dimethyl-4(1H)-pyridinone hydrochloride;
3-chloro-2,6-dimethyl-5-{4-[3-(methyl{[4-(methylsulfonyl)phenyl]methyl}amino)-1-propyn-1-yl]phenyl}-4(1H)-pyridinone hydrochloride;
3-chloro-5-(4-{3-[(3,3-dimethylbutyl)(methyl)amino]-1-propyn-1-yl}phenyl)-2,6-dimethyl-4(1H)-pyridinone hydrochloride;
3-chloro-2,6-dimethyl-5-(4-{3-[methyl(3,3,3-trifluoropropyl)amino]-1-propyn-1-yl}phenyl)-4(1H)-pyridinone hydrochloride;
3-chloro-5-(4-{3-[[(4-fluorophenyl)methyl](methyl)amino]-1-propyn-1-yl}phenyl)-2,6-dimethyl-4(1H)-pyridinone;
3-chloro-2,6-dimethyl-5-(4-{3-[methyl({4-[(trifluoromethyl)oxy]phenyl}methyl)amino]propyl}phenyl)-4(1H)-pyridinone;
3-chloro-2,6-dimethyl-5-{4-[3-(4-{4-[(trifluoromethyl)oxy]phenyl}-1-piperidinyl)propyl]phenyl}-4(1H)-pyridinone hydrochloride;
3-chloro-2,6-dimethyl-5-(4-{3-[methyl({4-[(trifluoromethyl)oxy]phenyl}methyl) amino]-1-propyn-1-yl}phenyl)-4-pyridinyl methyl[2-(methylamino)ethyl]carbamate hydrochloride;
3-chloro-2,6-dimethyl-5-{4'-[(4-{4-[(trifluoromethyl)oxy]phenyl}-1-piperidinyl)methyl]-4-biphenylyl}-4-pyridinyl [2-({[(1,1-dimethylethyl)oxy]carbonyl}amino)ethyl]methylcarbamate;
3-chloro-2,6-dimethyl-5-{4'-[(4-{4-[(trifluoromethyl)oxy]phenyl}-1-piperidinyl)methyl]-4-biphenylyl}-4-pyridinyl methyl[2-(methylamino)ethyl]carbamate;
3-chloro-2,6-dimethyl-5-{4'-[(4-{4-[(trifluoromethyl)oxy]phenyl}-1-piperidinyl)methyl]-4-biphenylyl}-4-pyridinyl acetate hydrochloride;
3-chloro-2,6-dimethyl-5-(4'-{[4-(trifluoromethyl)-1-piperidinyl]methyl}-4-biphenylyl)-4(1H)-pyridinone hydrochloride;
3-chloro-5-{4'-[(4-fluoro-1-piperidinyl)methyl]-4-biphenylyl}-2,6-dimethyl-4(1H)-pyridinone hydrochloride;
3-chloro-5-(4'-{[4-(1,1-dimethylethyl)-1-piperidinyl]methyl}-4-biphenylyl)-2,6-dimethyl-4(1H)-pyridinone hydrochloride;
3-chloro-5-{4'-[(3,3-difluoro-1-pyrrolidinyl)methyl]-4-biphenylyl}-2,6-dimethyl-4(1H)-pyridinone hydrochloride;
3-chloro-6-(hydroxymethyl)-2-methyl-5-{4'-[(4-{4-[(trifluoromethyl)oxy]phenyl}-1-piperidinyl)methyl]-4-biphenylyl}-4(1H)-pyridinone;
3-chloro-6-(hydroxymethyl)-2-methyl-5-{4'-[(4-{4-[(trifluoromethyl)oxy]phenyl}-1-piperidinyl)methyl]-4-biphenylyl}-4(1H)-pyridinone hydrochloride;
3-chloro-2-(hydroxymethyl)-6-methyl-5-{4'-[(4-{4-[(trifluoromethyl)oxy]phenyl}-1-piperidinyl)methyl]-4-biphenylyl}-4(1H)-pyridinone hydrochloride;
5-chloro-2,6-dimethyl-6'-{4-[(4-{4-[(trifluoromethyl)oxy]phenyl}-1-piperidinyl)methyl]phenyl}-3,3'-bipyridin-4(1H)-one hydrochloride;
5-chloro-2,6-dimethyl-6'-(4-{[4-(trifluoromethyl)-1-piperidinyl]methyl}phenyl)-3,3'-bipyridin-4(1H)-one;
5-chloro-6'-{4-[(4,4-difluoro-1-piperidinyl)methyl]phenyl}-2,6-dimethyl-3,3'-bipyridin-4(1H)-one;
5-chloro-6'-{1,1-difluoro-2-[4-(trifluoromethyl)-1-piperidinyl]ethyl}-2,6-dimethyl-3,3'-bipyridin-4(1H)-one; and
5-chloro-2,6-dimethyl-6'-({2-[4-(trifluoromethyl)-1-piperidinyl]ethyl}oxy)-3,3'-bipyridin-4(1H)-one hydrochloride.

As used herein, the term "pharmaceutically acceptable derivative", means any pharmaceutically acceptable salt, solvate, or prodrug e.g. ester or carbamate of a compound of Formula I, which upon administration to the recipient is capable of providing (directly or indirectly) a compound of Formula I, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. In one aspect of the invention, pharmaceutically acceptable derivatives are salts, solvates, esters or carbamates. In another aspect of the invention pharmaceutically acceptable derivatives are salts, solvates or esters. In a further aspect, pharmaceutically acceptable derivatives are salts or solvates. In a yet further aspect, pharmaceutically acceptable derivatives are salts. In another aspect, when R² or R³ are -(CH₂)ₙOH, pharmaceutically acceptable derivatives of the OH group of R² or R³ include -O(CO)C₁₋₆alkyl (for example -O(CO)CH₃) and -O(CO)N(C₁₋₄alkyl)-C₁₋₂alkyl-N⁺H₂(C₁₋₆alkyl). In a further aspect of the invention, pharmaceutically acceptable derivatives at the OH group of the 4-pyridinol tautomeric form include -O(CO)C₁₋₆alkyl (for example -O(CO)CH₃) and -O(CO)N(C₁₋₄alkyl)-C₁₋₂alkyl-N⁺H₂(C₁₋₆alkyl) and -O-PO₃ Na₂.

An ester of a compound of Formula I may be hydrolysable under *in vivo* conditions in the human body. Suitable pharmaceutically acceptable *in vivo* hydrolysable ester groups include those which break down readily in the human body to leave the parent acid or the salt thereof. An ester may be formed at a hydroxy group (OH) of a compound of Formula I using methods well known in the art involving reaction with the corresponding anhydride or the corresponding acid halide e.g. acid chloride, in the presence of a suitable base, for example pyridine. For example, esters may be C₁₋₆alkyl esters, wherein alkyl is as defined herein, e.g. methyl esters, ethyl esters and the like.

It will be appreciated that certain pharmaceutically acceptable salts of the compounds according to Formula I may be prepared, since the compounds of the invention are weakly acidic. Indeed, in certain embodiments of the invention, pharmaceutically acceptable salts of the compounds according to Formula I may be preferred over the respective free base or free acid because such salts impart greater stability or solubility to the molecule thereby facilitating formulation into a dosage form. The compounds of the present invention may also be administered as a pharmaceutically acceptable salt. Accordingly, the invention is further directed to pharmaceutically acceptable salts of the compounds according to Formula I.

As used herein, the term "pharmaceutically acceptable salts" refers to salts that retain the desired biological activity of the subject compound and exhibit minimal undesired toxicological effects. For a review on suitable salts see Berge et al, J. Pharm. Sci., 1977, 66, 1-19. The term "pharmaceutically acceptable salts" includes both pharmaceutically acceptable acid addition salts and pharmaceutically acceptable base addition salts. These pharmaceutically acceptable salts may be prepared *in situ* during the final isolation and purification of the compound, or by separately reacting the purified compound in its free acid or free base form with a suitable base or acid, respectively. The salt may precipitate from solution and be collected by filtration or may be recovered by evaporation of the solvent.

In certain embodiments, compounds according to Formula I may contain an acidic functional group and may therefore be capable of forming pharmaceutically acceptable base addition salts by treatment with a suitable base. A pharmaceutically acceptable base addition salt may be formed by reaction of a compound of Formula I with a suitable strong inorganic base, optionally in a suitable solvent such as an organic solvent, to give the base addition salt which may be isolated for example by crystallisation and filtration. Pharmaceutically acceptable base salts include include pharmaceutically acceptable metal salts, for example pharmaceutically acceptable alkali-metal or alkaline-earth-metal salts such as sodium, potassium, lithium or calcium.

In certain embodiments, compounds according to Formula I may contain a basic functional group and may therefore be capable of forming pharmaceutically acceptable acid addition salts by treatment with a suitable acid. A pharmaceutically acceptable acid addition salt may be formed by reaction of a compound of Formula I with a suitable strong inorganic or organic acid (such as hydrobromic, hydrochloric, hydroiodic, sulfuric, nitric, phosphoric, perchloric, p-toluenesulfonic, benzenesulfonic, methanesulfonic, ethanesulfonic, 2-hydroxyethanesulfonic, naphthalenesulfonic (e.g. 2-naphthalenesulfonic), optionally in a suitable solvent such as an organic solvent, to give the salt which is usually isolated for example by crystallisation and filtration. Pharmaceutically acceptable acid addition salts include a hydrobromide, hydrochloride, hydroiodide, sulfate, bisulfate, nitrate, phosphate, perchlorate, p-toluenesulfonate, benzenesulfonate, methanesulfonate, ethanesulfonate, 2-hydroxyethanesulfonate or naphthalenesulfonate (e.g. 2-naphthalenesulfonate) salt. In one embodiment, a pharmaceutically acceptable acid addition salt of a compound of Formula I is a salt of a strong acid, for example a hydrobromide, hydrochloride, hydroiodide, sulfate, nitrate, perchlorate, phosphate p-toluenesulfonic, benzenesulfonic, methanesulfonic salt.

The invention includes within its scope all possible stoichiometric and non-stoichiometric forms of the salts of the compounds of Formula I.

As used herein, the term "compounds of the invention" means the compounds according to Formula I and the pharmaceutically acceptable derivatives thereof. The term "a compound of the invention" means any one of the compounds of the invention as defined above.

The compounds of the invention may exist as solids or liquids, both of which are included in the invention. In the solid state, the compounds of the invention may exist as either amorphous material or in crystalline form, or as a mixture thereof. It will be appreciated that pharmaceutically acceptable solvates of compounds of the invention may be formed wherein solvent molecules are incorporated into the crystalline lattice during crystallisation. Solvates may involve non-aqueous solvents such as ethanol, isopropanol, dimethylsulfoxide (DMSO), acetic acid, ethanolamine, and ethyl acetate, or they may involve water as the solvent that is incorporated into the crystalline lattice. Solvates wherein water is the solvent that is incorporated into the crystalline lattice are typically referred to as "hydrates." The invention includes all such solvates.

It will be appreciated that compounds of the invention can exist in different tautomeric forms. In particular, compounds of Formula I may exist in the 4-pyridinol tautomeric form as follows:

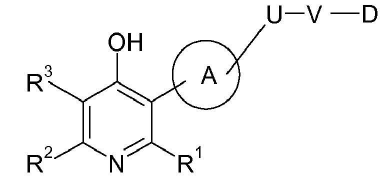

All possible tautomeric forms of the compounds of Formula I are contemplated to be within the scope of the present invention. In one aspect of the invention, there is provided compounds of Formula I in the 4-pyridone tautomeric form. In another aspect of the invention, there is provided compounds of Formula I in the 4-pyridinol tautomeric form. In a further aspect of the invention, there is provided a mixture of compounds of Formula I in the 4-pyridone and 4-pyridinol tautomeric forms. In a yet further aspect of the invention, the mixture of 4-pyridone and 4-pyridinol tautomeric forms of compounds of Formula I is an equilibrium mixture.

According to another aspect of the invention there is provided a compound of Formula I or a pharmaceutically acceptable derivative thereof for use in human or veterinary medical therapy.

The compounds of the invention can be useful in the treatment of certain parasitic infections such as parasitic protozoal infections by the malarial parasite *Plasmodium falciparum,* species of *Eimeria, Pneumocytis carnii, Trypanosoma cruzi, Trypanosoma brucei* or *Leishmania donovani.* In particular, the compounds of the invention can be useful for treatment of infection by *Plasmodium falciparum.* Accordingly, the invention is directed to methods of treating such conditions.

In one aspect of the invention, there is provided a compound of Formula I or a pharmaceutically acceptable derivative thereof, for use in therapy, for example the treatment of parasitic protozoal infections such as malaria, for example infection by *Plasmodium falciparum.*

In another aspect of the invention there is provided the use of a compound of Formula I or a pharmaceutically acceptable derivative thereof in the manufacture of a medicament for the treatment of parasitic protozoal infections such as malaria, for example a condition caused by infection by *Plasmodium falciparum.*

In another aspect of the invention there is provided a method for the treatment of a human or animal subject suffering from a parasitic protozoal infection such as malaria, for example infection by *Plasmodium falciparum,* comprising administering to said human or animal subject an effective amount of a compound of Formula I or a pharmaceutically acceptable derivative thereof, or a pharmaceutical composition comprising a compound of Formula I or a pharmaceutically acceptable derivative thereof.

The methods of treatment of the invention comprise administering a safe and effective amount of a compound according to Formula I or a pharmaceutically acceptable derivative thereof to a patient in need thereof.

As used herein, "treatment" means: (1) the amelioration or prevention of the condition being treated or one or more of the biological manifestations of the condition being treated, (2) the interference with (a) one or more points in the biological cascade that leads to or is responsible for the condition being treated or (b) one or more of the biological manifestations of the condition being treated, or (3) the alleviation of one or more of the symptoms or effects associated with the condition being treated. The skilled artisan will appreciate that "prevention" is not an absolute term. In medicine, "prevention" is understood to refer to the prophylactic administration of a drug to substantially diminish the likelihood or severity of a condition or biological manifestation thereof, or to delay the onset of such condition or biological manifestation thereof.

As used herein, "safe and effective amount" means an amount of the compound sufficient to significantly induce a positive modification in the condition to be treated but low enough to avoid serious side effects (at a reasonable benefit/risk ratio) within the scope of sound medical judgment. A safe and effective amount of a compound of the invention will vary with the particular compound chosen (e.g. depending on the potency, efficacy, and half-life of the compound); the route of administration chosen; the nature of the infection and/or condition being treated; the severity of the infection and/or condition being treated; the age, size, weight, and physical condition of the patient being treated; the medical history of the patient to be treated; the duration of the treatment; the nature of concurrent therapy; the desired therapeutic effect; and like factors, but can nevertheless be routinely determined by the skilled artisan.

As used herein, "patient" refers to a human or other animal.

The compounds of the invention may be administered by any suitable route of administration, including systemic administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages. Topical administration includes dermal application to the skin as well as intraocular, buccal (e.g. sub-lingually), rectal, intravaginal, and intranasal administration.

The compounds of the invention may be administered once only, or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. The dosage will also vary according to the nature of the intended treatment, wherein "treatment" is as hereinbelow defined, for example a greater dose of compound may be given for amelioration as compared with prevention of a condition being treated. Suitable dosing regimens for a compound of the invention depend on the pharmacokinetic properties of that compound, such as absorption, distribution, and half-life, which can be determined by the skilled artisan. In addition, suitable dosing regimens for a compound of the invention, including the duration such regimens are administered, depend on the route of administration of the compound, on the condition being treated, the severity of the condition being treated, the age and physical condition of the patient being treated, the medical history of the patient to be treated, the nature of any concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change. It will also be appreciated that if the compounds of the present invention are administered in combination with one or more additional active therapeutic agents as discussed further hereinbelow, the dosing regimen of the compounds of the invention may also vary according to the nature and amount of the one or more additional active therapeutic agents as necessary.

Typical daily dosages may vary depending upon the particular route of administration chosen. Typical daily dosages for oral administration range from about 0.01 to about 25 mg/kg, in one embodiment from about 0.1 to about 14 mg/kg. Typical daily dosages for parenteral administration range from about 0.001 to about 10 mg/kg; in one embodiment from about 0.01 to about 6 mg/kg. In one embodiment, the daily dose range of the compounds is from 100-1000 mg per day.

The compounds of Formula I may also be used in combination with other active therapeutic agents. The invention thus provides, in a further aspect, a combination comprising a compound of Formula I or a pharmaceutically acceptable derivative thereof together with a further active therapeutic agent. When a compound of Formula I or a pharmaceutically acceptable derivative thereof is used in combination with a second active therapeutic agent which is active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian.

The compounds of the present invention may be used alone or in combination with one or more additional active therapeutic agents, such as other antiparasitic drugs, for example antimalarial drugs.

Such other active therapeutic agents include antimalarial drugs such as chloroquine, mefloquine, primaquine quinine, artemisinin, halofantrine, doxycycline, amodiquine, atovaquone, tafenoquine, dapsone, proguanil, sulfadoxine, pyrimethamine, chlorcycloguanil, cycloguanil, and fansidar.

The combinations referred to above may conveniently be presented for use in the form of a pharmaceutical formulation and thus pharmaceutical formulations comprising a combination as defined above together with a pharmaceutically acceptable carrier and/or excipient comprise a further aspect of the invention. The individual components of such combinations may be administered either sequentially or simultaneously in separate or combined pharmaceutical formulations by any convenient route.

When administration is sequential, either the compound of the present invention or the one or more additional active therapeutic agent(s) may be administered first. When administration is simultaneous, the combination may be administered either in the same or different pharmaceutical composition. When combined in the same formulation it will be appreciated that the compound of the present invention and the one or more additional active therapeutic agent(s) must be stable and compatible with each other and the other components of the formulation. When formulated separately the compound of the present invention and the one or more additional active therapeutic agent(s) may be provided in any convenient formulation, conveniently in such manner as are known for such compounds in the art.

### Compositions

The compounds of the invention will normally, but not necessarily, be formulated into pharmaceutical compositions prior to administration to a patient. In one aspect, the invention is directed to pharmaceutical compositions comprising a compound of the invention. In another aspect the invention is directed to a pharmaceutical composition comprising a compound of the invention and one or more pharmaceutically acceptable carriers and/or excipients.

The carrier and/or excipient must be "acceptable" in the sense of being compatible with the other ingredients of the formulation and not deleterious to the recipient thereof.

The pharmaceutical compositions of the invention may be prepared and packaged in bulk form wherein a safe and effective amount of a compound of the invention can be extracted and then given to the patient such as with powders or syrups. Alternatively, the pharmaceutical compositions of the invention may be prepared and packaged in unit dosage form wherein each physically discrete unit contains a safe and effective amount of a compound of the invention. When prepared in unit dosage form, the pharmaceutical compositions of the invention typically contain from about 0.1 to 100mg, in another aspect 0.1 mg to about 50 mg of a compound of the invention.

The pharmaceutical compositions of the invention typically contain one compound of the invention. However, in certain embodiments, the pharmaceutical compositions of the invention contain more than one compound of the invention. For example, in certain embodiments the pharmaceutical compositions of the invention contain two compounds of the invention. In addition, the pharmaceutical compositions of the invention may optionally further comprise one or more additional active therapeutic compounds. The pharmaceutical compositions of the invention typically contain more than one pharmaceutically acceptable excipient. However, in certain embodiments, the pharmaceutical compositions of the invention contain one pharmaceutically acceptable excipient.

As used herein, the term "pharmaceutically acceptable" means suitable for pharmaceutical use.

The compound of the invention and the pharmaceutically acceptable excipient or excipients will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols and solutions; and (6) topical administration such as creams, ointments, lotions, solutions, pastes, sprays, foams, and gels.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carriage or transport of the compound or compounds of the invention from one organ, or portion of the body, to another organ, or portion of the body, once administered to the patient. Certain pharmaceutically acceptable excipients may be chosen for their ability to enhance patient compliance.

Suitable pharmaceutically acceptable excipients include the following types of excipients: binders, disintegrants, lubricants, glidants, granulating agents, coating agents, wetting agents, solvents, co-solvents, suspending agents, emulsifiers, sweeteners, flavouring agents, flavour masking agents, coloring agents, anticaking agents, humectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

In one aspect, the invention is directed to a solid or liquid oral dosage form such as a liquid, tablet, lozenge or a capsule, comprising a safe and effective amount of a compound of the invention and a carrier. The carrier may be in the form of a diluent or filler. Suitable diluents and fillers in general include lactose, sucrose, dextrose, mannitol, sorbitol, starch (e.g. corn starch, potato starch, and pre-gelatinized starch), cellulose and its derivatives (e.g. microcrystalline cellulose), calcium sulfate, and dibasic calcium phosphate. A liquid dosage form will generally consist of a suspension or solution of the compound or pharmaceutically acceptable derivative in a liquid carrier for example, ethanol, olive oil, glycerine, glucose (syrup) or water (e.g. with an added flavouring, suspending, or colouring agent). Where the composition is in the form of a tablet or lozenge, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers or a semi solid e.g. mono di-glycerides of capric acid, Gelucire™ and Labrasol™, or a hard capsule shell e.g. gelatin. Where the composition is in the form of a soft shell capsule e.g. gelatin, any pharmaceutical carrier routinely used for preparing dispersions or suspensions may be considered, for example aqueous gums or oils, and may be incorporated in a soft capsule shell.

An oral solid dosage form may further comprise an excipient in the form of a binder. Suitable binders include starch (e.g. corn starch, potato starch, and pre-gelatinized starch), gelatin, acacia, sodium alginate, alginic acid, tragacanth, guar gum, povidone, and cellulose and its derivatives (e.g. microcrystalline cellulose). The oral solid dosage form may further comprise an excipient in the form of a disintegrant. Suitable disintegrants include crospovidone, sodium starch glycolate, croscarmelose, alginic acid, and sodium carboxymethyl cellulose. The oral solid dosage form may further comprise an excipient in the form of a lubricant. Suitable lubricants include stearic acid, magnesium stearate, calcium stearate, and talc.

There is further provided by the present invention a process of preparing a pharmaceutical composition, which process comprises mixing at least one compound of Formula I or a pharmaceutically acceptable derivative thereof, together with a pharmaceutically acceptable carrier and/or excipient.

Preparations for oral administration may be suitably formulated to give controlled/extended release of the active compound.

### Abbreviations

In describing the invention, chemical elements are identified in accordance with the Periodic Table of the Elements. Abbreviations and symbols utilised herein are in accordance with the common usage of such abbreviations and symbols by those skilled in the chemical arts. The following abbreviations are used herein:
- AcOH: acetic acid
- AlMe₃: trimethyl aluminium
- approx.: approximately
- atm: atmosphere
- BH₃·SMe₂: borane dimethyl sulphide complex
- brine: saturated solution of sodium chloride
- *n*-Bu₄NBr: tetrabutylammonium chloride
- *t*-Bu₃P·HClO₄: tri-tert-butylphosphonium tetrafluoroborate
- n-BuLi: buthyllitium
- CDCl₃: deuterated chloroform
- CD₃OD: deuterated methanol
- CH₂Cl₂: dichloromethane
- CH₃CN: acetonitrile
- conc.: concentrated
- Cul: cooper (I) iodide
- DCC: *N*,*N*-dicyclohexylcarbodiimide
- DME: 1,2-dimethoxyethane
- DMF: N,N-dimethylformamide
- DMSO-d₆: deuterated dimethylsulfoxide
- DIPEA: di-isopropyl-ethyl-amine
- Eaton's reagent: phosphorus pentoxide, 7.7 wt. % in methanesulfonic acid
- Et₃N: triethyl amine
- Et₂O: diethyl ether
- EtOH: ethanol
- EtOAc: ethyl acetate
- ES MS: Electrospray mass spectrometry
- h: hour/hours
- Hex: hexane
- HCl: hydrochloric acid
- H₂O: water
- HPLC: High Performance Liquid Chromatography
- K₂CO₃: potassium carbonate
- KOH: potassium hydroxide
- LiAIH₄: lithium aluminium hydride
- MeOH: methanol
- MeNH₂: methylamine
- MgSO₄: magnesium sulphate
- N₂: nitrogen
- NaBH₄: sodium borohydride
- NaBH₃CN: sodium cyanoborohydride
- Na₂CO₃: sodium carbonate
- NaHB(AcO)₃: sodium triacetoxyborohydride
- NaHCO₃: sodium bicarbonate
- Nal: sodium iodide
- NaOH: sodium hydroxide
- Na₂SO₄: sodium sulfate
- Na₂S₂O₅: sodium metabisulfite
- NH₄Cl: ammonium chloride
- NH₄OH: ammonium hydroxide
- NBS: N-bromosuccinimide
- NCS: N-chlorosuccinimide
- NMP: 1-methyl-2-pyrrolidinone
- NMR: Nuclear Magnetic Resonance spectroscopy
- Pd(OAc)₂: palladium(II) acetate
- Pd(PPh₃)₄: tetrakis(triphenylphosphine)palladium (0)
- Pd(PPh₃)₂Cl₂: bis(triphenylphosphine)palladium(II) dichloride
- PPh₃: triphenylphosphine
- (*i*-PrO)₃B: triisopropyl borate
- *i*-PrOH: isopropyl alcohol
- sat: saturated
- TCCA: trichloroisocyanuric acid
- TFA: trifluoroacetic acid
- TOTU: *O*-[(ethoxycarbonyl)cyanomethyl-enamino]-*N*,*N*,*N*',*N*'-tetramethyluronium tetrafluoroborate
- THF: tetrahydrofurane

### Compound Preparation

The general procedures used to synthesise the compounds of Formula I are described in reaction Schemes 1-4 and are illustrated in the Examples.

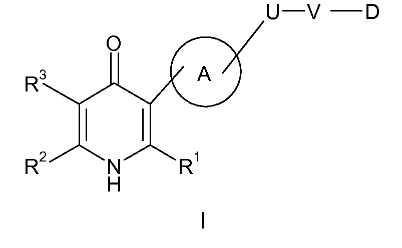

Throughout the specification, general Formulae are designated by Roman numerals I, II, III, IV etc. Subsets of compounds of Formula I are defined as Ia, Ib, Ic, Id and Ie; subsets of other Formulae are expressed in an analogous fashion.

Compounds of Formula (Ia), which are compounds of Formula I wherein E, R¹, R², R³, A and Cy are as defined according to Formula (I); U represents 1-propyn-1-yl; and W represents 0 or -CH₂-; wherein the optional substituents are as defined in Formula (I), may be prepared from compounds of Formula (II), 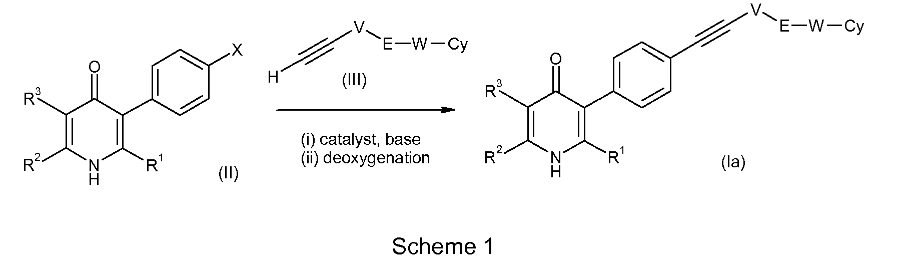 wherein R¹, R², R³ are as defined according to Formula (I) and X is a halo, for example bromo, or triflate (trifluoromethanesulfonyloxy) group, according to Scheme 1 by cross-coupling reaction with a compound of Formula (III) wherein V, E, W and Cy are as defined above for compounds of Formula (1 a), in the presence of a catalyst, for example copper (I) iodide, and a suitable palladium catalyst, for example bis(triphenylphosphine)palladium(II)chloride, in the presence of a base, for example triethylamine, in an inert atmosphere, and at an elevated temperature, for example 100 - 120°C.

Alternatively, compounds of Formula (1a) may be prepared from compounds of Formula (II) by reaction with a compound of Formula (III) wherein V, E, W and Cy are as defined according to Formula (Ia), in the presence of a base, for example pyrrolidine, and a suitable palladium catalyst, for example bis(triphenylphosphine)palladium(II)chloride, in the presence of triphenylphosphine, in an inert atmosphere.

Compounds of Formula (I) may be prepared from compounds of Formula (IV) 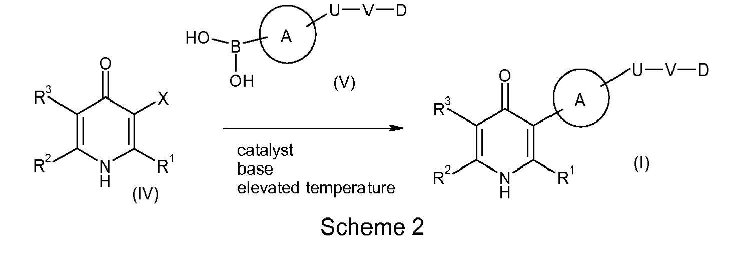 wherein R¹, R², R³ are as defined according to Formula (I) and X is halo, for example iodo, or triflate group, according to Scheme 2 by reaction with a compound of Formula (V) wherein A, V and D are as defined in Formula (I), and U is as defined in Formula (I) except for -C₂alkynyl-, in the presence of a base, for example sodium carbonate or potassium carbonate, and a suitable catalyst, for example Pd(OAC)₂ and tetrabutylammonium bromide, in a suitable solvent, for example, DMF, water or ethanol, at an elevated temperature, for example 100-160°C, in an inert atmosphere.

Compounds of Formula (Ib), which are compounds of Formula I wherein R¹, R², R³, R⁴ and Cy are as defined according to Formula (I); A represents phenyl; U represents 1-propyn-1-yl; V represents -CH₂-; D represents E-W-Cy; E represents -NR⁴-; and W represents -CH₂-;, wherein the optional substituents are as defined in Formula (I), may be prepared from compounds of Formula (Ib)', 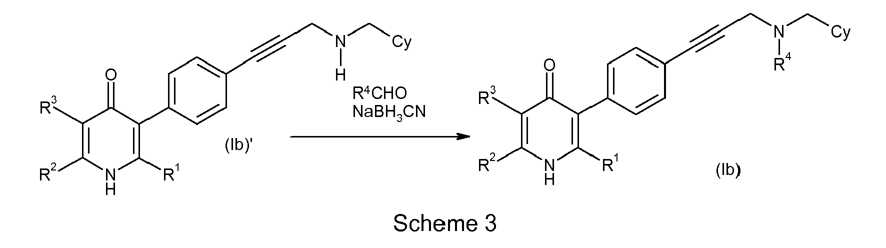 wherein R¹, R², R³, A, U, V, D, E, W and Cy are as defined above for Formula (Ib) and R⁴ is hydrogen, according to Scheme 3 by reductive amination using an appropriate reducing agent, for example sodium cyanoborohydride, with the appropriate aldehyde, R⁴CHO, wherein R⁴ is as defined in Formula (I), in an appropriate solvent, for example methanol, dichloromethane, DCE or mixtures thereof, at ambient temperature, in an inert atmosphere.

Compounds of Formula (1c), which are compounds of Formula (I) wherein R¹, R², R³ and R⁴, W and Cy are as defined according to Formula (I); A represents phenyl; U represents 1-propyn-1-yl; V represents -CH₂-; D represents E-W-Cy; E represents -NR⁴- or 5-7-membered saturated or partially saturated heterocyclic ring containing one or two nitrogen atoms; may be prepared from compounds of Formula (Ic)' 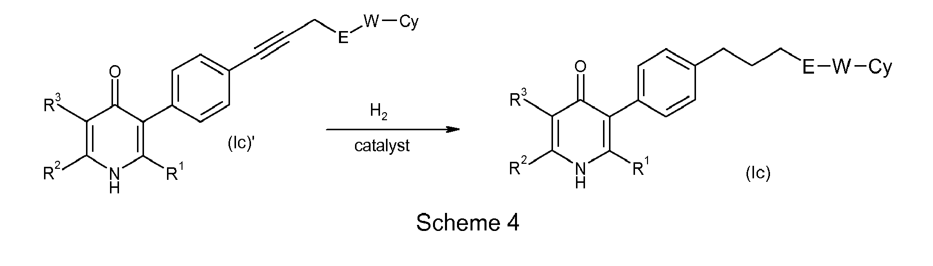 wherein R¹, R², R³, E, W and Cy are as defined above for Formula (I) according to Scheme 4, by hydrogenation using H₂ and an appropriate palladium catalyst, for example palladium 10% w/w on activated charcoal, under pressure, in an appropriate solvent, for example methanol, at ambient temperature, in an inert atmosphere.

Compounds of Formula (I) as defined above may be prepared from compounds of Formula (VI) 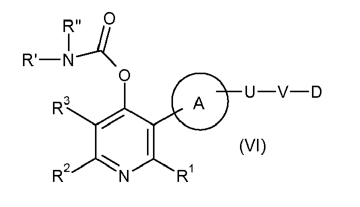 wherein R¹, R², R³, A, U, V and D are as defined for Formula (I) and R' and R" are independently C₁₋₆alkyl, for example isopropyl, by reacting with an acid, for example hydrochloric acid, in a suitable solvent, for example methanol, at an elevated temperature, for example 100-110°C, in an inert atmosphere.

Compounds of Formula (VI) may be prepared from compounds of Formula (VII) 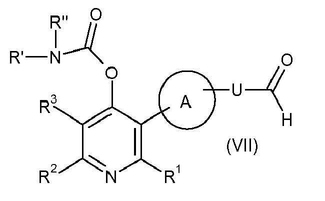 wherein R¹, R², R³, A, U and R' and R" are as defined for compounds of Formula (VI), by a reductive amination reaction with a compound of Formula (VIII)

V-E-W-Cy (VIII)

wherein V, E, W and Cy are as defined in Formula (I), in a suitable solvent, for example dichloroethane, THF or mixtures of both, together with a suitable reducing agent, for example sodium cyanoborohydride, in the presence of a suitable acid, for example acetic acid, (or treatment with NaHB(OAc)₃ in a suitable solvent such as a mixture of DCE and THF) at an ambient temperature.

Compounds of Formula I may be prepared directly from compounds of Formula (XX), which are compounds of Formula (VII) without a protecting group on the hydroxy group of the pyridinol, by reacting with a compound of Formula (VIII) as defined above, under similar conditions to those described for the reductive amination reaction above.

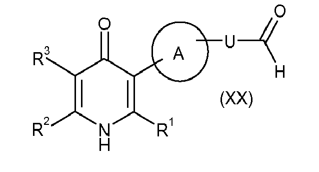

Compounds of Formula (VII) may be prepared from compounds of Formula (IX) 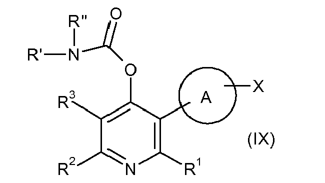 wherein R¹, R², R³ and A are as defined for Formula (I), R' and R" are independently C₁₋₆alkyl, for example isopropyl, and X is a leaving group such as halo or OTf, by reacting with a compound of Formula (X) 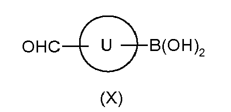 wherein U is as defined for Formula (I), in a suitable solvent, for example toluene, ethanol or a mixture of both, in the presence of a catalyst, for example bis(triphenylphosphine)palladium(II)chloride, in the presence of a base, for example sodium hydrogen carbonate, at an ambient temperature. Alternatively, the same reaction may be performed with a compound of Formula (X'), which is a compound of Formula (X) in a lower oxidation state (hydroxymethyl rather than aldehyde) then the resulting compound converted to the desired compound of Formula (VII) using an oxidation step such as treatment with manganese dioxide in suitable solvent such as a mixture of DCM and methanol.

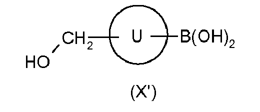

Compounds of Formula (XX) may be prepared by a reaction between compounds of Formula (II) and compounds of Formula (X) under similar conditions to those described above for the reaction between (IX) and (X).

Compounds of Formula (VI) may alternatively be prepared directly from compounds of Formula (IX), wherein R¹, R², R³ and A are as defined for Formula (I) and R' and R" are independently C₁₋₆alkyl, for example isopropyl, by reacting with a compound of Formula (XIX), wherein U, V and D are as defined for Formula (I), in the presence of a suitable palladium catalyst, such as PdCl₂(dppf) and a suitable base, for example Ba(OH)₂, in a suitable solvent such as a mixture of DME and water, at elevated temperature.

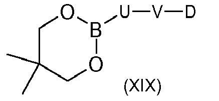

Compounds of Formula (XIX) may be prepared from 4-{4-[(trifluoromethyl)oxy]phenyl}piperidine according to standard procedures, for example according to the method described in WO 2006/067216 (page 41), or treating 4-{4-[(trifluoromethyl)oxy]phenyl}piperidine with a suitable base such as N,N-diisopropylethylamine and 4-(5,5-dimethyl-1,3,2-dioxaborinan-2-yl)benzaldehyde in a suitable solvent such as THF.

Compounds of Formula (IX), wherein R¹ or R² is hydroxymethyl, may be prepared from compounds of Formula (IX) wherein R¹ or R² is methyl, by an oxidation reaction using a suitable oxidising agent such as m-CPBA, followed by treatment with a suitable reagent such as acetic anhydride at elevated temperature such as at 130-150°C, followed by treatment with a suitable base such as NaOH in a s suitable solvent such as MeOH.

Compounds of Formula (IX) may be prepared from compounds of Formula (II), by reacting with a compound of Formula (XI) 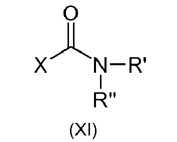 wherein R' and R" are independently C₁₋₆alkyl, for example isopropyl, and X is a halo, for example chloro, in a suitable solvent, for example pyridine, at an elevated temperature, for example 80-90°C.

Compounds of Formula (XI) are commercially available or may be synthesised according to literature methods.

Compounds of Formula (II) may be prepared from compounds of Formula (XII) 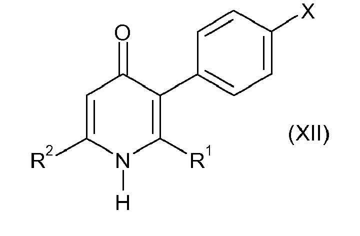 wherein R¹ and R² are as defined for Formula (I) and X is a halo, for example bromo, by reacting with a suitable halogenating agent, in a suitable solvent, for example dichloromethane, methanol or mixtures thereof, under elevated temperature. For example, when X is chloro, the halogenating agent may be N-chlorosuccinimide.

Compounds of Formula (XII) may be prepared from compounds of Formula (XIII) 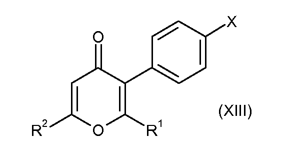 wherein R¹ and R² are as defined for Formula (I) with an optional protecting group and X is halo, for example bromo, by reacting with aqueous ammonia in a suitable solvent, for example methanol or ethanol, (or using ammonium hydroxide in methanol) at an elevated temperature, for example 130-150°C.

Compounds of Formula (XIII) may be prepared from compounds of Formula (XIV) 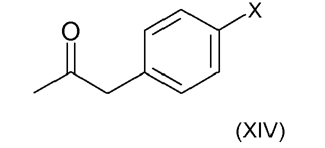 wherein and X is a halo, by reacting it with a reagent such as Eaton's reagent or other dehydrating agents, for example polyphosphoric acid, in the presence of acetic anhydride, at an elevated temperature, for example, 90-100°C, in an inert atmosphere.

Compounds of Formula (XIV) are commercially available or may be synthesised using literature procedures.

Compounds of Formula (III) wherein V, E, W and Cy are as defined for Formula (I), may be prepared from compounds of Formula (XV)

H≡V-E-W (XV)

by reacting with a compound Cy-CHO, using an appropriate reducing agent, for example sodium cyanoborohydride, in the presence of a suitable organic acid, for example acetic acid, in a suitable solvent, for example acetonitrile, at an ambient temperature, in an inert atmosphere.

Compounds of Formula (XV) are commercially available or may be synthesised using methods found in the literature.

Compounds of Formula (IV) are commercially available or may be synthesised using methods found in the literature, for example WO 2006/094799.

Compounds of Formula (V) wherein A, U V and D are as defined in Formula (I), may be prepared by compounds of Formula (XVI) 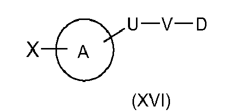 wherein A, U, V and D are as defined in Formula (I) and X is a halo, for example , by reaction with n-butyl lithium followed by a boronating agent, for example triisopropylborate, in a suitable solvent, for example THF, at a reduced temperature, for example -78°C, followed by hydrolysis with hydrochloric acid, in an inert atmosphere.

Compounds of Formula (XVI) wherein A, U, V and D are as defined in Formula (I) and X is halo, may be prepared from compounds of Formula (XVII)

H-U-V-E-W-Cy (XVII)

wherein E, W and Cy are as defined in Formula (I), by reaction with compounds of Formula (XVIII) 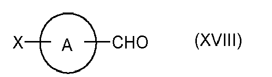 wherein A is as defined in Formula (I) and X is halo, using an appropriate reducing agent, for example sodium cyanoborohydride, in the presence of a suitable acid, for example acetic acid, at an ambient temperature, in an inert atmosphere.

Compounds of Formula (XVI) may alternatively be prepared from compounds of Formula (XXI),

E-W-Cy (XXI)

wherein E, W and Cy are as as defined in Formula (I), by reaction with compounds of Formula (XXII), wherein A, U and V are as defined in Formula (I), X is halo, and Y is a leaving group such as OTf, in the presence of a suitable solvent such as DMF.

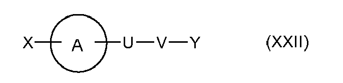

Compounds of Formulae (XVII), (XVIII), (XXI) and (XXII) are available commercially or may be synthesised using literature methods.

It will be readily apparent to those skilled in the art that further compounds of Formula I may be prepared using methods analogous to those outlined above, or by reference to the experimental procedures detailed in the Examples provided herein. For example, the reaction to convert pyranone compounds (XIII) to pyridinone compounds (XII) may be carried out at a later stage in the synthesis of compounds of Formula (I). Similarly the halogenation step to convert compounds (XII) to compounds (II) may be carried out at an earlier or later stage in the synthesis of compounds of Formula (I). The choice of order of the synthetic steps will depend on a number of factors, such as compatibilty of the resulting subsituents with subsequent reaction conditions.

Those skilled in the art will appreciate that in the preparation of the compound of Formula I or a pharmaceutically acceptable derivative thereof, it may be necessary and/or desirable to protect one or more sensitive groups in the molecule or the appropriate intermediate to prevent undesirable side reactions. Suitable protecting groups for use according to the present invention are well known to those skilled in the art and may be used in a conventional manner. See, for example, "Protective groups in organic synthesis" by T.W. Greene and P.G.M. Wuts (John Wiley & sons 1991) or "Protecting Groups" by P.J. Kocienski (Georg Thyme Vela 1994). Examples of suitable amino protecting groups include acyl type protecting groups (e.g. formal, trifluoroacetyl, acetyl), aromatic urethane type protecting groups (e.g. benzyloxycarbonyl (Cbz) and substituted Cbz), aliphatic urethane protecting groups (e.g. 9-fluorenylmethoxycarbonyl (Fmoc), t-butyloxycarbonyl (Boc), isopropyloxycarbonyl, cyclohexyloxycarbonyl) and alkyl or aralkyl type protecting groups (e.g. benzyl, trityl, chlorotrityl). Examples of suitable oxygen protecting groups may include for example alky silyl groups, such as trimethylsilyl or *tert*butyldimethylsilyl; alkyl ethers such as tetrahydropyranyl or *tert*-butyl; or esters such as acetate.

### Examples

The following examples illustrate the invention. These examples are not intended to limit the scope of the invention, but rather to provide guidance to the skilled artisan to prepare and use the compounds, compositions, and methods of the invention. While particular embodiments of the invention are described, the skilled artisan will appreciate that various changes and modifications can be made without departing from the spirit and scope of the invention.

### Intermediate 1

### Methyl({4-[(trifluoromethyl)oxy]phenyl}methyl)amine hydrochloride (N-Methyl-1-{4-[(trifluoromethyl)oxy]phenyl}methanamine hydrochloride)

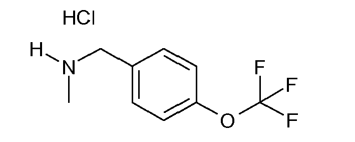

To a solution of 5.0 g of 4-(trifluoromethoxy)benzaldehyde in 40 ml of MeOH were added 4.9 ml of 40% aqueous MeNH₂. The mixture was cooled to 0°C, then 497 mg of NaBH₄ were added portionwise. The reaction mixture was allowed to stir at room temperature overnight, the organic solvents were evaporated and the resulting aqueous mixture extracted with CH₂Cl₂. The combined organic layers were dried over Na₂SO₄, filtered and concentrated to dryness under vacuum. The resulting residue was dissolved in Et₂O, cooled at 0°C and 8.0 ml of 4.0 M HCl in dioxane were added. The precipitate was filtered off, thoroughly washed with cool Et₂O and dried under vacuum to give 5.176 g (81%) of the title compound as a white solid.
¹H NMR (δ, ppm, CDCl₃): 9.96 (br s, 2H), 7.64 (d, 2H); 7.25 (d, 2H); 4.05 (t, 2H); 2.51 (t, 3H). [ES MS] m/z: 206 (MH+).

### Intermediate 2

### Methyl(2-propyn-1-yl){[4-(trifluoromethyl)phenyl]methyl}amine (N-Methyl-N-{[4-(trifluoromethyl)phenyl]methyl}-2-propyn-1-amine)

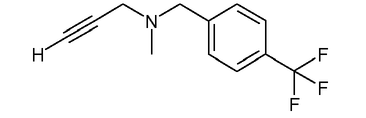

In a round bottom flask under argon atmosphere were placed 1.03 g of 4-(trifluoromethyl)benzaldehyde, 7 ml of dry CH₃CN, 200 mg of 3A molecular sieves, 0.53 g of *N*-methyl-2-propyn-1-amine and 1.625 g of NaHB(AcO)₃ consecutively. The mixture was stirred 10 min at room temperature, then 0.30 ml of AcOH were added dropwise. The resulting mixture was stirred 45 min at room temperature. The mixture was filtered, the filter cake washed with CH₃CN and the resulting solution concentrated under vacuum. The residue was partitioned between H₂O and EtOAc, the organic layer was washed three times with 1 N HCl, the combined aqueous layers were basified with NaOH and extracted with CH₂Cl₂ three times. The combined organic layers were washed with brine, dried over Na₂SO₄, filtered and concentrated to dryness to afford 1.07 g (80%) of the title compound as a pale yellow oil.
¹H NMR (δ, ppm, CDCl₃): 7.60-7.58 (d, 2H); 7.51-7.48 (d, 2H); 3.67 (s, 2H); 3.35 (s, 2H); 2.38 (s, 3H); 2.32 (s, 1H).

Intermediates 3-5 were prepared by a method analogous to that described for Intermediate 2 replacing 4-(trifluoromethyl)benzaldehyde with the commercial aldehyde shown in Table 1.

**Table 1**

| | | | | |
|---|---|---|---|---|
| 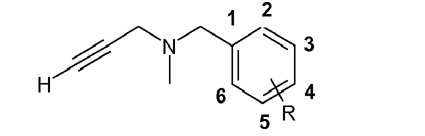 | | | | |

| **Int.** | **R** | **Starting Aldehyde** | **Chemical name** | **Physical Data** |
|---|---|---|---|---|
| 3 | 4-OCF₃ | 4-(trifluoromet hoxy)benzal dehyde | Methyl(2-propyn-1-yl)({4-[(trifluoromethyl)oxy]phenyl} methyl)amine. (*N*-Methyl-*N*-({4-[(trifluoromethyl)oxy]phenyl} methyl)-2-propyn-1-amine) | ¹H NMR (δ, ppm, CDCl₃): 7.38 (d, 2H); 7.17 (d, 2H); 3.58 (s, 2H); 3.32 (d, 2H); 2.35 (s, 3H); 2.29 (t, 1H). |
| 4 | 2-CF₃ | 2-(trifluoromet hyl)benzalde hyde | Methyl(2-propyn-1-yl){[2-(trifluoromethyl)phenyl]meth yl}amine. *(N*-Methyl-*N*-{[2-(trifluoromethyl)phenyl]meth yl}-2-propyn-1-amine) | ¹H NMR (δ, ppm, CDCl₃): 7.78-7.75 (d, 1H); 7.65-7.62 (d, 1H); 7.55-7.50 (t, 1H); 7.37-7.32 (t, 1H); 3.76 (s, 2H); 3.38-3.37 (d, 2H); 2.35 (s, 3H); 2.29-2.27 (t, 1H). |
| 5 | 2,4-CF₃ | 2,4-bis(trifluorom ethyl)benzal dehyde | {[2,4-Bis(trifluoromethyl)phenyl]m ethyl}methyl(2-propyn-1-yl)amine. *(N*-{[2,4-Bis(trifluoromethyl)phenyl]m ethyl}-*N*-methyl-2-propyn-1-amine) | ¹H NMR (δ, ppm, CDCl₃): 7.99-7.96 (d, 1H); 7.89 (s, 1H); 7.80-7.77 (d, 1H); 3.81 (s, 2H); 3.38 (s, 2H); 2.35 (s, 3H); 2.29 (s, 1H). |

### Intermediate 6

### Propyl(2-propyn-1-yl)({4-[(trifluoromethyl)oxy]phenyl}methyl)amine (N-Propyl-N-({4-[(trifluoromethyl)oxy]phenyl}methyl)-2-propyn-1-amine)

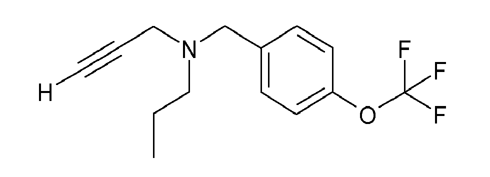

To a solution of 4-(trifluoromethoxy)benzaldehyde (ALDRICH, 1.167 g) in DCE (25 ml) were added propylamine (ALDRICH, 0.458 ml), AcOH (PANREAC, 0.547 ml) and NaHB(AcO)₃ (ALDRICH, 2.92 g) consecutively. The mixture was stirred at room temperature for 3 days, then diluted with CH₂Cl₂ and washed successively with 1 N NaOH and 1 N HCl. The organic layer was dried over Na₂SO₄, filtered and evaporated to dryness under vacuum. Purification by column chromatography on silica gel, eluting with mixtures CH₂Cl₂/MeOH v/v 100:0 and 20:1 gave 0.663 g of propanamine intermediate which was dissolved in dry CH₃CN under inert atmosphere and K₂CO₃ (ALDRICH, 0.773 g) and propargyl bromide (FLUKA, 0.342 ml) were added. The mixture was stirred at room temperature for 18 h, then diluted with EtOAc and washed with H₂O. The organic layer was dried over Na₂SO₄, filtered and evaporated to dryness under vacuum. Purification by column chromatography on silica gel, eluting with CH₂Cl₂ gave 0.395 g of the title compound as a yellow oil.
¹H NMR (δ, ppm, CDCl₃): 7.38 (d, 2H); 7.16 (d, 2H); 3.62 (s, 2H); 3.31 (d, 2H); 2.50 (t, 2H); 2.22 (t, 1H); 1.53 (m, 2H); 0.92 (t, 3H). [ES MS] m/z 272 (MH+).

### Intermediate 7

### Cyclopropyl(2-propyn-1-yl)({4-[(trifluoromethyl)oxy]phenyl}methyl)amine (N-2-Propyn-1-yl-N-({4-[(trifluoromethyl)oxy]phenyl}methyl)cyclopropanamine)

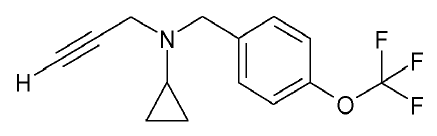

Intermediate 7 was prepared by a method analogous to that described for Intermediate 6 replacing propylamine with cyclopropylamine.
¹H NMR (δ, ppm, CDCl₃): 7.33 (d, 2H); 7.14 (d, 2H); 3.83 (s, 2H); 3.30 (d, 2H); 2.27 (t,
1H); 2.15 (m, 1H); 0.57-0.51 (m, 2H); 0.46 (m, 2H). [ES MS] m/z 270 (MH+).

### Intermediate 8

### 2-Propyn-1-yl({4-[(trifluoromethyl)oxy]phenyl}methyl)amine (N-({4-[(Trifluoromethyl)oxy]phenyl}methyl)-2-propyn-1-amine)

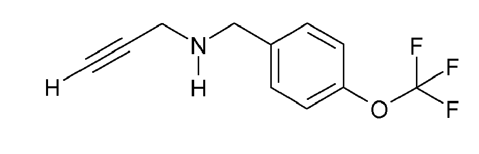

To a solution of 0.216 ml of propargyl amine and 500 mg of 4-[(trifluoromethyl)oxy]benzaldehyde in 5.0 ml of DCE were added 670 mg of NaHB(AcO)₃ and 2.5 ml of DCE. The mixture was stirred overnight. Because the reaction had not taken place; additional 0.216 ml of propargyl amine, 5.0 ml of MeOH and 0.12 g of NaBH₄ were added. The mixture was stirred for 3 h 20 min and additional 0.06 g of NaBH₄ were added in order to complete the reaction. After 1 h 50 min the mixture was concentrated under vacuum and was added H₂O and EtOAc. The layers were separated, the aqueous layer was extracted with EtOAc two times, and the combined organic layers were washed with brine, dried over Na₂SO₄, filtered and concentrated to dryness. The crude was purified by chromatography on silica gel eluting with EtOAc-Hex 10:90 to 50:50 to afford 0.428 mg (71%) of the title compound as a colourless oil.
¹H NMR (δ, ppm, CDCl₃): 7.39 (d, 2H); 7.18 (d, 2H); 3.89 (s, 2H); 3.43 (d, 2H); 2.27 (t, 1H). [ES MS] m/z: 230 (MH+).

### Intermediate 9

### [2-(Methyloxy)ethyl]2-propyn-1-yl({4-r(trifluoromethyl)oxy]phenvnmethyl)amine (N-[2-(Methyloxx)ethyl]-N-({4-[(trifluoromethyl)oxy]phenyl}methyl)-2-propyn-1-amine)

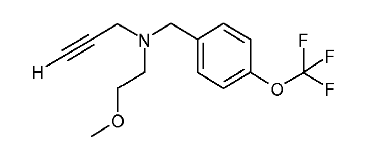

To a solution of 0.188 g of Intermediate 8 in 3.0 ml of CH₃CN under N₂ atmosphere were added Nal (0.061 g), 0.227 g of K₂CO₃ and 0.093 ml of 2-bromoethyl-methyl-ether consecutively. The mixture was stirred overnight at 70°C. Then additional 0.093 ml of 2-bromoethyl-methyl-ether and the resulting mixture heated at 80°C until completion. The reaction was filtered off, the filter cake washed with CH₂Cl₂ and the resulting solution concentrated to dryness. The crude was purified by chromatography on silica gel eluting with EtOAc-Hex 2:98 to 90:10 to afford 0.223 g (94%) of the title compound as a colourless oil.
¹H NMR (δ, ppm, CDCl₃): 7.40 (d, 2H); 7.16 (d, 2H); 3.69 (s, 2H); 3.52 (t, 2H); 3.40 (d, 2H); 3.35 (s, 3H); 2.77 (t, 2H); 2.25 (t, 1H).

### Intermediate 10

### 1,1-Dimethylethyl 4-hydroxy-4-{4-[(trifluoromethyl)oxy]phenyl}-1-piperidinecarboxylate

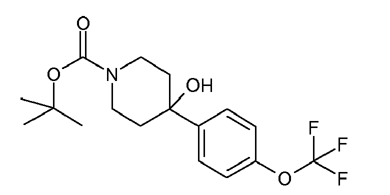

A solution of 0.893 ml of 1-bromo-4-[(trifluoromethyl)oxy]benzene in 6.0 ml of dry THF under argon atmosphere was cooled to -78°C and 3.6 ml of n-BuLi 2.0 M were added. The reaction mixture was stirred at -78°C for 45 min and then was added a solution of 1.437 g of 1,1-dimethylethyl-4-oxo-1-piperidinecarboxylate in 6.0 ml of THF. The reaction mixture was stirred at -78°C for 1 h, then was quenched with 10 ml of mixture 1:1 of sat. NH₄Cl and H₂O. This mixture was stirred at room temperature and extracted with EtOAc three times (3 x 20 ml). The combined organic layers were washed with brine, dried over Na₂SO₄, filtered and concentrated to dryness. The residue was purified by chromatography on silica gel eluting with Et₂O-Hex 10:90 to 50:50 to afford 1.802 mg (83%) of the title compound as a white solid.
¹H NMR (δ, ppm, CDCl₃): 7.50 (d, 2H); 7.21 (d, 2H); 4.05 (br d, 2H); 3.23 (td, 2H); 1.98 (td, 2H); 1.71 (d, 2H); 1.48 (s, 9H). [ES MS] m/z: 262 (MH+).

### Intermediate 11

### 4-{4-[(Trifluoromethyl)oxy]phenyl}-1,2,3,6-tetrahydropyridine.

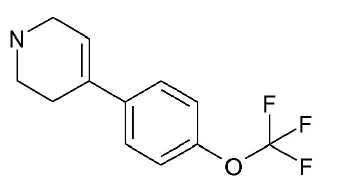

To a solution of 1.80 g of Intermediate 10 in 13 ml of AcOH were added 6.5 ml of conc. HCl. The reaction mixture was heated to 80 - 90°C until completion, then it was poured onto 150 g of ice. The mixture was made basic with NaOH (until approx. pH=13) and was extracted with CH₂Cl₂ three times. The combined organic layers were dried over Na₂SO₄, filtered, concentrated to dryness to give 1.05 g (87%) of the crude compound as a yellow solid that was used without further purification.
¹H NMR (δ, ppm, CDCl₃): 7.39 (d, 2H); 7.16 (d, 2H); 6.14-6.11 (m, 1H); 3.54 (q, 2H); 3.11 (t, 2H); 2.47-2.41 (m, 2H).

### Intermediate 12

### 4-(4-Bromophenyl)-1,2,3,6-tetrahydropyridine

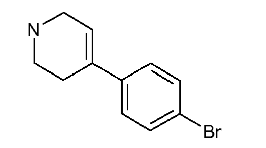

Intermediate 12 was prepared by a method analogous to that described for Intermediate 11 replacing Intermediate 10 with 4-(4-bromophenyl)-4-piperidinol (ALDRICH).
¹H NMR (δ, ppm, CD₃OD): 7.46-7.43 (d, 2H); 7.33-7.31 (d, 2H); 6.18 (s, 1H); 3.43 (s, 2H); 3.02 (s, 2H); 2.44 (s, 2H). [ES MS] m/z: 238 (MH+).

### Intermediate 13

### 4-{4-[(Trifluoromethyl)oxy]phenyl}piperidine.

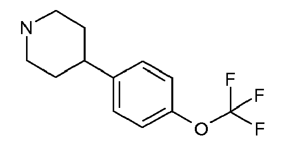

In a hydrogenation reactor was dissolved 1.0 g of Intermediate 11 in 20 ml of MeOH and 6.2 ml of 1 N HCl under N₂ atmosphere. Then were added 0.219 g of palladium 10% w/w on activated charcoal and the mixture was hydrogenated at 3 atm for 17 h 15 min. The catalyst was removed by filtration and the resulting solution was made basic with 2N NaOH and extracted with EtOAc. The combined organic layers were washed with brine, dried over Na₂SO₄, filtered and concentrated to dryness to give 0.783 g (78%) of the crude compound as a yellow oil that was used without further purification.
¹H NMR (δ, ppm, CDCl₃): 7.23 (d, 2H); 7.14 (d, 2H); 3.21 (br d, 2H); 2.75 (td, 2H); 2.63 (tt, 1H); 1.89-1.80 (m, 3H); 1.63 (qd, 2H). [ES MS] m/z: 246 (MH+).

### Intermediate 14

### 4-Phenyl-1-(2-propyn-1-yl)piperidine

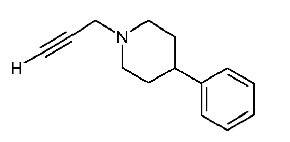

To a solution of 2.53 g of 4-phenylpiperidine in 120 ml of CH₃CN under N₂ atmosphere were added 2.61 g of K₂CO₃. The mixture was cooled to 0°C and 1.415 ml of propargyl bromide were added. The reaction was stirred overnight at room temperature, then EtOAc and H₂O were added. The layers were separated, the aqueous layer was extracted with EtOAc two times, and the combined organic layers were washed with brine, dried over MgSO₄, filtered and concentrated to dryness. The crude was purified by chromatography on silica gel eluting with MeOH-CH₂Cl₂ 0.5:99.5 to 5:95 to afford 2.415 g (77%) of the title compound as a pale brown solid.
¹H NMR (δ, ppm, CDCl₃): 7.33-7.18 (m, 5H); 3.38 (d, 2H); 3.04 (d, 2H); 2.56-2.46 (m, 1H); 2.42-2.33 (m, 2H); 2.28 (t, 1H); 1.90-1.82 (m, 4H). [ES MS] m/z: 200 (MH+).
Intermediates 15-19 were prepared by methods analogous to that described for Intermediate 14 replacing 4-phenylpiperidine with the appropriate amine shown in Table 2.

**Table 2**

| | | | | | |
|---|---|---|---|---|---|
| 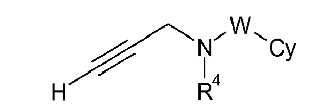 | | | | | |

| **Int.** | **-W-Cy** | **R⁴** | **Starting amine** | **Chemical name** | **Physical Data** |
|---|---|---|---|---|---|
| 15^{a} | 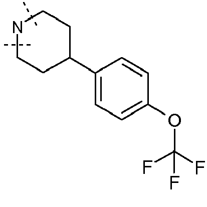 | | Intermediate 13 | 1-(2-Propyn-1-yl)-4-{4-[(trifluoromethyl)oxy] phenyl}piperidine. | ¹H NMR (δ, ppm, CDCl₃): 7.24 (d, 2H); 7.14 (d, 2H); 3.38 (d, 2H); 3.03 (br d, 2H); 2.58-2.47 (m, 1H); 2.38 (td, 2H); 2.28 (t, 1H); 1.90-1.75 (m, 4H). [ES MS] m/z: 284 (MH+). |
| 16 | Bn | Bn | bis(phenylm ethyl)-amine | *N,N-* Bis(phenylmethyl)-2-propyn-1-amine. Bis(phenylmethyl)2-propyn-1-ylamine | ¹H NMR (δ, ppm, CDCl₃): 7.42-7.48 (m, 10H); 3.69 (s, 4H); 3.27 (s, 2H); 2.29 (s, 1H). [ES MS] m/z: 236 (MH+). |
| 17 | 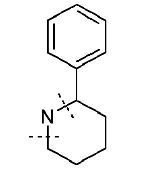 | | 2-phenylpiperi dine | 2-Phenyl-1-(2-propyn-1-yl)piperidine. | ¹H NMR (δ, ppm, CDCl₃): 7.38-7.24 (m, 5H); 3.27 (dd, 1H); 3.18 (dd, 1H); 3.08 (dd, 1H); 3.00 (d, 2H); 2.61-2.52 (m, 1H); 2.20 (t, 1H); 1.83-1.56 (m, 5H); 1.46-1.35 (m, 1H). |
| 18^{a} | 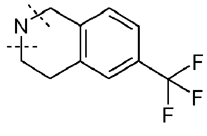 | | 6-(trifluorometh yl)-1,2,3,4-tetrahydroiso quinoline | 2-(2-Propyn-1-yl)-6-(trifluoromethyl)-1,2,3,4-tetrahydroisoquinoline. | ¹H NMR (δ, ppm, CDCl₃): 7.37-7.36 (m, 2H); 7.15 (d, 1H); 3.80 (s, 2H); 3.54 (d, 2H); 2.99 (t, 2H); 2.86 (t, 2H); 2.29 (t, 1H). |
| 19^{a} | 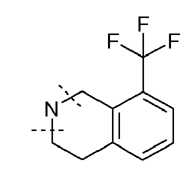 | | 8-(trifluorometh yl)-1,2,3,4-tetrahydroiso quinoline | 2-(2-Propyn-1-yl)-8-(trifluoromethyl)-1,2,3,4-tetrahydroisoquinoli ne. | ¹H NMR (δ, ppm, CDCl₃): 7.47 (d, 1H); 7.31-7.20 (m, 2H); 3.93 (s, 2H); 3.57 (d, 2H); 3.03 (t, 2H); 2.88 (t, 2H); 2.31 (t, 1H). [ES MS] m/z: 240 (MH+). |

| | | | | | |
|---|---|---|---|---|---|
| ⁸ Cs₂CO₃ was used instead K₂CO₃ as base and acetone instead CH₃CN as solvent. | | | | | |

### Intermediate 20

### 1-(1,1-Dimethyl-2-propyn-1-yl)-4-phenylpiperidine.

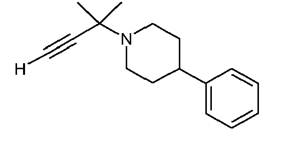

In a round bottom flask were placed 242 mg of 4-phenylpiperidine, 1.0 mg of Cu(0), 1.5 ml of a 40% aqueous KOH and 29 µL of the 3-chloro-3-methyl-1-butyne consecutively. This mixture was stirred at room temperature, six consecutive additions of 58 µL of the 3-chloro-3-methyl-1-butyne and 0.5 ml of a 40% aqueous KOH were added (each 8 h approx.) for 3 days. The reaction mixture was diluted with H₂O and was extracted three times with EtOAc. The combined organic layers were washed with brine, dried over Na₂SO₄, filtered and concentrated to dryness. The crude was purified by chromatography on silica gel eluting with Et₂O-Hex 10:90 to 50:50 to afford 200 mg (59%) of the title compound as a white solid.
¹H NMR (δ, ppm, CDCl₃): 7.33-7.18 (m, 5H); 3.23 (d, 2H); 2.53 (tt, 1H); 2.33 (s, 1H); 2.32 (td, 2H); 1.94-1.78 (m, 4H); 1.46 (s, 6H). [ES MS] m/z: 228 (MH+).

### Intermediate 21

### 1-[(4-Bromophenyl)methyl]-4-{4-[(trifluoromethyl)oxy]phenyl}piperidine.

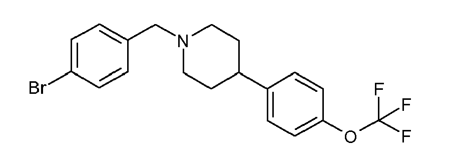

To a solution of 508 mg of Intermediate 13 in 7.2 ml of DCE under N₂ atmosphere were added 0.4 g of 4-bromobenzaldehyde, 0.30 ml of Et₃N and 0.611 g of NaHB(AcO)₃ consecutively. The mixture was stirred overnight at room temperature, then CH₂Cl₂ and 1 N NaOH were added consecutively. The layers were separated, and the aqueous layer was extracted with CH₂Cl₂, and the combined organic layers were washed with brine, dried over Na₂SO₄, filtered and concentrated to dryness. The crude was purified by chromatography on silica gel eluting with EtOAc-Hex 5:95 to 20:80 to afford 0.573 mg (77%) of the title compound as a colourless oil.
¹H NMR (δ, ppm, CDCl₃): 7.46 (d, 2H); 7.25-7.22 (m, 4H); 7.14 (d, 2H); 3.51 (s, 2H); 2.99 (br d, 2H); 2.57-2.46 (m, 1H); 2.09 (br s, 2H); 1.79 (br s, 4H).

### Intermediate 22

### [(4-Bromophenyl)methyl]methyl({4-[(trifluoromethyl)oxyl]phenyl}methyl)amine (1-(4-Bromophenyl)-N-methyl-N-({4-[(trifluoromethyl)oxy]phenyl}methyl)methanamine)

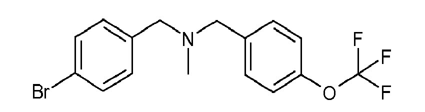

Intermediate 22 was prepared by a method analogous to that described for Intermediate 21 replacing Intermediate 13 with Intermediate 1.
¹H NMR (δ, ppm, CDCl₃): 7.45 (d, 2H); 7.38 (d, 2H); 7.25 (d, 2H); 7.17 (d, 2H); 3.49 (d,
4H); 2.17 (s, 3H). [ES MS] m/z: 374 (MH+).

### Intermediate 23

### 4-(4-Bromophenyl)-1-({4-[(trifluoromethyl)oxy]phenyl}methyl)-1,2,3,6-tetrahydropyridine.

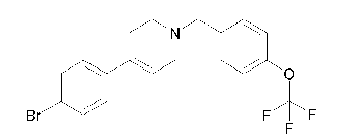

To a solution of 1.90 g of Intermediate 12 in 40 ml of DCE under argon atmosphere were added 3 g of 3A molecular sieves, 1.97 g of 4-[(trifluoromethyl)oxy]benzaldehyde and 2.54 g of NaHB(AcO)₃ consecutively. The mixture was stirred 10 min at room temperature and 1.0 ml of AcOH was added dropwise. The resulting mixture was stirred 3 h at room temperature, then was filtered off, the filter cake washed with CH₂Cl₂ and the resulting solution concentrated under vacuum. The residue was partitioned between H₂O and EtOAc, the organic layer was washed with brine, dried over Na₂SO₄, filtered, and concentrated to dryness. The crude was treated with 30ml of HCl 1N solution, the precipitate obtained was filtered, washed with hexane and dried under vacuum. The solid was treated with 25 ml NaOH 1 N solution and stirred for 15 min, extracted with EtOAc and the combined organic layers were washed with brine, dried over Na₂SO₄, filtered and concentrated to dryness to give 2.73 g (83%) of the title compound as a yellow oil.
¹H NMR (δ, ppm, CDCl₃): 7.44-7.40 (m, 4H); 7.26-7.17 (m, 4H); 6.06 (m, 1H); 3.65 (s, 2H); 3.17-3.16 (d, 2H); 2.74-2.71 (t, 2H); 2.54 (s, 2H).

### Intermediate 24

### 1-[3-(3-Bromophenyl)-2-propyn-1-yl]-4-{4-[(trifluoromethyl)oxy]phenyl}piperidine.

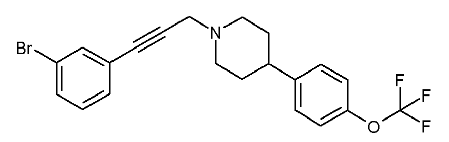

To a solution of 0.10 g of Intermediate 15 in 2.0 ml of Et₃N under N₂ atmosphere were added 0.0094 g of Cul, 0.035 g of Pd(PPh₃)₂Cl₂ and 0.069 ml of 1-bromo-3-iodobenzene consecutively. The reaction mixture was heated to room temperature for 2 h 40 min, then poured onto H₂O and extracted with CH₂Cl₂ three times. The combined organic layers were dried over Na₂SO₄, filtered and concentrated to dryness and the residue was purified by chromatography on silica gel eluting with EtOAc-Hex 5:95 to 30:70 to afford 0.209 g (97%) of the title compound as a red-brown oil.
¹H NMR (δ, ppm, CDCl₃): 7.61 (s, 1H); 7.46 (d, 1H); 7.39 (d, 1H); 7.26-7.14 (m, 5H); 3.63 (s, 2H); 3.14 (d, 2H); 2.59-2.46 (m, 3H); 1.91 (br s, 4H).

### Intermediate 25

### 5-(4-Bromophenyl)-2-thiophenecarbaldehyde.

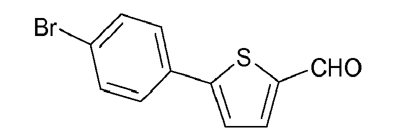

To a solution of 0.406 g of (5-formyl-2-thienyl)boronic acid and 0.566 g of 1-bromo-4-iodobenzene in 6 ml of DME and 4 ml of EtOH under N₂ atmosphere were added 6 ml of 2N Na₂CO₃ and 0.0702 g of Pd(PPh₃)₂Cl₂ consecutively. The resulting mixture was heated to 50°C for 3 h. The mixture was extracted with EtOAc, the organic layer was dried over Na₂SO₄, filtered and concentrated to dryness. The residue was purified by chromatography on silica gel eluting with EtOAc-Hex 0:100 to 10:90 to afford 322 mg (60%) of the title compound as a yellow solid.
¹H NMR (δ, ppm, CDCl₃): 9.90 (s, 1H); 7.74 (d, 1H); 7.56 (d, 4H); 7.40 (d, 1H). [ES MS] m/z: 267 (MH+).

### Intermediate 26

### {[5-(4-Bromophenyl)-2-thienyl]methyl}methyl({4[(trifluoromethyl)oxy]phenyl} methyl)amine

### (1-[5-(4-Bromophenyl)-2-thienyl]-N-methyl-N-({4-[(trifluoromethyl)oxy]phenyl} methyl)methanamine)

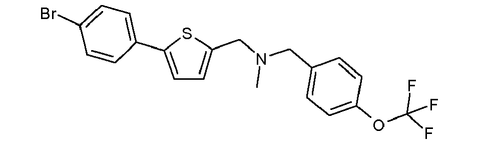

To a solution of 0.3 g of Intermediate 25 and 0.26 g of Intermediate 1 in 8.5 ml of DCE under N₂ atmosphere were added dropwise 0.226 ml of Et₃N and 0.342 g of NaHB(AcO)₃ consecutively. The mixture was stirred overnight at room temperature and 1N NaOH was added. The resulting mixture was extracted with CH₂Cl₂, the combined organic layers were dried over Na₂SO₄, filtered and concentrated to dryness. The crude was purified by chromatography on silica gel eluting with EtOAc-Hex 0:100 to 10:90 to afford 0.383 g of the desired compound with 10% of the starting aldehyde. This mixture was re-purified on silica gel eluting with EtOAc-Hex 0:100 to 10:90 to afford 0.348 g (67%) of the title compound as a colourless oil.
¹H NMR (δ, ppm, CDCl₃): 7.47 (dd, 4H); 7.42 (d, 2H); 7.18 (d, 2H); 7.15 (d, 1H); 6.90 (d, 1H); 3.75 (s, 2H); 3.58 (s, 2H); 2.28 (s, 3H). [ES MS] m/z: 456 (MH+).

### Intermediate 27

### {[5-(4-Bromophenyl)-2-furyl]methyl}methyl({4-[(trifluoromethyl)oxy]phenyl} methyl)amine

### (1-[5-(4-Bromophenyl)-2-furanyl]-N-methyl-N-({4-[(trifluoromethyl)oxy]phenyl} methyl)methanamine)

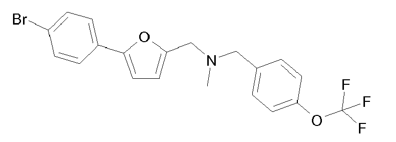

Intermediate 27 was prepared by a method analogous to that described for Intermediate 26 replacing Intermediate 25 with 5-(4-bromophenyl)-2-furancarbaldehyde.
¹H NMR (δ, ppm, CDCl₃): 7.51 (dd, 4H); 7.39 (d, 2H); 7.17 (d, 2H); 6.61 (d, 1H); 6.30 (d,
1H); 3.66 (s, 2H); 3.58 (s, 2H); 2.31 (s, 3H). [ES MS] m/z: 440 (MH+).

### Intermediate 28

### 3-Iodo-N-methyl-N-({4-[(trifluoromethyl)oxy]phenyl}methyl)benzamide.

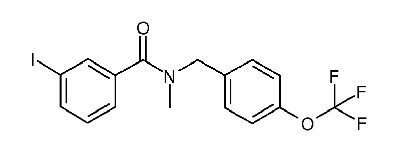

To a solution of 1.0 g of 3-iodobenzoic acid in 20 ml of CH₂Cl₂ under argon atmosphere was added via cannula a solution of 1.11 g of DCC in 22 ml of CH₂Cl₂. The resulting mixture was stirred for 1 h, then was added a solution of 0.65 g of Intermediate 1 and 0.414 ml of Et₃N in 13 ml of CH₂Cl₂. The resulting mixture was stirred overnight, diluted with Et₂O, filtered off and the filter cake washed with Et₂O. The filtrate was washed with 10% NaHCO₃ and brine, dried over MgSO₄, filtered and concentrated under vacuum. The residue was purified by chromatography on silica gel eluting with EtOAc-Hex 10:90 to 50:50 to afford 1.063 g (91 %) of the title compound as a colourless oil.
¹H NMR (δ, ppm, CDCl₃): 7.79 and 7.75 (s, 1H); 7.39 (s, 1H); 7.23 (d, 2H); 7.21-7.11 (m, 2H); 4.72 and 4.49 (s, 2H); 3.02 and 2.88 (s, 3H). [ES MS] m/z: 436 (MH+).

### Intermediate 29

### 5-Bromo-N-methyl-N-({4-[(trifluoromethyl)oxy]phenyl}methyl)-1-benzothiophene-2-carboxamide.

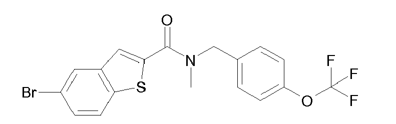

In a round bottom flask under argon atmosphere were placed 0.3 g of 5-bromo-1-benzothiophene-2-carboxylic acid, 0.459 g of TOTU (O-[(ethoxycarbonyl)cyanomethyl-enamino]-*N*,*N*,*N*',*N*'-tetramethyluronium tetrafluoroborate) and 0.423 g of Intermediate 1 in 12 ml of CH₂Cl₂. The mixture was cooled to 0 °C and 0.49 ml of Et₃N were added. The reaction mixture was stirred overnight at room temperature, then diluted with CH₂Cl₂, washed with 1 N NaOH and brine, dried over Na₂SO₄, filtered and concentrated to dryness under vacuum. The residue was purified by chromatography on silica gel eluting with EtOAc-Hex 10:90 to 20:80 to afford 0.471 mg (91%) of the titled compound as a pale yellow oil.
¹H NMR (δ, ppm, DMSO-d₆-80°C): 8.11 (s, 1H); 7.94 (d, 1H); 7.73 (s, 1H); 7.55 (dd, 1H); 7.42 (d, 2H); 7.33 (d, 2H); 4.75 (s, 2H); 3.12 (s, 3H). [ES MS] m/z: 445 (MH+).

### Intermediate 30

### [(3-Iodophenyl)methyl]methyl({4-[(trifluoromethyl)oxy]phenyl}methyl)amine (1-(3-Iodophenyl)-N-methyl)-N-({4-[(trifluoromethyl)oxy]phenyl}methyl)methanamine)

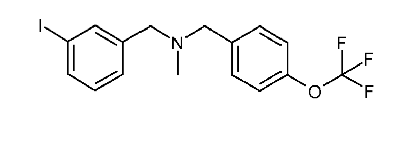

To a solution of 1.05 g of Intermediate 28 in 10 ml of THF under N₂ atmosphere were rapidly added 7.2 ml of AlMe₃ 2.0 M in hexane. The reaction was stirred at room temperature 1 h, then was heated to reflux for 2 h. Then 1.45 ml of BH₃·SMe₂ complex (10.0 M) were added at room temperature and the resulting mixture was refluxed until completion. The mixture was cooled to room temperature and water (10 ml) followed by 8 ml of 2N HCl were added and the mixture stirred for three days. The mixture was neutralized with 10% NaHCO₃ and extracted successively with EtOAc. The combined organic layers were dried over MgSO₄, filtered and concentrated to dryness to obtain 1.04 g (99%) of the crude compound as a colourless oil that was used without purification.
¹H NMR (δ, ppm, CDCl₃): 7.72 (s, 1H); 7.58 (d, 1H); 7.38 (d, 2H); 7.33 (d, 1H); 7.18 (d, 2H); 7.16 (t, 1H); 3.50 (d, 4H); 2.18 (s, 3H). [ES MS] m/z: 422 (MH+).

### Intermediate 31

### [(5-Bromo-1-benzothien-2-yl)methyl]methyl({4-[(trifluoromethyl)oxy] phenyl}methyl)-amine

### (1-(5-Bromo-1-benzothien-2-yl)-N-methyl-N-({4-[(trifluoromethyl)oxy]phenyl} methyl)methanamine)

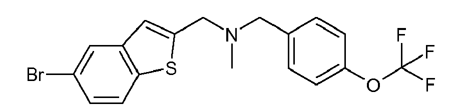

To a solution of 0.465 g of Intermediate 29 in 10 ml of anhydrous THF at room temperature under argon atmosphere were added 0.31 ml of BH₃·SMe₂ complex (10.0 M), the resulting mixture was heated to reflux until completion. The reaction was quenched by adding water and the mixture extracted with *tert*-butylmethyl ether. The organic layer were washed with brine, dried over Na₂SO₄, filtered and concentrated to dryness to obtain 0.428 mg (95%) of the crude compound as a brown oil that was used without purification.
¹H NMR (δ, ppm, CDCl₃): 7.95 (d, 1H); 7.87 (s, 1H); 7.68 (d, 1H); 7.51 (d, 2H); 7.43 (br s, 1H); 7.22 (d, 2H); 4.28 (dd, 2H); 4.02 (dd, 2H); 2.35 (br s, 3H).

### Intermediate 32

### (4-{[Methyl({4-[(trifluoromethyl)oxy]phenyl}methyl)amino]methyl}phenyl)boronic acid.

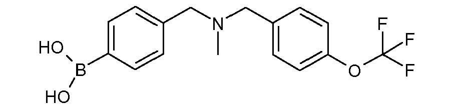

A solution of 0.76 g of Intermediate 22 and 0.58 ml of (*i*-PrO)₃B in 5.0 ml of THF under argon atmosphere was cooled to -78°C, then 1.25 ml of n-BuLi 2.4 M were added. The reaction mixture was stirred at -78°C for 2 h and then was warmed up to room temperature and stirred overnight. The reaction was quenched with 4 ml of 6 N HCl, stirred for 1 h, and then neutralized with 1N NaOH and 10% NaHCO₃. The reaction mixture was extracted with EtOAc and the combined organic layers were dried over Na₂SO₄, filtered and concentrated to dryness. The residue was purified by chromatography on silica gel eluting with MeOH-CH₂Cl₂ 0:100 to 20:80 to afford 0.549 mg (80%) of the title compound as a pale yellow oil.
¹H NMR (δ, ppm, DMSO-d₆- D₂O): 7.74 (d, 2H); 7.46 (d, 2H); 7.31-7.28 (m, 4H); 3.49 (br s, 4H); 2.05 (s, 3H). [ES MS] m/z: 340 (MH+).
Intermediates 33-36 were prepared by a method analogous to that described for Intermediate 32 using 1.1-1.5 equivalents of (*i*-PrO)₃B and *n*-BuLi and replacing Intermediate 22 with the appropriate bromine shown in Table 3.

**Table 3**

| | | | | | | | |
|---|---|---|---|---|---|---|---|
| 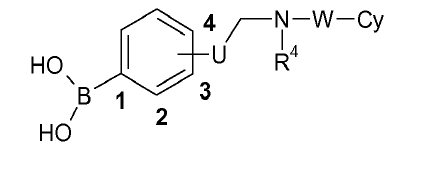 | | | | | | | |

| **Int.** | **Pos.** | **U** | **-W-Cy** | **R4** | **Starting Int.** | **Chemical name** | **Physical Data** |
|---|---|---|---|---|---|---|---|
| 33^{a} | 3 | - | 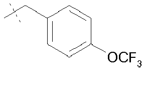 | Me | 30 | (3-([Methyl((4-[(trifluoromethyl) oxy]phenyllmeth yl)amino]methyl} phenyl)boronic acid. | ¹H NMR (δ, ppm, DMSO-d₆ - D₂O): 7.73 (d, 2H); 7.37 (d, 2H); 7.28-7.23 (m, 4H); 3.49 (s, 2H); 2.90 (d, 2H); 2.57-2.48 (m, 1H); 2.03 (t, 2H); 1.74-1.61 (m, 4H). |
| 34 | 4 | 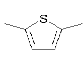 | 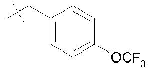 | Me | 26 | [4-(5-{[Methyl({4-[(trifluoromethyl) oxy]phenyl}meth yl)amino]methyl} -2-thienyl)phenyl]-boronic acid | ¹H NMR (δ, ppm, DMSO-d₆ - D₂O): 7.79 (d, 2H); 7.58 (d, 2H); 7.48 (d, 2H); 7.39 (d, 1H); 7.33 (d, 2H); 6.99 (d, 1H); 3.73 (s, 2H); 3.56 (s, 2H); 2.15 (s, 3H). [ES MS] m/z: 420 [M-H]⁻. |
| 35 | 4 | 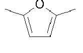 | 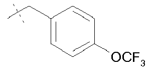 | Me | 27 | [4-(5-{[Methyl({4-[(trifluoromethyl) oxy]phenyl}meth yl)amino]methyl} -2-furanyl)phenyl]-boronic acid | ¹H NMR (δ, ppm, DMSO-d₆ - D₂O): 7.82 (d, 2H); 7.64 (d, 2H); 7.47 (d, 2H); 7.34 (d, 2H); 6.93 (d, 1H); 6.46 (d, 1H); 3.63 (br s, 4H); 2.19 (br s, 3H). |
| 36 | 4 | - | 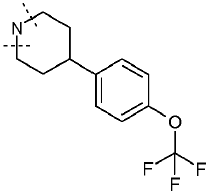 | | 21 | {4-[(4-{4-[(Trifluoromethyl )oxy]phenyl}-1-piperidinyl)meth yl]phenyl}boroni c acid. | ¹H NMR (δ, ppm, DMSO-d₆ - D₂O): 7.73 (d, 2H); 7.37 (d, 2H); 7.28-7.23 (m, 4H); 3.49 (s, 2H); 2.90 (d, 2H); 2.57-2.48 (m, 1H); 2.03 (br t, 2H); 1.74-1.61 (m, 4H). [ES MS] m/z: 380 (MH+). |
| 37 | 3 | 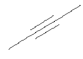 | 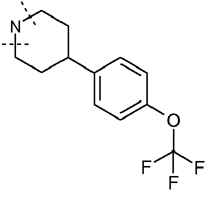 | | 24 | {3-[3-(4-{4-[(Trifluoromethyl )oxy]phenyl}-1-piperidinyl)-1-propyn-1-yl]phenyl}boroni c acid. | ¹H NMR (δ, ppm, DMSO-d₆ - D₂O): 7.83 (s, 1H); 7.73 (d, 1H); 7.46 (d, 1H); 7.38-7.30 (m, 3H); 7.25 (d, 2H); 3.52 (s, 2H); 2.94 (d, 2H); 2.58-250 (m, 1H); 2.40-2.28 (m, 2H); 1.79-1.59 (m, 4H). [ES MS] m/z: 404 (MH+). |

| | | | | | | | |
|---|---|---|---|---|---|---|---|
| ^{a} The reaction was warmed up to -35°C for 3 h | | | | | | | |

### Intermediate 38

### (2-{[Methyl({4-[(trifluoromethyl)oxy]phenyl}methyl)amino]methyl}-1-benzothien-5-yl)boronic acid.

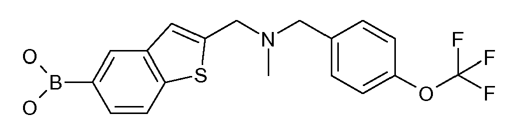

A solution of 0.408 g of Intermediate 31 in 5.0 ml of THF under argon atmosphere was cooled to -78°C, then were added 0.30 ml of (*i*-PrO)₃B and 0.54 ml of n-BuLi 1.6 M dropwise consecutively. The reaction mixture was stirred at -78°C for 1 h 30 min and then was quenched with of 6 N HCl. The mixture was warmed up to room temperature and was neutralized with saturated NaHCO₃. The resulting mixture was extracted with EtOAc twice and the combined organic layers were washed with brine, dried over Na₂SO₄, filtered and concentrated to dryness. The residue was purified by chromatography on silica gel eluting first with EtOAc-Hex 10:90 to 100:0 and then with MeOH-CH₂Cl₂ 20:80 to afford 0.245 g (65%) of the title compound as a white solid.
¹H NMR (δ, ppm, DMSO-d₆ - D₂O): 8.27 and 8.16 (s, 1H); 7.91 and 7.84 (d, 1H); 7.69 and 7.63 (d, 1H); 7.48 (d, 2H); 7.37-7.31 (m, 3H); 3.82 (s, 2H); 3.57 (s, 2H); 2.17 (s, 3H).

### Intermediate 39

### {4-[1-({4-[(Trifluoromethyl)oxy]phenyl}methyl)-1,2,3,6-tetrahydro-4-pyridinyl]phenyl}boronic acid.

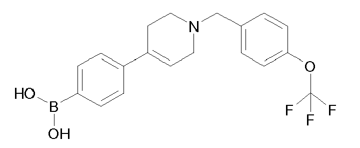

A solution of 0.500 g of Intermediate 23 and 0.364 ml of (*i*-PrO)₃B in 6.0 ml of THF was cooled to -78°C, then were added 0.856 ml of n-BuLi 1.7M, the reaction mixture was stirred at -78°C for 2 h and then was warmed up to room temperature overnight. The reaction was quenched with 1 ml of 6 N HCl. This mixture was stirred for 1 h, and then was neutralized with 1N NaOH and 10% NaHCO₃. The resulting mixture was extracted with EtOAc and the combined organic layers were dried over Na₂SO₄, filtered and concentrated to dryness. The residue was purified by chromatography on silica gel eluting with MeOH-CH₂Cl₂ 0:100 to 10:90 to afford 415 mg (90%) of the title compound as a pale yellow oil.
¹H NMR (δ, ppm, DMSO-d₆): 8.03 (s, 2H); 7.77-7.75(d, 4H); 7.42-7.39 (d, 4H); 6.20 (s, 1H); 4.09 (m, 2H); 3.16-3.15 (m, 4H). [ES MS] m/z: 378 (MH+).

### Intermediate 40

### 3-Butyn-1-yl methanesulfonate

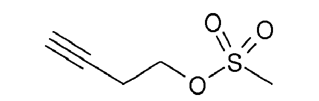

To a cooled solution (0-4°C, ice/H₂O bath) of 3-butyn-1-ol (ALDRICH, 0.76 ml) in dry CH₂Cl₂ (50 ml) were added dropwise Et₃N (FLUKA, 2.1 ml) and methanesulfonyl chloride (ALDRICH, 0.93 ml) consecutively. The mixture was stirred at room temperature for 1 h, then was quenched with 1N NH₄Cl and extracted with EtOAc. The organic layer was washed with 1N NH₄Cl (2x) and brine (1x), then dried over Na₂SO₄ and filtered. Elimination of the solvent gave 1.48 g of the title compound as a pale yellow liquid.
¹H NMR (δ, ppm, CDCl₃): 4.31 (t, 2H); 3.06 (s, 3H); 2.66 (td, 2H); 2.07 (t, 1H).

### Intermediate 41

### 3-Butyn-1-yl(methyl)({4-[(trifluoromethyl)oxy]phenyl}methyl)amine (N-Methyl-N-({4-[(trifluoromethyl)oxy]phenyl}methyl)-3-butyn-1-amine)

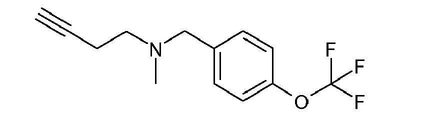

To a solution of Intermediate 1 (0.480 g) in dry acetonitrile (6 ml) under N₂ atmosphere were added K₂CO₃ (0.792 g) and Intermediate 40 (0.324 g) consecutively. The mixture was refluxed for 4 days, then concentrated to dryness under vacuum. EtOAc and H₂O were added, the organic layer washed with H₂O (2x) and brine (1x), then dried over Na₂SO₄. Elimination of the solvent under vacuum gave an oil which was purified by column chromatography on silica gel, eluting with a mixture 3% EtOAc/CH₂Cl₂ to afford 0.180 g of the title compound as a colourless liquid.
¹H NMR (δ, ppm, CDCl₃): 7.36 (d, 2H); 7.16 (d, 2H); 3.54 (s, 2H); 2.64 (t, 2H); 2.41 (td, 2H); 2.25 (s, 3H); 1.98 (t, 1H). [ES MS] m/z 258 (MH+).

### Intermediate 42

### 2-{4-[(Trifluoromethyl)oxy]phenyl}ethano)

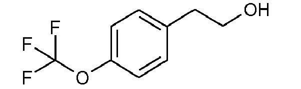

To a solution of 4-(trifluoromethoxy)phenylacetic acid (ALDRICH, 2 g) in 15 ml of dry THF and 15 ml of dry Et₂O under inert atmosphere was added LiAIH₄ (ALDRICH, 1.034 g) portionwise. The mixture was stirred at room temperature for 16 h, then Na₂SO₄·10H₂O in THF (27 ml) was added and the mixture heated to 70°C for 1 h, then filtered off. Evaporation of the solvent under vacuum gave 1.54 g of the title compound as a colourless oil.
¹H NMR (δ, ppm, CDCl₃): 7.28 (m, 2H); 7.18 (d, 2H); 3.88 (t, 2H); 2.89 (t, 2H).

### Intermediate 43

### Methyl(2-propyn-1-yl)(2-{4-[(trifluoromethyl)oxy]phenynethyl}amine (N-Methyl-N-(2-{4-[(trifluoromethyl)oxy]phenyl}ethyl)-2-propyn-1-amine)

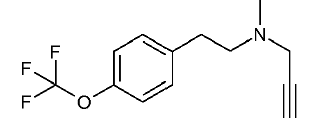

To a solution of Dess-Martin periodinane (1,1,1-tris(acetyloxy)-1,1-dihydro-1,2-benziodoxol-3-(1H)-one) (ALDRICH, 3.79 g) in CH₂Cl₂ (40 ml) was added a solution of Intermediate 42 (1.54 g) in CH₂Cl₂ (40 ml) dropwise. The mixture was stirred at room temperature under inert atmosphere for 1 h. Then were added Et₂O (30 ml), saturated NaHCO₃ (25 ml) and saturated Na₂S₂O₅ (25 ml), the resulting mixture was stirred for 25 min. The layers were separated and the aqueous one re-extracted with Et₂O. The combined organic layers were washed with NaHCO₃, H₂O and brine, dried over Na₂SO₄, filtered and concentrated to dryness to give 1.067 g of yellow oil which was dissolved in dry CH₃CN (50 ml). *N*-methylpropargylamine (ALDRICH, 0.470 g), NaHB(AcO)₃ (1.44 g), 3A molecular sieves and AcOH (1.6 ml) were added consecutively and the mixture stirred at room temperature under inert atmosphere overnight. Filtration through celite, washing with CH₂Cl₂ and evaporation of solvents gave a crude which was diluted with EtOAc and 1N HCl. The aqueous layer was basified with 1N NaOH and extracted with CH₂Cl₂. The combined organic layers were dried over Na₂SO₄, filtered and concentrated to give 0.376 g of the title compound.
¹H NMR (δ, ppm, CDCl₃): 7.23 (d, 2H); 7.13 (d, 2H); 3.41 (d, 2H); 2.81-2.66 (m, 4H); 2.37 (s, 3H); 1.70 (m, 1H).

### Intermediate 44

### 3-(4-Bromophenyl)-2,6-dimethyl-4H-pyran-4-one

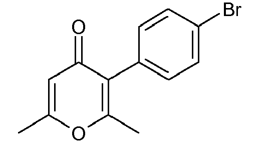

In a three necked round bottom flask were placed Eaton's reagent (ALDRICH, 72 ml) and acetic anhydride (30 ml). The mixture was heated to 95°C, then a solution of 4-bromophenylacetone (ALDRICH, 18.6 g) in acetic anhydride (30 ml) was added dropwise. After 1 h of stirring the mixture was poured onto ice/H₂O and extracted with toluene (3x400 ml). The combined organic layers were washed with H₂O (2x300 ml) and sat NaHCO₃ (2x300 ml), then dried over Na₂SO₄. Elimination of the solvent gave crude which was purified by column chromatography on silica gel, eluting with mixtures Hex/EtOAc v/v 5:1 and 3:1. 14.7 g of the title compound were obtained as a reddish powder.
¹H NMR (δ, ppm, DMSO-d₆): 7.59 (m, 2H); 7.17 (m, 2H); 6.20 (m, 1H); 2.27 (s, 3H); 2.14 (s, 3H).

### Intermediate 45

### 3-(4-Bromophenyl)-2,6-dimethyl-4(1H)-pyridinone

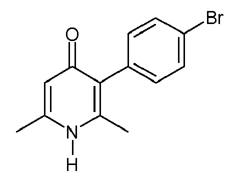

To a solution of Intermediate 44 (7 g) in ethanol (20 ml) was added commercial aqueous ammonia 30%. The suspension thus obtained was introduced in a steel reactor and heated to 140°C for 8 h, then allowed to cool to room temperature overnight. The precipitate was filtered and washed successively with H₂O (20 ml) and EtOAc (2x20 ml). 5.16 g of the title compound were obtained as a pale brown powder after drying under vacuum.
¹H NMR (δ, ppm, DMSO-d₆): 11.13 (bs, 1H); 7.52 (m, 2H); 7.11 (m, 2H); 5.92 (m, 1H); 2.18 (s, 3H); 2.04 (s, 3H).

### Intermediate 46

### 5-(4-Bromophenyl)-3-chloro-2,6-dimethyl-4(1H)-pyridinone

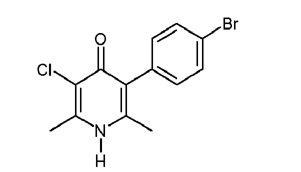

### METHOD A:

To a solution of Intermediate 45 (1 g) in a mixture CH₂Cl₂/MeOH v/v 2:1 (45 ml) was added portionwise N-chlorosuccinimide (0.64 g) at room temperature, and the mixture refluxed overnight. After cooling to room temperature, it was concentrated to dryness under vacuum. The resulting residue was triturated with CH₃CN and filtered. The precipitate was collected by filtration, washed with CH₃CN and dried under vacuum. 0.77 g of the title compound was obtained as a white powder.

### METHOD B:

To a solution of Intermediate 45 (2.63 g) in 375 ml of CH₂Cl₂:MeOH (2:1 mixture) cooled with a H₂O-ice-salt bath, was added TCCA (trichloroisocyanuric acid, 880 mg). After addition a white precipitate was formed. After 45 min of stirring, the mixture was concentrated to dryness, washed with 0.5M NaOH for 30 min, filtered and washed with H₂O and Et₂O-Hex to give 2.60 g (88%) of the desired compound as a white solid.
¹H NMR (δ, ppm, DMSO-d₆): 11.64 (bs, 1H); 7.55 (m, 2H); 7.13 (m, 2H); 2.41 (s, 3H); 2.06 (s, 3H).

### Intermediate 47

### 1,1-Dimethylethyl methyl(2-propyn-1-yl)carbamate

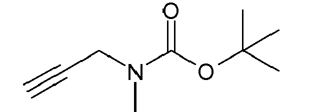

To a solution of di-*tert*-butyl dicarbonate (ALDRICH, 7.64 g) in dry MeOH (55 ml) under inert atmosphere at 0°C was added N-methylpropargylamine (2.69 ml). The mixture was stirred at room temperature overnight and evaporated under vacuum to give 5.22 g of the title compound.
¹H NMR (δ, ppm, CDCl₃): 4.03 (bs, 2H); 2.91 (s, 3H); 2.20 (t, 1H); 1.46 (s, 9H).

### Intermediate 48

### 1,1-Dimethylethyl {3-[4-(5-chloro-2,6-dimethyl-4-oxo-1,4-dihydro-3-pyridinyl)phenyl]-2-propyn-1-yl}methylcarbamate

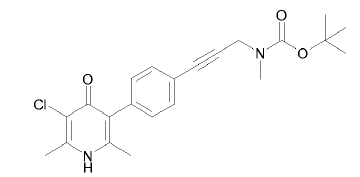

To a solution of intermediate 46 (4.93 g) and Intermediate 47 (4 g) in pyrrolidine (25 ml) under argon atmosphere was added Pd(PPh₃)₄ (ALDRICH, 1.82 g). The mixture was heated to 80°C overnight, then was diluted with CH₂Cl₂ at room temperature and filtrated through a pad of celite. 1N HCl (100 ml) was slowly added onto the filtrate and the two layers partitioned. The organic layer was washed with 1N HCl (2 x 50 ml), dried over Na₂SO₄, filtered and concentrated to dryness under reduced pressure. The solid obtained was washed with CH₃CN (3 times) and dried to give 3.75 g of the title compound.
¹H NMR (δ, ppm, DMSO-d₆): 11.64 (bs, 1H); 7.43 (d, 2H); 7.18 (d, 2H); 4.26 (bs, 2H); 2.88 (s, 3H); 2.37 (s, 3H); 2.06 (s, 3H); 1.42 (s, 9H). [ES⁺ MS] m/z 401 (MH⁺).

### Intermediate 49

### 3-Chloro-2,6-dimethyl-5-{4-[3-(methylamino)-1-propyn-1-yl]phenyl}-4(1H)-pyridinone hydrochloride

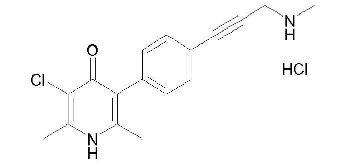

To a solution of intermediate 48 (3.4 g) in 40 ml of MeOH was added acetyl chloride (FLUKA, 3 ml). The mixture was stirred at room temperature under N₂ atmosphere overnight, then was concentrated under reduced pressure. Dichloromethane was added and the precipitate filtered and dried under vacuum to give 2.92 g of the title compound as a brown solid.
¹H NMR (δ, ppm, DMSO-d₆): 11.99 (bs, 1H); 9.25 (bs, 2H); 7.51 (d, 2H); 7.26 (d, 2H); 4.15 (bs, 2H); 2.66 (s, 3H); 2.41 (s, 3H); 2.10 (s, 3H).

### Intermediate 50

### 3-Chloro-2,6-dimethyl-5-{4-[3-(methylamino)-1-propyn-1-yl]phenyl}-4(1H)-pyridinone

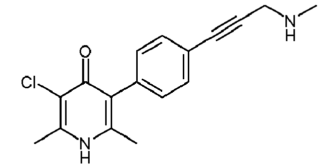

To the Intermediate 49 (1.87 g) was added 10% NaHCO₃ (55 ml) and CH₂Cl₂ (55 ml). The mixture was stirred and then the layers were separated. The aqueous layer was extracted with CH₂Cl₂. Combined organic layers were dried over Na₂SO₄, filtered and concentrated to dryness to give 1.34 g of the title compound.
¹H NMR (δ, ppm, DMSO-d₆): 11.68 (bs, 1H); 7.41 (d, 2H); 7.18 (d, 2H); 3.53 (bs, 2H); 2.38 (s, 3H); 2.36 (s, 3H); 2.07 (s, 3H).

### Intermediate 51

### 3-Chloro-5-(4-iodophenyl)-2,6-dimethyl-4(1H)-pyridinone

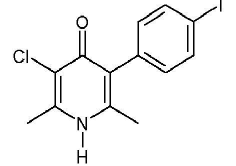

In a sealed tube was placed Intermediate 46 (2 g), KI (5.31 g), Cul (0.609 g), NMP (15 ml) and *N*,*N'*-dimethylethylenediamine (0.564 g), the mixture was heated to 140°C for 1 day. Then, H₂O was added over the crude and the solid precipitated was filtered. The brown solid obtained was suspended in NH₄OH (28% in H₂O) and the mixture was neutralized with 2N HCl. The pale brown solid precipitated was filtered and dried under vacuum to give 2.19 g of the title compound.
¹H NMR (δ, ppm, DMSO-d₆): 7.73 (d, 2H); 7.00 (d, 2H); 2.38 (s, 3H); 2.07 (s, 3H).

### Intermediate 52

### 3-(4-Bromophenyl)-5-chloro-2,6-dimethyl-4-pyridinyl bis(1-methylethyl)carbamate.

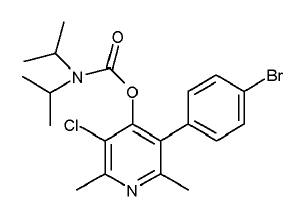

To a solution of 15 g of Intermediate 46 in 200 ml of pyridine were added *N*,*N-*diisopropylcarbamoyl chloride (ALDRICH, 15.64 g) and the mixture was heated at 85°C for 24 h. After cooling to room temperature, it was concentrated to dryness under vacuum. The resulting residue was dissolved in EtOAc (250 ml) and washed with H₂O (100 ml) and brine (100 ml). The organic layer was dried over MgSO₄ and concentrated to dryness to give 16.5 g of the title compound as a pale brown solid.
¹H-NMR (δ, ppm, DMSO-d₆): 7.63 (dm, 2H); 7.18 (m, 2H); 3.75 (m, 3H); 2.57 (s, 3H); 2.22 (s, 3H); 1.05- 0.86 (m, 12H).

### Intermediate 53

### 3-Chloro-5-(4'-formyl-4-biphenyl)-2,6-dimethyl-4-pyridinyl bis(1-methylethyl) carbamate

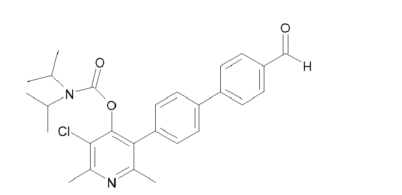

To a solution of Intermediate 52 (16.5 g) in a mixture of toluene (140 ml) and EtOH (70 ml) was added 4-formylphenylboronic acid (ALDRICH, 14.1 g), the resulting suspension was deoxygenated by bubbling argon for 5 min. Saturated NaHCO₃ (70 ml) and Pd(PPh₃)₂Cl₂ (ALDRICH, 2.63 g) were added successively and the mixture was refluxed for 1.5 h, then allowed to cool to room temperature. The two layers of the reaction were separated, the organic layer was washed with NaHCO₃ (10%), H₂O and brine and then dried over Na₂SO₄. Elimination of the solvent under vacuum gave a crude product which was purified by column chromatography on silica gel, eluting with mixtures CH₂Cl₂/MeOH v/v 100:5 to afford the title compound (12 g).
¹H-NMR (δ, ppm, CDCl₃): 10.08 (s, 1H); 7.98 (dm, 2H); 7.76 (dm, 2H); 7.66(dm, 2H); 7.37 (m, 2H); 3.84-3.72 (m, 2H); 2.68 (s, 3H); 2.36 (s, 3H); 1.14- 0.90 (m, 12H).

### Intermediate 54

### 3-Chloro-2,6-dimethyl-5-{4'-[(4-{4[(trifluoromethyl)oxy]phenyl}-1-piperidiyl}methyl-4-biphenylyl}-4-pyridinyl bis(1-methylethyl)carbamate chlorhydrate

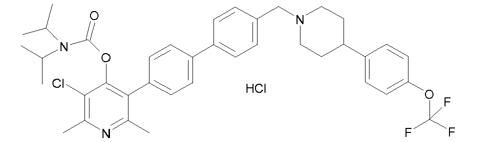

To a solution of Intermediate 13 (3.4 g) in a mixture of dichloroethane (50 ml) and THF (50 ml) under argon atmosphere, was added a solution of Intermediate 53 (4.9 g) in THF (100 ml). The mixture was stirred 30 min and then NaHB(AcO)₃ (2.9 g) was added portionwise at room temperature. After 12 h the reaction was completed and it was concentrated to dryness under vacuum. The resulting residue was treated with H₂O (50 ml) and extracted with CH₂Cl₂ (200 ml). The organic layer was washed with HCl 1 N (2 x 50 ml), dried over MgSO₄ and concentrated to dryness. The crude product (8.4 g) was triturated in Et₂O, collected by filtration and washed with the same solvent. 6.8 g of the title compound were obtained as a yellowish solid.
¹H-NMR (δ, ppm, DMSO-d₆): 10.75 (bs, 1H); 7.77 (m, 6H); 7.34 (m, 6H); 4.36 (m, 2H); 3.73 (m, 4H); 3.45 (m, 2H); 3.04 (m, 2H); 2.87 (m, 1H); 2.61 (s, 3H); 2.29 (s, 3H); 2.00 (m, 4H); 1.05- 0.84(m, 12H).

### Intermediate 55

### 1,1-Dimethylethyl {2-[(chlorocarbonyl)(methyl)amino]ethyl}carbamate

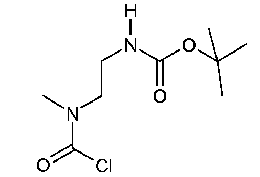

A solution of triphosgene (0.712g) in dry dichloromethane (5 ml) was cooled to 4°C in an ice-water bath under nitrogen. To this solution was added a solution of 1,1-dimethylethyl [2-(methylamino)ethyl]carbamate* (1.15g) and dry pyridine (1 ml) in dry dichloromethane (5 ml) with vigorous stirring. The mixture became orange and a yellow precipitate appeared. The mixture was allowed to warm to room temperature and stirred for 1 h. Aqueous hydrochloric acid (0.1N, 25 ml) was added and the two phases partitioned. The organic layer was washed with 0.1N HCl (4x50 ml), dried over Na₂SO₄ and concentrated to dryness to afford 1.1g of an orange oily residue which became solid upon standing in the freezer for 48h.
¹H NMR (δ, ppm, CDCl₃): 3.7-3.3 (bm, 4H); 3.2 and 3.1 (2bs, 3H); 2.9 (bs, 3H); 1.45 (bs, 9H)
* Prepared according to Lit ref: PCT Int. Appl., 2007/026027. 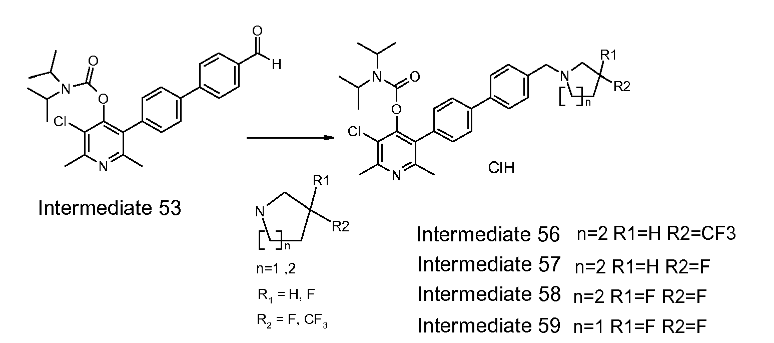 Intermediates 56-59 were prepared using a method analogous to that described for Intermediate 54, replacing Intermediate 13 with the commercially available amine shown
   in Table 4. Intermediates 56-59 were each purified by flash chromatography using mixtures of dichloromethane/methanol as eluent.

**Table 4**

| **Int.** | **Amine** | **Starting Aldehyde** | **Chemical name** | **Physical Data** |
|---|---|---|---|---|
| 56^{a} | 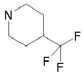 | Intermediate 53 | 3-chloro-2,6-dimethyl-5-(4'-{[4-(trifluoromethyl)-1-piperidinyl]methyl}-4-biphenylyl)-4-pyridinyl bis(1-methylethyl)carbam ate | ¹H NMR (δ, ppm, DMSO-d₆): 7.63 (m, 4H); 7.34 (m, 4H); 3.73 (m, 2H); 3.51 (s, 2H); 2.90 (m, 2H); 2.58 (s, 3H); 2.27 (s, 3H); 1.98 (m, 2H); 1.83-1.75 (m, 3H); 1.07- 0.82 (m, 12H). [ES MS] m/z: 602 (MH+). |
| 57^{a,b,} | 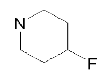 | Intermediate 53 | 3-chloro-5-{4'-[(4-fluoro-1-piperidinyl)methyl]-4-biphenylyl}-2,6-dimethyl-4-pyridinyl bis(1-methylethyl)carbam ate | ¹H NMR (δ, ppm, DMSO-d₆): 7.72-7.69 (d, 2H); 7.62-7.60 (d, 2H); 7.40-7.37 (d; 2H); 7.30-7.27 (d, 2H); 4.76-4.70 (d, 1H); 3.71 (m, 2H); 3.50 (s, 2H); 2.57 (s, 3H); 2.27 (s, 3H); 0.93-0.81 (m, 6H).[ES MS] m/z: 552 (MH+). |
| 58^{b} | 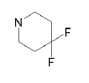 | Intermediate 53 | 3-chloro-5-{4'-[(4,4-difluoro-1-piperidinyl)methyl]-4-biphenylyl}-2,6-dimethyl-4-pyridinyl bis(1-methylethyl)carbam ate hydrochloride | ¹H NMR (δ, ppm, DMSO-d₆): 11.55 (s, 1H); 7.80-7.71 (m, 6H); 7.35 (m, 2H); 4.41 (br s, 2H); 3.74 (m, 2H); 3.43 (m, 2H); 3.17 (m, 2H); 2.62 (s, 3H); 2.31 (s, 3H); 1.04-0.83 (m, 12H). [ES MS] m/z: 570 (MH+). |
| 59^{a,b} | 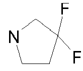 | Intermediate 53 | 3-chloro-5-{4'-[(3,3-difluoro-1-pyrrolidinyl)methyl]-4-biphenylyl}-2,6-dimethyl-4-pyridinyl bis(1-methylethyl)carbam ate | ¹H NMR (δ, ppm, DMSO-d₆): 7.71 (d, 2H); 7.63 (d, 2H); 7.40 (d, 2H); 7.30-7.28 (m, 2H); 3.79-3.69 (m, 2H); 3.65 (s, 2H); 2.88 (m, 2H); 2.71 (m, 2H); 2.32-2.18 (m, 5H); 1.04- 0.83 (m, 12H). [ES MS] m/z: 556 (MH+). |

| | | | | |
|---|---|---|---|---|
| ^{a}The product was isolated as the free amine by neutralizing with NaHCO₃ in the work-up process. ^{b}The amine, commercially available as the hydrochloride, was prepared by treating the hydrochloride with N,N-diisopropylethylamine (1.2 eq) at rt. | | | | |

### Intermediate 60

### 2-({[(1,1-dimethylethyl)(dimethyl)silyl]oxy}methyl)-6-methyl)-4-oxo-4H-pyran-3-yl trifluoromethanesulfonate

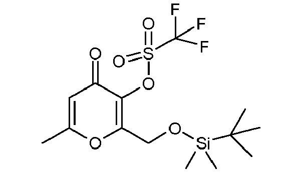

The title compound, Intermediate 60, was prepared according to a procedure similar to that described for Intermediate 6 in WO2007/138048.

### Intermediate 61

### 3-(4-bromophenyl)-2-({[(1,1-dimethylethyl)(dimethyl)silyl]oxy}methyl)-6-methyl-4H-pyran-4-one

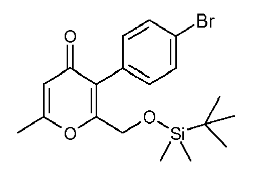

In a 250 ml round bottom flask were placed Intermediate 60 (2.87 g), 4-bromophenyl boronic acid (ALDRICH, 1.64g), tetrakis(triphenylphosphine)palladium(0) (ALDRICH, 0.906g) and sodium carbonate (1.51 g) in a mixture of EtOH/H₂O (75:7.5ml). The mixture was deoxygenated by bubbling argon for 15 min. After heating for 22h at 40°C under argon atmosphere, the reaction was not complete and 246 mg of tetrakis(triphenylphosphine)palladium(0) were added and the mixture was stirred at 40°C overnight. Then the ethanol was evaporated under vacuum. The crude was dissolved in water (150 ml) and extracted with ethyl acetate (3x100 ml). The organic phase was washed with brine (100 ml), dried over Na₂SO₄, filtered and concentrated to afford a reddish residue which was purified by column chromatography on silica gel eluting with mixtures ethyl acetate/hexane. 1.15 g of the title compound were obtained as a yellow solid.
¹H-NMR(δ, ppm, CDCl₃): 7.53(d, 2H); 7.18(d, 2H); 6.23(s, 1H); 4.35(s, 2H); 2.32(s, 3H); 0.89(s, 9H); 0.04(s, 6H)

### Intermediate 62

### 3-(4-bromophenyl)-2-(hydroxymethyl)-6-methyl-4(1H)-pyridinone

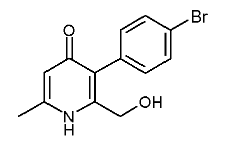

A suspension of 286 mg of Intermediate 61 in methanol (5 ml) was introduced in a microwave tube with magnetic stirring, 30% aqueous ammonia (10 ml) was added and the resulting suspension heated at 140 °C for 45 minutes under microwave radiation. Upon cooling, the organic solvent was evaporated under vacuum and the crude was diluted with water (25ml) and extracted with dichloromethane (3x25ml). The combined organic extracts were washed with Na₂SO₄, filtered and concentrated to dryness, to give 125 mg of the title compound as a beige solid.
¹H-NMR(δ, ppm, DMSO-d₆): 11.01(bd, 1H); 7.53(d, 2H); 7.13(d, 2H); 5.96(s, 1H); 5.52(bs, 1H); 4.18(bd, 2H); 2.23(s, 3H)

### Intermediate 63

### 3-(4-bromophenyl)-5-chloro-2-(hydroxymethyl)-6-methyl-4(1H)-pyridinone

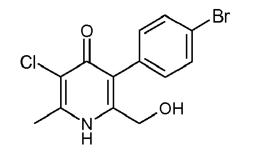

In a 50 ml round bottom flask was dissolved Intermediate 62 (270 mg) in dichloromethane/methanol v/v 2:1 (5/2.5ml) and trichloroisocyanuric acid (ALDRICH, 85.3 mg) was added. The solution was stirred under nitrogen atmosphere for 30 minutes. A white solid that appeared, was filtered and the filtrate was evaporated to dryness, to give 280g of the title compound as an off-white powder.
¹H-NMR(δ, ppm, DMSO-d₆): 11.53(bd, 1H); 7.55(d, 2H);; 7.17(d, 2H); 5.59(bd, 1H); 4.23(bd, 2H); 2.44(s, 3H)

### Intermediate 64

### 4'-[5-chloro-2-(hydroxymethyl)-6-methyl-4-oxo-1,4-dihydro-3-pyridinyl]-4-biphenyl-carbaldehyde

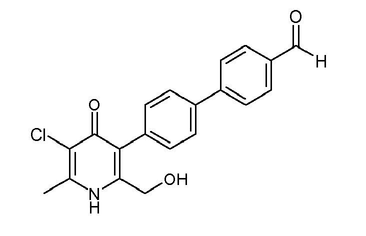

To a solution of Intermediate 63 (150 mg) in a mixture of toluene (10ml) and ethanol (10 ml) was added commercial 4-formylphenyl boronic acid (102 mg) and the resulting suspension was deoxygenated by bubbling argon for 15 minutes. 95.39 mg of Na₂CO₃, 2 ml of water and tetrakis(triphenylphosphine)palladium(0) (ALDRICH, 57 mg) were added successively and the mixture heated to 60-70°C. As the reaction progressed slowly five portions of 52 mg of tetrakis(triphenylphosphine)palladium(0) and 19 mg of 4-formylphenyl boronic acid were added over the next five days. Then the ethanol was evaporated under vacuum. The crude was dissolved in water (20 ml) and extracted with ethyl acetate (3x20 ml). The organic phase was washed with brine (10 ml), dried over Na₂SO₄, filtered and concentrated to afford a residue which was purified by column chromatography on silica gel eluting with mixtures dichloromethane/methanol. 55 mg of the title compound were obtained as a yellow solid.
¹H-NMR(δ, ppm, DMSO-d₆): 11.53(bs, 1H); 10.06(s, 1H); 8.02-7.94(m, 4H); 7.78(d, 2H);
7.35(d, 2H); 5.61 (bd, 1H); 4.26(bs, 2H); 2.46(s, 3H)

### Intermediate 65

### 3-(4-Bromophenyl)-5-chloro-2,6-dimethyl-4-pvridinyl bis(1-methylethyl)carbamate.

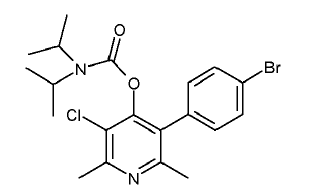

To a solution of 15g of 3-(4-bromophenyl)-5-chloro-2,6-dimethyl-4(1H)-pyridinone in 200ml of pyridine were added 2 equivalents of N,N-diisopropylcarbamoil chloride (ALDRICH 15.64g) and the mixture was heated at 85°C for 24 hours. After cooling to room temperature, it was concentrated to dryness under vacuum. The resulting residue was dissolved in ethyl acetate (250ml) and washed with water(100ml) and brine (100ml). The organic layer was dried over magnesium sulphate and concentrated to dryness to give 16.5g of the title compound as a pale brown solid.
¹H-NMR(δ, ppm, DMSO-d₆): 7.63(d, 2H); 7.18(m, 2H); 3.75(m, 2H); 2.57(s, 3H); 2.22(s, 3H); 1.05, 0.97, 0.86(3m, 12H).

### Intermediate 66

### 3-(4-bromophenyl)-5-chloro-2,6-dimethyl-1-oxido-4-pyridinyl bis(1-methylethyl) carbamate

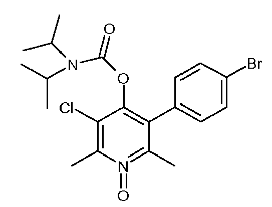

To a solution of Intermediate 65 (2 g) in dichloromethane (25 ml), was added metachloroperbenzoic acid (1.09 g) under argon atmosphere. The mixture was stirred 24h at room temperature. The solvent was evaporated and the crude product was purified by column chromatography on silica gel, eluting with mixtures dichloromethane/methanol to afford title compound (2.07g) as a white solid.
¹H-NMR(δ, ppm, CDCl₃): 7.55(d, 2H); 7.18(d, 1H); 7.06(d, 1H); 3.85, 3.72(2m, 2H); 2.75(s,
3H); 2.34(s, 3H); 1.15, 1.06, 0.90 (3m, 12H).

### Intermediates 67 and 68

### 3-(4-bromophenyl)-5-chloro-2-(hydroxymethyl)-6-methyl-4-pyridinyl bis(1-methylethyl) carbamate and 3-(4-bromophenyl)-5-chloro-6-(hydroxymethyl)-2-methyl-4-pyridinyl bis(1-methylethyl)carbamate

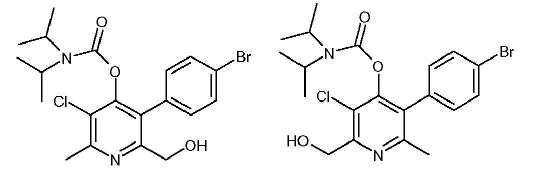

Intermediate 66 (2.07 g) was dissolved in acetic anhydride (40 ml), the mixture was refluxed at 140°C for 3 hours. After cooling at room temperature, the solvent was eliminated to dryness and the crude product, isolated as a black oil (2.3g), was dissolved in methanol (50 ml). 15 ml of NaOH 1N were added to the mixture at room temperature. After 12 hours the reaction was completed and methanol was eliminated in vacuo. The resulting residue was treated with water (20ml) and extracted with dichloromethane (3x25ml). The organic layer was washed with brine (2x10ml), dried over MgSO₄ and concentrated to dryness. The crude product was purified by column chromatography on silica gel, eluting with mixtures dichloromethane/methanol to afford a mixture of regioisomers, Intermediate 67 and 68, (1:1.8). (1.13 g) as a pure white solid,
¹H-NMR(δ, ppm, CDCl₃): 7.54(d, 2H); 7.12(d, 2H); 4.80, 4.39 (2s, 2H); 4.48 (bs, 1H, OH); 3.85, 3.73 (2m, 2H); 2.71, 2.36 (2s, 3H); 1.15, 1.06, 0.94 (3m, 12H).

### Intermediate 69 and Intermediate 70

### 3-chloro-6-(hydroxymethyl)-2-methyl-5-{4'-[(4-{4-[(trifluoromethyl)oxy]phenyl}-1-piperidinyl)methyl]-4-biphenylyl}-4-pyridinyl bis(1-methylethyl)carbamate trifluoroacetate salt, and 3-chloro-2-(hydroxymethyl)-6-methyl-5-{4'-[(4-{4-[(trifluoromethyl)oxy]phenyl}-1-piperidinyl)methyl]-4-biphenylyl}-4-pvridinyl bis(1-methylethyl)carbamate trifluoroacetate salt.

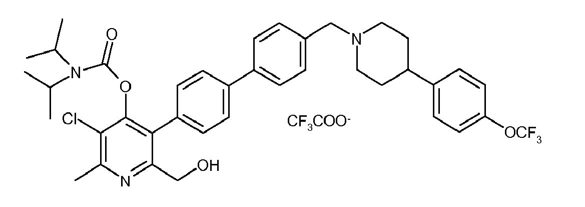

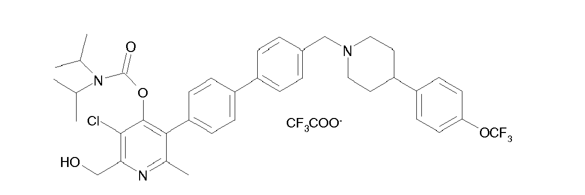

A mixture of Intermediates 67 and 68 (1.84 g) was dissolved in DME/H₂O (120 ml, 2:1) under argon atmosphere. [1,1'-Bis(diphenylphosphino)ferrocene]dichloro-palladium (II) (Aldrich, 144mg) and barium hydroxide (677 mg) were added successively. 1-{[4-(5,5-dimethyl-1,3,2-dioxaborinan-2-yl)phenyl]methyl}-4-{4-[(trifluoromethyl)oxy]phenyl} piperidine (1.77 g) (Intermediate 71) was added in portionwise. The solution was stirred at 80°C for 12 hours. After cooling to room temperature, the solvent was eliminated in vacuo. The resulting residue was treated with water (20ml) and extracted with dichloromethane (3x25ml). The organic layer was washed with brine (2x10ml), dried over MgSO₄ and concentrated to dryness. The crude product was purified by column chromatography on silica gel, eluting with mixtures dichloromethane/methanol to afford a mixture of regioisomers, Intermediate 69 and 70, (2.1 g) in a mixture (1:1.8).

The regioisomers were separated by preparative HPLC method: COLUMN: SUNFIRE 50x150 mm., GRADIENT: WATER, 0.1%TFA-ACN, 0.1%TFA 50:50; 3min 50:50; 15min 0:100; 20min, 0:100 WAVELENGHT: 210 nm FLOW: 100 ml/min.
Evaporation of the solvents in the appropriate fractions yielded Intermediate 69 trifluoroacetate salt, which was isolated as yellowish oil (285 mg).
¹H-NMR(δ, ppm, DMSO-d₆): 9.52(bs, 1H); 7.79(m, 4H); 7.64(m, 2H); 7.34(m, 6H); 4.39(bs, 2H); 4.29(s, 2H); 3.75(m, 2H); 3.52(m,2H); 3.12(m, 2H); 2.88(m, 1H); 2.64(s, 3H); 2.02(m,
2H); 1.87(m, 2H); 1.04, 0.94, 0.84 (3m, 12H).

Evaporation of the solvents in the appropriate fractions gave Intermediate 70 as the trifluoroacetate salt, which was isolated as a yellowish oil (200 mg).
¹H-NMR(δ, ppm, DMSO-d₆): 9.53(bs, 1H); 7.81 (m, 4H); 7.63(m, 2H); 7.34(m, 6H); 4.66(s,
2H); 3.40(bs, 2H); 3.75(m, 2H); 3.53(m,2H); 3.13(m, 2H); 2.87(m, 1H); 2.32(s, 3H);
2.03(m, 2H); 1.87(m, 2H); 1.05, 0.94, 0.85 (3m, 12H).

### Intermediate 71

### 1-{[4-(5,5-dimethyl-1,3,2-dioxaborinan-2-yl)phenyl]methyl}-4-{4-[(trifluoromethyl)oxy]phenyl} piperidine

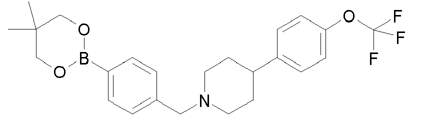

Intermediate 71 was prepared according to a method similar to that described in WO 2006/067216 (page 41), as described below:
A mixture of 4-{4-[(trifluoromethyl)oxy]phenyl}piperidine (4.91 g, 20.03 mmol) and N,N-diisopropylethylamine (5.95 ml, 33.4 mmol) in anhydrous THF (25ml) was stirred at room temp for 15 minutes. To this solution was added while stirring at 0-5°C 4-(5,5-dimethyl-1,3,2-dioxaborinan-2-yl)benzaldehyde (3.639 g, 16.69 mmol) and the mixture was stirred at 50°C overnight. The residue was concentrated to dryness under vacuum and redissolved in anhydrous ethanol (25ml). To this solution previously cooled at 0°C was added sodium borohydride (840 mg, 22.2 mmol) in small portions during 1h. The resulting mixture was stirred at room temperature. After 3 hours the reaction was completed. Solvent was evaporated under vacuum and residue was cooled to 0°C and partitioned between dichloromethane and water. Aqueous phase was extracted with dichloromethane (2x20ml). Combined organic phase was dried (Na₂SO₄), filtered and concentrated to dryness to give 5.25 g of a brown solid. Residue was triturated with few drops of methanol and acetonitrile. Solid was filtrated through a glass frited funnel and filtrate was concentrated under vacuum to dryness. 3.4 g of the title compound as an off-white solid were collected and dried under vacuum.
¹H-NMR(δ, ppm, CDCl₃): 7.77(d, 2H); 7.35(d, 2H); 7.23(d, 2H); 7.12(d, 2H); 3.77(s, 4H); 3.59(d, 2H); 3.02(m, 2H); 2.50(s, 1H); 2.1 0(m,2H); 1.80(m, 2H); 1.66(m, 2H); 1.02(s, 3H).

### Intermediate 72

### 3-{6-[4-(hydroxymethyl)phenyl]-3-pyridinyl}-2,6-dimethyl-4H-pyran-4-one

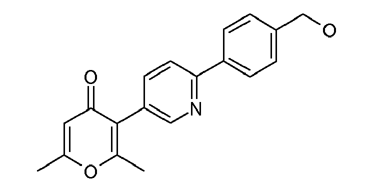

A solution of 5.0 g of 5-(2,6-dimethyl-4-oxo-4H-pyran-3-yl)-2-pyridinyl trifluoromethanesulfonate and 2.72 g of [4-(hydroxymethyl)phenyl]boronic acid in a mixture of toluene:EtOH (90ml:45ml) was deaereated with Argon for 15 min. To this solution was added 502 mg of bis(triphenylphosphine)palladium(II) chloride and deaereated with Argon for another 15 min. To this solution was added 43 ml of saturated solution sodium bicarbonate dropwise during 10 min. The reaction mixture was heated at 85 °C under argon for 2h. The reaction was filtered through celite and the organic solvent was removed under vacuum, the crude was dissolved with DCM, washed with 10% NaHCO₃, sat. NH₄Cl and sat. NaCl, the organic layers were dried over Na₂SO₄, filtered and concentrated to dryness to afford 4.95 g of the title compound as a brown solid.
¹H NMR (δ, ppm, DMSO-d₆ ): 8.48 (dd, 1H); 8.08 (d, 2H); 7.99 (dd, 1H); 7.73 (dd, 1H); 7.43 (d, 2H); 6.25 (d, 1H); 5.25 (t, 1H); 4.56 (d, 2H); 2.30 (s, 3H); 2.23 (s, 3H).

### Intermediate 73

### 6'-[4-(hydroxymethyl)phenyl]-2,6-dimethyl-3,3'-bipyridin-4(1H)-one

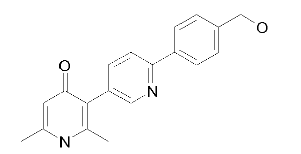

In a microwave flask, 500 mg of Intermediate 72 was suspended in 6 ml of EtOH, 12 ml of 32% ammonia solution was added; the reaction mixture was heated at 140°C in the microwave (pₘₐₓ=16bar; 70min; very high adsorption), the reaction mixture was filtered and evaporated under vacuum, the crude was triturated with DCM and Et₂O to afford 390 mg of the title compound as a brown solid.
¹H NMR (δ, ppm, DMSO-d₆): 11.24 (s, 1H); 8.43 (dd, 1H); 8.06 (d, 2H); 7.93 (d, 1H); 7.67 (dd, 1H); 7.42 (d, 2H); 5.97 (s, 1H); 5.25 (t, 1H); 4.55 (d, 2H); 2.21 (s, 3H); 2.14 (s, 3H).

### Intermediate 74

### 5-chloro-6'-[4-(hydroxymethyl)phenyl]-2,6-dimethyl-3,3'-bipyridin-4(1H)-one

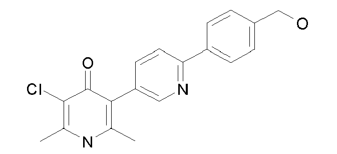

To a solution of 391 mg of Intermediate 73 in a mixture of DCM:MeOH (20ml:2ml) cooled at 0°C under nitrogen was added portionwise 120.5 mg of 1,3,5-trichloro-1,3,5-triazine-2,4,6(1H,3H,5H)-trione, the mixture was stirred for 2h at 0°C, it was allowed to reach room temperature, filtered and evaporated under vacuum, the crude was triturated with DCM to afford 352 mg of the title compound as a yellow solid.
¹H NMR (δ, ppm, DMSO-d₆): 11.74 (s, 1H); 11.15 (s, 1H); 8.46 (d, 1H); 8.07 (d, 2H); 7.97 (d, 1H); 7.71 (dd, 1H); 7.43 (d, 2H); 4.56 (s, 2H); 2.40 (s, 3H); 2.16 (s, 3H).

### Intermediate 75

### 4-(5'-chloro-2',6'-dimethyl-4'-oxo-1',4'-dihydro-3,3'-bipyridin-6-yl)benzaidehyde

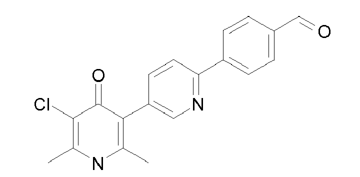

To a solution of 290 mg of Intermediate 74 in a mixture of DCM:MeOH (12ml:12ml) was added 591 mg of manganese dioxide every 24 hours until the reaction was completed (4 days). The reaction was filtered through celite and the organic solvent was removed under vacuum to afford 260 mg of the title compound as a yellow solid.
¹H NMR (δ, ppm, DMSO-d₆): 10.07 (s, 1H); 8.54 (d, 1H); 8.35 (d, 2H); 8.11 (d, 1H); 8.03 (d, 2H); 7.79 (dd, 2H); 2.39 (s, 3H); 2.16 (s, 3H).

### Intermediate 76

### Ethyl (5-bromo-2-pyridinyl)(difluoro)acetate

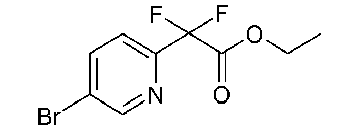

To a suspension of copper powder (ALDRICH, 3.29 g, 51.7 mmol) in 30ml anhydrous DMF under argon atmosphere, ethyl bromodifluoroacetate 98% (ALDRICH, 2.98 ml, 23.22 mmol) and 2,5-dibromopyridine (ALDRICH, 5 g, 21.11 mmol) were added. The mixture was stirred and heated to 60°C for 20h. Then the mixture was allowed to cool to room temperature, and diluted with ethyl acetate. A quenching solution of potassium dihydrogenphosphate (20ml) was added and the mixture was stirred for 30 min. The solid (green cooper salts) were filtered off and washed with ethyl acetate. Layers were separated and organic layer was washed with water (2x30ml). Organic layer was dried over anhydrous sodium sulphate, filtered and concentrated. The crude was purified by flash-master chromatography using Hexane/Ethyl Acetate as eluent to afford the title compound.
¹H-NMR (δ, ppm, DMSO-d₆): 8.85 (s, 1H); 7.58-7.55 8.33 (d, 1H); 7.82 (d, 1H); 4.36-4.28 (m, 2H); 1.24-1.18 (m, 3H).

### Intermediate 77

### 2-(5-bromo-2-pyridinyl)-2,2-difluoroethanol

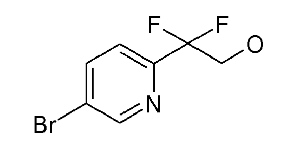

Ethyl (5-bromo-2-pyridinyl)(difluoro)acetate (Intermediate 76, 900 mg, 3.21 mmol) was dissolved in anhydrous methanol and the solution was cooled to 0°C. Sodiumborohydride (ALDRICH, 182 mg, 4.82 mmol) was added portionwise to the reaction mixture over 30 minutes at 0°C. Reaction was allowed to warm to room temperature and stirred overnight. Another equivalent of sodiumborohydride (ALDRICH, 182 mg, 4.82 mmol) was then added at 0°C and the mixture was stirred 2 hours longer. Reaction was quenching by adding 1ml 1N HCl at 0°C and concentrated under reduced pressure. Water was added and aqueous layer was extracted with methyl tert-butyl ether (3x20ml). Combined organic layer was dried over anhydrous sodium sulphate, filtered and concentrated. The crude was purified by flash-master chromatography using DCM/MeOH as eluent to afford the title compound.
¹H-NMR (δ, ppm, DMSO-d₆): 8.83 (s, 1H); 8.23 (d, 1H); 7.64 (d, 1H); 5.57 (t, 1H); 4.02-3.91 (m, 2H).

### Intermediate 78

### 2-(5-bromo-2-pyridinyl)-2,2-difluoroethyl trifluoromethanesulfonate

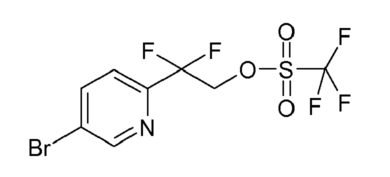

2-(5-bromo-2-pyridinyl)-2,2-difluoroethanol (Intermediate 77, 300 mg, 1.260 mmol) was dissolved in anhydrous dichloromethane and 2,6-di-terc-butyl-4-methylpyridine (ALDRICH, 388 mg, 1.891 mmol) was added. Solution was cooled to -78°C under argon atmosphere and trifluoromethanesulfonic anhydride (ALFA-AESAR, 427 mg, 1.512 mmol) was added drop wise over 20 minutes. Reaction mixture was allowed to warm to room temperature overnight. Solvent was removed under reduced pressure and this crude will be used in the next step without further purification.
¹H-NMR (δ, ppm, DMSO-d₆): 8.89 (s, 1H); 8.35 (d, 1H); 7.82 (d, 1H); 5.52-5.43 (m, 2H).

### Intermediate 79

### 5-bromo-2-{1,1-difluoro-2-[4-(trifluoromethyl)-1-piperidinyl]ethyl}pvridine

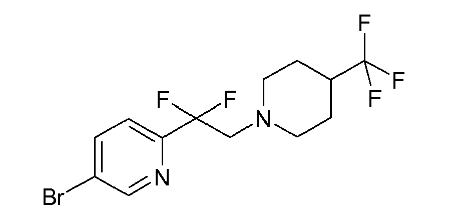

4-(trifluoromethyl)piperidine hydrochloride (ALDRICH, 571 mg, 3.73 mmol) was dissolved in 2ml DMF and DIPEA (FLUKA, 0.638 ml, 3.73 mmol) was added to release the free base. This solution was mixed with 2-(5-bromo-2-pyridinyl)-2,2-difluoroethyl trifluoromethanesulfonate (Intermediate 78, 460 mg, 1.243 mmol) in 5ml anhydrous DMF. The reaction mixture was stirred and heated at 60°C for 2 hours and 72 hours more at room temperature. The mixture was diluted in ethyl acetate (10ml) and washed with sat. NH₄Cl. Layers were separated and organic layer was dried over anhydrous sodium sulphate, filtered and concentrated. The crude was purified by flash-master chromatography using Hexane/Ethyl Acetate as eluent to yield 230 mg of the title compound.
¹H-NMR (δ, ppm, DMSO-d₆): 8.81 (s, 1H); 8.25-8.22 (m, 1H); 7.65 (m, 1H); 3.19 (t, 2H); 2.82 (m, 2H); 2.26 (m, 3H); 1.65 (m, 2H); 1.32-1.18 (m, 2H).

### Intermediate 80

### 2-{1,1-difluoro-2-[4-(trifluoromethyl)-1-piperidinyl]ethyl}-5-(4,4,5,5-tetramethyl-1,3,2-dioxaborolan-2-yl)pyridine

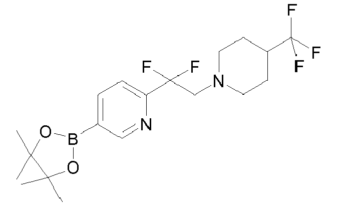

A flask charged with [1,1'-Bis(diphenylphosphino)ferrocene]dichloro-palladium(II) (ALDRICH, 11.76 mg, 0.016 mmol), potassium acetate (ALDRICH, 79 mg, 0.80 mmol) and bis(pinacolato)diboron (ALDRICH, 74.9 mg, 0.295 mmol) was flushed with argon. 5-bromo-2-{1,1-difluoro-2-[4-(trifluoromethyl)-1-piperidinyl] ethyl} pyridine (Intermediate 79, 100 mg, 0.268 mmol) was added dissolved in 2 ml DMSO. The mixture was stirred under argon atmosphere at 80°C for 1 15 min. Water was added and aqueous layer was extracted with toluene (3x20ml) Combined organic layer was dried over anhydrous sodium sulphate, filtered and concentrated. The crude was dissolved in dichloromethane, and the white solid precipitated was filtered off. Solvent was removed under reduced pressure and product was used in the next step without further purification.

### Intermediate 81

### 5-chloro-6'-{1,1-difluoro-2-[4-(trifluoromethyl)-1-piperidinyl]ethyl}-2,6-dimethyl-3,3'-bipyridin-4-yl bis(1-methylethyl)carbamate

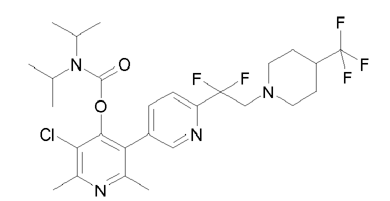

3-chloro-5-iodo-2,6-dimethyl-4-pyridinyl bis(1-methylethyl)carbamate (Intermediate 86, 116 mg, 0.283 mmol) and 2-{1,1-difluoro-2-[4-(trifluoromethyl)-1-piperidinyl]ethyl}-5-(4,4,5,5-tetramethyl-1,3,2-dioxaborolan-2-yl)pyridine (Intermediate 80, 85 mg, 0.202 mmol) were dissolved in 2 ml anhydrous DMF under nitrogen atmosphere. [1,1'-Bis(diphenylphosphino)ferrocene]dichloro-palladium(II) (ALDRICH, 29.6 mg, 0.040 mmol) was added and then potassium carbonate (ALDRICH, 84 mg, 0.607 mmol). The mixture was stirred at 80°C overnight. More [1,1'-bis(diphenylphosphino)ferrocene]dichloro-palladium (II) (0.1 eq, 15mg) was added and mixture was stirred for 4 hours longer. Reaction mixture was diluted with Ethyl Acetate and quenched by adding saturated NaHCO3. Layers were separated and organic layer was washed with saturated NH4Cl and saturated NaCi. Combined organic layer was dried over anhydrous sodium sulphate, filtered and concentrated. The crude was purified by flash-master chromatography using Hexane/Ethyl Acetate as eluent and repurified by preparative HPLC (Sunfire 19x150 gradient Water TFA 0.1% : ACN TFA 0.1% 80:20; 3 min 80:20; 15 min 20:80; 20min 20:80) to yield the title compound.
¹H-NMR (δ, ppm, DMSO-d₆): 9.11 (s, 1H); 8.42-8.38 (m, 1H); 7.84-7.81 (m, 1H) 7.09 (m, 1H); 4.09-3.91 (m, 3H); 2.90-2.87 (m, 3H); 2.52 (s, 3H); 2.41 (s, 3H); 2.34-2.22 (m, 4H); 1.69-1.65 (m, 3H); 1.32-1.21 (m, 12H).

### Intermediate 82

### 2,6-dimethyl-3-[6-(2-propen-1-yloxy)-3-pyridinyl]-4H-pyran-4-one

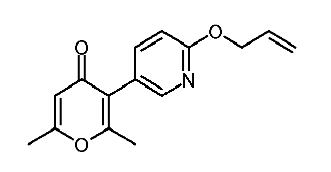

5-(2,6-dimethyl-4-oxo-4H-pyran-3-yl)-2(1H)-pyridinone (prepared in a similar manner to that described in Intermediate 4 of WO2006094799, 2 g, 9.21 mmol) was dissolved in 50 ml anhydrous toluene and silver carbonate (FLUKA, 1.52 g, 5.52 mmol) was added. Then, allylbromide (ALDRICH, 1.60 ml, 18.41 mmol) was added. The mixture was stirred at room temperature for 2 days. Solution turned form yellow to dark grey. The suspension was first filtered through paper-sintered glass funnel and after filtered through cameo to get a yellow solution. Solvent was removed under reduced pressure. The crude was purified by flash-master chromatography using Dichloromethane/Methanol as eluent to afford 1.75 g of the title compound.
¹H-NMR (δ, ppm, CD₃OD): 7.98 (s, 1H); 7.56 (dd, 1H); 6.87 (dd, 1H); 6.26 (s, 1H); 6.16-6.03 (m, 1H); 5.42-5.35 (m, 1H); 5.25-5.20 (m, 1H); 4.84-4.82 (m, 2H); 2.35 (s, 3H); 2.25 (s, 3H). [ES MS] m/z: 258 (MH+).

### Intermediate 83

### 2,6-dimethyl-6'-(2-propen-1-yloxy)-3,3'-bipyridin-4(1H)-one

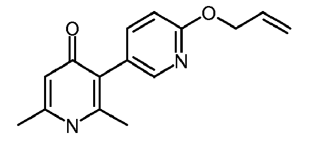

2,6-dimethyl-3-[6-(2-propen-1-yloxy)-3-pyridinyl]-4H-pyran-4-one (Intermediate 82, 1.75 g, 6.80 mmol) was weighed in a 20 ml microwave vial and 30% aqueous NH₃ (10ml, 6.80 mmol)/ 2 ml MeOH were then added. The reaction vessel was sealed and heated in a Biotage Initiator to 140°C for 90 minutes. After cooling the reaction, a white precipitate appeared. It was filtered and washed with ice water (3 ml) to yield 1.57 g of the title compound.
¹H-NMR (δ, ppm, CD₃OD): 7.95 (d, 1H); 7.54 (dd, 1H); 6.86 (dd, 1H); 6.27 (s, 1H); 6.16-6.03 (m, 1H); 5.42-5.35 (m, 1H); 5.25-5.20 (m, 1H); 4.83-4.81 (m, 2H); 2.33 (s, 3H); 2.18 (s, 3H). [ES MS] m/z: 257 (MH+).

### Intermediate 84

### 5-chloro-2,6-dimethyl-6'-(2-propen-1-yloxy)-3,3'-bipyridin-4(1H)-one

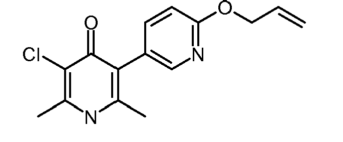

2,6-dimethyl-6'-(2-propen-1-yloxy)-3,3'-bipyridin-4(1H)-one (Intermediate 83, 1.11 g, 4.33 mmol) was dissolved in 5 ml MeOH / 1 ml CH₂Cl₂ under nitrogen atmosphere and the solution was cooled to 0°C.Then trichloroisocianuric acid (ALDRICH, 0.302 g, 1.299 mmol) was slowly added in portions. Mixture was stirred at 0°C for 2h until the reaction was complete. A white solid appeared; it was filtered off and washed with MeOH. Filtrate was concentrate to dryness. Then, the crude was suspended in 5 ml 0.5N NaOH and 5 ml MeOH. The mixture was stirred for 1 hour. Water was added, precipitated was filtrated and washed with cooled water. The mixture was again suspended in 5 ml 0.5N NaOH and 5 ml MeOH and stirred for 1 hour more, the precipitated solid was filtered and washed with ice water. The solid was dried under vacuum overnight to get 1.2 g of the title compound.
¹H-NMR (δ, ppm, DMSO-d₆): 7.86 (d, 1H); 7.47 (dd, 1H); 6.74 (dd, 1H);; 6.14-6.02 (m, 1H); 5.41-5.33 (m, 1H); 5.24-5.19 (m, 1H); 4.78 (m, 2H); 2.20 (s, 3H); 1.93 (s, 3H). [ES MS] m/z: 291 (MH+).

### Intermediate 85

### [(5'-chloro-2',6'-dimethyl-4'-oxo-1',4'-dihydro-3,3'-bipyridin-6-yl)oxy]acetaldehyde

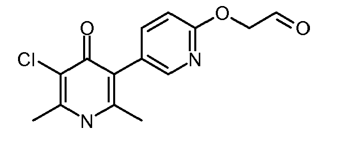

5-chloro-2,6-dimethyl-6'-(2-propen-1-yloxy)-3,3'-bipyridin-4(1*H*)-one (Intermediate 84, 500 mg, 1.72 mmol) was dissolved in a dioxane/water mixture in a 50ml round bottom flask. Then, supported OsO₄ (ALFAAESAR) was carefully added, 2,6-lutidine (ALDRICH, 184 mg, 1.72 mmol) and finally sodium periodate (ACROS, 368 mg, 1.72 mmol) were added. The mixture was mechanical-stirred overnight. Reaction was filtered, and washed with THF/water (2x10ml) and MeOH (5ml). Solvent was removed and the aqueous solution was back-extracted with CH₂Cl₂. Desired aldehyde remained in the organic layer, which was lyophilized. The solid obtained was dissolved in MeOH/CH₂Cl₂ and purified by flash-master chromatography using Dichloromethane/Methanol as eluent to afford 140 mg of the title compound.
¹H-NMR (δ, ppm, DMSO-d₆): 11.66 (s, 1H); 7.94-7.89 (m, 1H);7.61-7.51 (m, 1H); 6.98-6.82 (m, 1H); 4.21-4.11 (m, 2H); 3.28 (s, 2H); 2.37 (s, 3H); 2.10 (s, 3H).

### Intermediate 86

### 3-chloro-5-iodo-2,6-dimethyl-4-pyridinyl N,N-diisopropylcarbamate

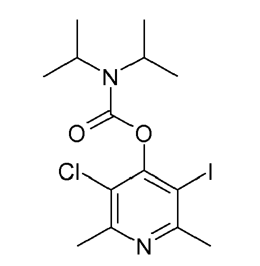

To a solution of 3-chloro-5-iodo-2,6-dimethyl-4(1H)pyridinone (3.5g, Intermediate 63 in WO2006094799) in pyridine (46ml), was added N,N-diisopropyl carbamoyl chloride (5.05g) and the mixture heated to 85°C for 24h. After cooling to room temperature, the mixture was concentrated to dryness under vacuum and the resulting residue dissolved in ethyl acetate (200 ml). The solution was washed with water (70ml), then dried over magnesium sulphate, filtered and concentrated to dryness. The residue was dissolved in Tert-butyl-methylether (200 ml) and the remaining solid removed by filtration. The solution thus obtained was washed with 1N NaOH (75 ml), water (75ml), 1N NH4Cl (75 ml), water (75 ml) and brine (75 ml), dried over sodium sulphate and concentrated to dryness to afford the title compound (4.27g) as a brown solid which was used for the next step without further purification.
¹H-NMR (δ, ppm, CDCl₃): 4.17 (m, 1H); 3.97 (m, 1H); 2.75 (s, 3H); 2.58 (s, 3H)

### Example 1

### 3-Chloro-2,6-dimethyl-5-(4-{3-[methyl({4-[(trifluoromethyl)oxy]phenyl}methyl)amino]-1-propyn-1-yl}phenyl)-4(1H)-pyridinone

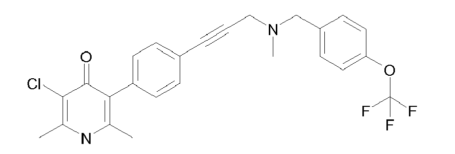

### METHOD A:

To a solution of Intermediate 46 (0.166 g) in DMF (8 ml) were added successively Pd(PPh₃)₂Cl₂ (0.050 g), Cul (0.040 g), Intermediate 3 (0.450 g) and Et₃N (4 ml). The resulting suspension was deoxygenated by bubbling argon for 5 minutes and heated to 110°C. After 8 h of heating, Et₃N was evaporated under vacuum and DMF eliminated by washing with 16 ml of a mixture EtOAc/NH₄Cl 1N (1:1). The organic layer was dried over Na₂SO₄ and the solution concentrated to dryness under vacuum to afford a crude product which was purified by column chromatography on silica gel, eluting with mixtures CH₂Cl₂/MeOH v/v 10:1 to afford 0.158 g of the title compound.

### METHOD B:

In a 2 L reactor under N₂ was placed 1.28 L of pyrrolidine and 202.4 g of Intermediate 3. Then were added 200.0 g of Intermediate 46, 25.2 g of PPh₃ and 11.2 g of Pd(PPh₃)₂Cl₂ consecutively. The mixture was stirred at 80-85°C °C for 22h. After cooling to room temperature the reaction mixture was filtered through a pad of celite which was washed with CH₂Cl₂. The resulting solution was diluted with 2 L of EtOH and then was concentrated under vacuum to 1.5 L of mixture and stored at room temperature overnight. The precipitate obtained was filtered and washed with 100 ml of cool EtOH. The resulting brown solid was washed twice with EtOH and then twice with CH₂Cl₂ and then dried over vacuum to give 215.25 g (71%) of the title compound as a white solid.
¹H NMR (δ, ppm, DMSO-d₆): 11.55 (s, 1H); 7.46 (d, 4H); 7.32 (d, 2H); 7.19 (d, 2H); 3.62 (s, 2H); 3.53 (s, 2H); 2.37 (s, 3H); 2.28 (s, 3H); 2.07 (s, 3H). [ES MS] m/z: 475 (MH+).

Examples 2-18 were prepared by methods analogous to that described for Example 1 as shown in Tables 5, 6, 7 and 8 replacing Intermediate 3 with the appropriate amine.

**Table 5**

| | | | | | |
|---|---|---|---|---|---|
| 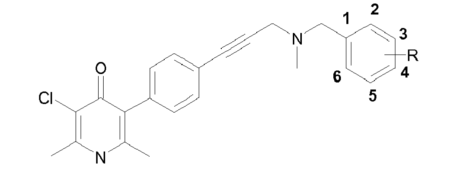 | | | | | |

| **Ex.** | **R** | **Meth** | **Starting Int.** | **Chemical name** | **Physical Data** |
|---|---|---|---|---|---|
| 2 | 4-CF₃ | A | 2 | 3-Chloro-2,6-dimethyl-5-{4-[3-(methyl{[4-(trifluoromethyl)phenyl ]methyl}amino)-1-propyn-1-yl]phenyl}-4(1*H*)-pyridinone | ¹H NMR (δ, ppm, CD₃OD): 7.66-7.56 (dd, 4H); 7.52-7.49 (d, 2H); 7.23-7.20 (d, 2H); 3.76 (s, 2H); 3.54 (s, 2H); 2.50 (s, 3H); 2.41 (s, 3H); 2.17 (s, 3H). [ES MS] m/z: 459 (MH+). |
| 3 | 2-CF₃ | A | 4 | 3-Chloro-2,6-dimethyl-5-{4-[3-(methyl{[2-(trifluoromethyl)phenyl ]methyl}amino)-1-propyn-1-yl]phenyl}-4(1*H*)-pyridinone | ¹H NMR (δ, ppm, CD₃OD): 7.83-7.81 (d, 1H); 7.69-7.66 (d, 1H); 7.63-7.58 (t, 1H); 7.50-7.47 (d, 2H); 7.45-7.40 (t, 1H); 7.22-7-19 (d, 2H); 3.86 (s, 2H); 3.60 (s, 2H); 2.50 (s, 3H); 2.39 (s, 3H); 2.17 (s, 3H). [ES MS] m/z: 459 (MH+). |
| 4 | 2,4- CF₃ | A | 5 | 3-(4-{3-[{[2,4-Bis(trifluoromethyl)ph enyl]methyl}(methyl)a mino]-1-propyn-1-yl}phenyl)-5-chloro-2,6-dimethyl-4(1*H*)-pyridinone | ¹H NMR (δ, ppm, CD₃OD): 8.12-8.09 (d, 1H); 7.96-7.93 (d, 1H); 7.93 (s, 1H); 7.49-7.46 (d, 2H); 7.22-7.19 (d, 2H); 3.94 (s, 2H); 3.64 (s, 2H) 2.50 (s, 3H); 2.42 (s, 3H); 2.17 (s, 3H). [ES MS] m/z: 527 (MH+). |
| 5 | H | A | *N*-methyl-*N*-propargyl benzylami ne | 3-Chloro-2,6-dimethyl-5-(4-(3-(methyl(phenylmethyl) amino)-1-propyn-1-yl) phenyl-4(1H)-pyridinone | ¹H NMR (δ, ppm, DMSO): 11.62 (S, 1H); 7.47-7.17 (m, 9H); 3.59 (s, 2H); 3.51 (s, 2H); 2.37 (s, 3H); 2.28 (s, 3H); 2.08 (s, 3H). EM-(M/Z): 389.20. |

**Table 6**

| | | | | | |
|---|---|---|---|---|---|
| 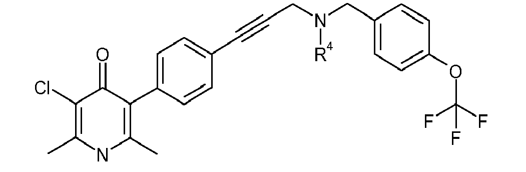 | | | | | |

| **Ex.** | **R⁴** | **Meth** | **Starting Int.** | **Chemical name** | **Physical Data** |
|---|---|---|---|---|---|
| 6^{a,b} | Pr | A | 6 | 3-Chloro-2,6-dimethyl-5-(4-{3-[propyl({4-[(trifluoromethyl)oxy]p henyl}methyl)amino]-1-propyn-1-yl}phenyl)-4(1*H*)-pyridinone trifluoroacetate | ¹H NMR (δ, ppm, CDCl₃): 7.61 (d, 2H); 7.44 (d, 2H); 7.28-7.19 (m, 4H); 4.30 (bs, 2H); 3.99 (bs, 2H); 3.05 (m, 2H); 2.46 (s, 3H); 2.10 (s, 3H); 1.82 (m, 2H); 0.98 (t, 3H). [ES MS] m/z 503 (MH+). |
| 7^{b,c} | cyclopropyl | A | 7 | 3-Chloro-5-(4-{3-[cydopropyl({4-[(trifluoromethyl)oxy]p henyl}methyl)amino]-1-propyn-1-yl}phenyl)-2,6-dimethyl-4(1*H*)-pyridinone | ¹H NMR (δ, ppm, CDCl₃): 12.27 (bs, 1H); 7.41 (d, 2H); 7.34 (d, 2H); 7.15-7.11 (m, 4H); 3.88 (s, 2H); 3.51 (s, 2H); 2.31 (s, 2H); 2.18 (m, 1H); 1.96 (s, 3H); 0.60-0.47 (m, 4H). [ES MS] m/z 503 (MH+). |
| 8 | H | A | 8 | 3-Chloro-2,6-dimethyl-5-(4-{3-[({4-[(trifluoromethyl)oxy]p henyl}methyl)amino]-1-propyn-1-yl}phenyl)-4(1*H*)-pyridinone. | ¹H NMR (δ, ppm, DMSO-d₆): 7.49 (d, 2H); 7.41 (d, 2H); 7.31 (d, 2H); 7.17 (d, 2H); 3.84 (s, 2H); 3.54 (s, 2H); 2.37 (s, 3H); 2.07 (s, 3H). [ES MS] m/z: 461 (MH+). |
| 9 | | B | 9 | 3-Chloro-2,6-dimethyl-5-(4-{3-[[2-(methyloxy)ethyl]({4-[(trifluoromethyl)oxy]p henyl}-methyl)amino]-1-propyn-1-yl}phenyl)-4(1*H*)-pyridinone. | ¹H NMR (δ, ppm, DMSO-d₆): 11.62 (s, 1H); 7.48 (d, 2H); 7.45 (d, 2H); 7.32 (d, 2H); 7.19 (d, 2H); 3.74 (s, 2H); 3.58 (s, 2H); 3.49 (t, 2H); 3.23 (s, 3H); 2.72 (t, 2H); 2.37 (s, 3H); 2.07 (s, 3H). [ES MS] m/z: 519 (MH+). |

| | | | | | |
|---|---|---|---|---|---|
| ^{a} The product was isolated as trifluoroacetate salt after purification by HPLC (mobile phase: CH₃CN/H₂O with 0.1 % of TFA). ^{b} Reaction under microwave irradiation at 120°C for 10 min. ^{c} The product was isolated as neutral amine after purification by HPLC (mobile phase: CH₃CN/H₂O with 0.1% of TFA), then basic treatment (2N NaOH) and extraction with CH₂Cl₂. | | | | | |

**Table 7**

| | | | | | |
|---|---|---|---|---|---|
| All the experiments were carried out following the method A as described for Example 1. | | | | | |
| 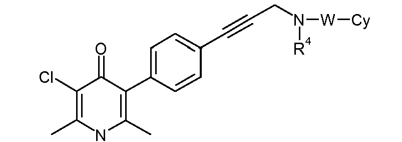 | | | | | |

| **Ex.** | **-W-Cy** | **R⁴** | **Starting Int.** | **Chemical name** | **Physical Data** |
|---|---|---|---|---|---|
| 10^{a} | 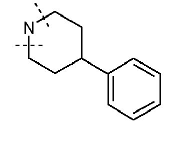 | | 14 | 3-Chloro-2,6-dimethyl-5-{4-[3-(4-phenyl-1-piperidinyl)-1-propyn-1-yl]phenyl}-4(1*H*)-pyridinone. | ¹H NMR (δ, ppm, DMSO-d₆): 11.63 (s, 1H); 7.45 (d, 2H); 7.31-7.17 (m, 7H); 3.57 (br s, 2H); 2.98 (br s, 2H); 2.55-2.48 (m, 1H); 2.37 (s, 3H); 2.35 (br s, 2H); 2.07 (s, 3H); 1.81-1.63 (m, 4H). [ES MS] m/z: 431 (MH+). |
| 11^{b,d,e} | 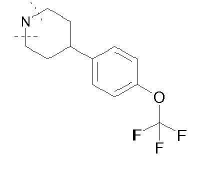 | | 15 | 3-Chloro-2,6-dimethyl-5-{4-[3-(4-{4-[(trifluoromethyl)oxy] phenyl}-1-piperidinyl)-1-propyn-1-yl]phenyl}-4(1*H*)-pyridinone hydrochloride. | ¹H NMR (δ, ppm, DMSO-d₆): 12.02 (s, 1H); 11.04 (s, 1H); 7.58 (d, 2H); 7.38 (d, 2H); 7.34 (d, 2H); 7.27 (d, 2H); 4.39 (s, 2H); 3.67 (d, 2H); 3.22 (br s, 2H); 2.93 (br s, 1H); 2.41 (s, 3H); 2.10 (s, 3H); 2.04 (br s, 4H). [ES MS] m/z: 515 (MH+). |
| 12^{c,d} | Bn | Bn | 16 | 3-(4-{3-[Bis(phenylmethyl)a mino]-1-propyn-1-yl}phenyl)-5-chloro-2,6-dimethyl-4(1*H*)-pyridinone hydrochloride. | ¹H NMR (δ, ppm, DMSO-d₆-D₂O): 7.63-7.41 (m, 12H); 7.27 (d, 2H); 4.44 (br s, 4H); 3.93 (s, 2H); 2.39 (s, 3H); 2.09 (s, 3H). [ES MS] m/z: 467 (MH+). |
| 13^{b,d} | 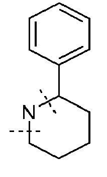 | | 17 | 3-Chloro-2,6-dimethyl-5-{4-[3-(2-phenyl-1-piperidinyl)-1-propyn-1-yl]phenyl}-4(1*H*)-pyridinone hydrochloride | ¹H NMR (δ, ppm, DMSO-d₆): 12.27 (br s, 1H); 11.81 (br s, 1H); 7.74 (br s, 2H); 7.59 (d, 2H); 7.51-7.39 (m, 5H); 7.27 (d, 2H); 4.40 (t, 1H); 4.04 (d, 1H); 3.67 (d, 2H); 3.31 (br s, 1H); 2.42 (s 3H), 2.25-2.16 (m; 2H), 2.11 (s; 3H), 1.94-1.81 (m, 4H). [ES MS] m/z: 431 (MH+). |
| 14^{a,d} | 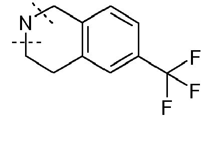 | | 18 | 3-Chloro-2,6-dimethyl-5-(4-(3-[6-(trifluoromethyl)-3,4-dihydro-2(1*H*)-isoquinolinyl]-1-propyn-1-yl}phenyl)-4(1*H*)-pyridinone hydrochloride. | ¹H NMR (δ, ppm, DMSO-d₆-D₂O): 7.67-7.50 (m, 5H); 7.24 (d, 2H); 4.62 (br s, 2H); 4.53 (br s, 2H); 3.61 (s, 2H); 3.22 (br s, 2H); 2.37 (s, 3H); 2.06 (s, 3H). [ES MS] m/z: 471 (MH+). |
| 15^{a,d} | 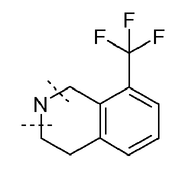 | | 19 | 3-Chloro-2,6-dimethyl-5-(4-{3-[8-(trifluoromethyl)-3,4-dihydro-2(1*H*)-isoquinolinyl]-1-propyn-1-yl}phenyl)-4(1*H*)-pyridinone hydrochloride. | ¹H NMR (δ, ppm, DMSO-d₆-D₂O): 7.70 (d, 1H); 7.62 (d, 1H); 7.56-7.51 (m, 3H); 7.27 (d, 2H); 4.68 (br s, 2H); 4.63 (s, 2H); 3.71 (br s, 4H); 3.28 (br s, 2H); 2.41 (s, 3H); 2.09 (s, 3H). [ES MS] m/z: 471 (MH+). |

| | | | | | |
|---|---|---|---|---|---|
| ^{a} Reaction under microwave irradiation at 120°C for 15-35 min. ^{b} Reaction under microwave irradiation at 80°C for 5-6 min, PPh₃ was replaced with ^{t}Bu₃P·HBF₄. ^{c} Reaction under microwave irradiation at 140°C for 15 min, PPh₃ and ^{t}Bu₃P·HBF₄ were added. ^{d} The final product was isolated as hydrochloride salt after addition of 4N HCl in dioxane over a solution of the product as free base in MeOH. ^{e} Intermediate 46 was replaced with Intermediate 51 in the experimental procedure. | | | | | |

**Table 8**

| | | | | |
|---|---|---|---|---|
| All the experiments were carried out following the method A as described for Example 1. | | | | |
| 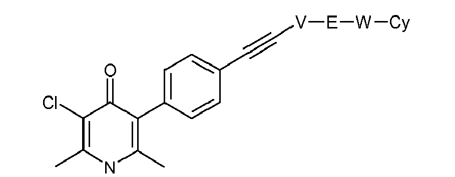 | | | | |

| **Ex.** | **-V-E-W-Cy** | **Star. Int.** | **Chemical name** | **Physical Data** |
|---|---|---|---|---|
| 16^{a} | 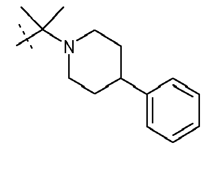 | 20 | 3-Chloro-2,6-dimethyl-5-{4-[3-methyl-3-(4-phenyl-1-piperidinyl)-1-butyn-1-yl]phenyl}-4(1H)-pyridinone. | ¹H NMR (δ, ppm, DMSO-d₆): 11.62 (s, 1H); 7.41 (d, 2H); 7.30-7.21 (m, 5H); 7.17 (d, 2H); 3.22 (d, 2H); 2.98 (br s, 2H); 2.56-2.42 (m, 1H); 2.37 (s, 3H); 2.32-2.25 (m, 2H); 2.07 (s, 3H); 1.84-1.63 (m, 4H); 1.44 (s, 6H). [ES MS] m/z: 459 (MH+). |
| 17^{a,b} | 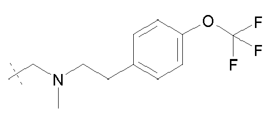 | 43 | 3-Chloro-2,6-dimethyl-5-(4-{3-[methyl(2-{4-[(trifluoromethyl)oxy] phenyl}ethyl)amino]-1-propyn-1-yl}phenyl)-4(1*H*)-pyridinone hydrochloride | ¹H NMR (δ, ppm, DMSO-d₆): 11.97 (bs, 1H); 11.02 (bs, 1H); 7.55 (d, 2H); 7.45 (d, 2H); 7.35 (d, 2H); 7.26 (d, 2H); 4.43 (bs, 2H); 3.44 (m, 2H); 3.12 (t, 2H); 2.94 (s, 3H); 2.40 (s, 3H); 2.09 (s, 3H). [ES⁺ MS] m/z 489 (MH⁺). |
| 18^{b,c} | 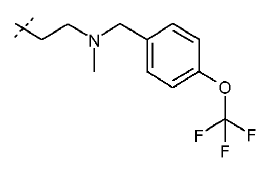 | 41 | 3-Chloro-2,6-dimethyl-5-(4-{4-[methyl({4-[(trifluoromethyl)oxy] phenyl}methyl)amin o]-1-butyn-1-yl}phenyl)-4(1*H*)-pyridinone hydrochloride | ¹H NMR (δ, ppm, DMSO-d₆): 11.9 (bs, 1H); 10.9 (bs, 1H); 7.76 (d, 2H); 7.49 (d, 2H); 7.45 (d, 2H); 7.19 (d, 2H); 4.51 (dd, 2H); 4.32 (dd, 2H); 3.06 (t, 2H); 2.72 (d, 2H); 2.39 (s, 3H); 2.08 (s, 3H). [ES MS] m/z 489 (MH+). |

| | | | | |
|---|---|---|---|---|
| ^{a} Reaction under microwave irradiation at 120°C for 11-35 min. ^{b} The final product was isolated as hydrochloride salt after addition of 4N HCl in dioxane over a solution of the product as free base in MeOH. ^{c} Reaction under microwave irradiation at 140°C for 15 min. | | | | |

### Example 19

### 3-Chloro-2,6-dimethyl-5-(4-{3-[methyl({4-[(trifluoromethyl)oxy]phenyl} methyl)amino]-1-propyn-1-yl}phenyl)-4(1H)-pyridinone hydrochloride

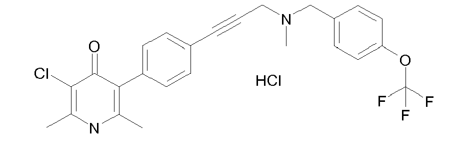

In a round bottom flask under N₂ atmosphere were placed 215.25 g of Example 1 and 2.9 L of MeOH. Then, 0.34 L of a solution of HCl in dioxane (4.0 M) were added dropwise in order to keep the temperature below 30°C. The resulting mixture was stirred at room temperature for 30 min, then filtered. The resulting solution was concentrated under vacuum and the residue suspended in 2 L of *i*-PrOH. The solution was concentrated under vacuum to 1 L of mixture and then stored at 5°C overnight. The precipitate obtained was filtered under N₂ atmosphere and then dried at 40°C under vacuum to afford 195.59 g (85%) of the title compound as a white solid.
¹H NMR (δ, ppm, DMSO-d₆): 12.47 (s, 1H); 11.55 (s, 1H); 7.81 (d, 2H); 7.59 (d, 2H); 7.49 (d, 2H); 7.28 (d, 2H); 4.46 (d, 2H); 4.27 (d, 2H); 2.80 (s, 3H); 2.44 (s, 3H); 2.13 (s, 3H). [ES MS] m/z: 475 (MH+).

### Example 20

### 3-Chloro-2,6-dimethyl-5-(4-{3-[propyl({4-[(trifluoromethyl)oxy]phenyl}methyl)amino]-1-propyn-1-yl}phenyl)-4(1H)-pyridinone

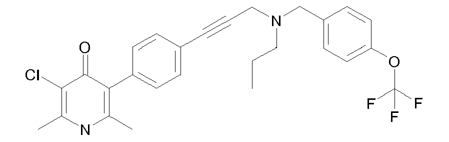

Example 6 (55 mg) was suspended in 2N NaOH and extracted with CH₂Cl₂ (2x). The organic layer was dried over Na₂SO₄, filtered and concentrated to give 29 mg of the title compound as a yellow solid.
¹H NMR (δ, ppm, CDCl₃): 7.38 (m, 4H); 7.15-7.08 (m, 4H); 3.65 (s, 2H); 3.49 (s, 2H); 2.54 (t, 2H); 2.28 (s, 3H); 1.93 (s, 3H); 1.55 (m, 2H); 0.92 (t, 3H). [ES MS] m/z 503 (MH+).

### Example 21

### 3-Chloro-2,6-dimethyl-5-(4-{3-[propyl({4-[(trifluoromethyl)oxy]phenyl}methyl)amino]-1-propyn-1-yl}phenyl)-4(1H)-pyridinone hydrochloride

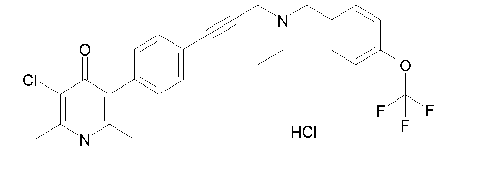

Example 6 (0.234 mg) was suspended in MeOH and 2N HCl (0.512 ml) was added dropwise. The resulting solution was concentrated to dryness to afford 0.245 g of the title compound as a pale brown solid.
¹H NMR (δ, ppm, CDCl₃): 11.94 (bs, 1H); 11.19 (bs, 1H); 7.80 (d, 2H); 7.57 (d, 2H); 7.50 (d, 2H); 7.26 (d, 2H); 4.56-4.29 (m, 4H); 3.15 (m, 2H); 2.40 (s, 3H); 2.09 (s, 3H); 1.80 (m, 2H); 0.92 (t, 3H). [ES MS] m/z 503 (MH+).

### Example 22

### 3-Chloro-2,6-dimethyl-5-{4-[3-(4-{4-[(trifluoromethyl)oxy]phenyl}-1-piperidinyl)-1-propyn-1-yl]phenyl}-4(1H)-pyridinone trifluoroacetate

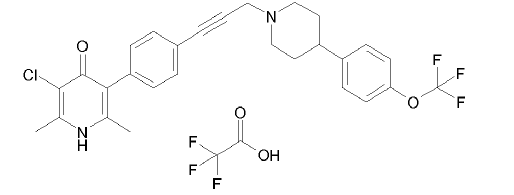

In a microwave reactor were placed Intermediate 46 (96 mg) and Intermediate 15 (130 mg) solved in 1 ml of DMF. Then, Cul (18 mg), Pd(PPh₃)₂Cl₂ (21 mg), ^{t}Bu₃P·HClO₄ (27 mg) and Et₃N (0.171 ml) were added consecutively. The resulting mixture was heated at 120 °C under microwave radiation for 10 min. An additional amount of Intermediate 15 (87 mg) and DMF (0.5 ml) were added and the mixture was further heated at 120 °C for 10 min. H₂O was added to the mixture and the resulting precipitated solid was filtered off and the filter cake washed with H₂O. 0.5N HCl and MeOH were added to the solid, the mixture was filtered, and the filtrate was neutralized with 1N NaOH and extracted with EtOAc. The resulting crude of reaction was purified by preparative HPLC (eluant: CH₃CN-H₂O with 0.1% of TFA) three times to give 9.1 mg of a 1:1 mixture of trifluoroacetate salt and free base of the desired compound as a pale yellow solid.
¹H NMR (δ, ppm, DMSO-d₆) of the 1:1 mixture: 11.69 (bs, 2H); 10.17 (bs, 1H); 7.54-7.58 (m, 4H); 7.32-7.40 (m, 8H); 7.26 (d, 2H); 7.13 (d, 2H); 4.43 (s, 4H); 3.71 (d, 4H); 3.17-3.25 (m, 4H); 2.89-2.97 (m, 2H); 2.38 (s, 6H); 2.21 (m, 4H); 2.08 (s, 6H); 1.80-1.93 (m, 4H).

### Example 23

### 3-Chloro-2,6-dimethyl-5-(4-{[methyl({4-[(trifluoromethyl)oxy]phenyl}methyl)amino]-methyl}phenyl)-4(1H)-pyridinone hydrochloride

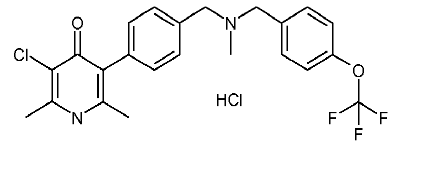

In a microwave reactor were placed 55.3 mg of 3-chloro-5-iodo-2,6-dimethyl-4(1*H*)-pyridinone,¹ 72.8 mg of Intermediate 32, 1.0 ml of H₂O, 63 mg of *n*-Bu₄NBr and 62 mg of Na₂CO₃. Then were added 2.2 mg of Pd(OAc)₂ and the resulting mixture was heated to 150°C for 6 min. After cooling, the resulting mixture was extracted with EtOAc, dried over MgSO₄, filtered, concentrated to dryness and the residue was purified by preparative HPLC to obtain the crude amine which was treated with 1.0 N HCl to obtain 16.9 mg (15%) of the title compound as a white solid.
¹H NMR (δ, ppm, DMSO-d₆-D₂O): 7.70 (d, 2H); 7.55 (d, 2H); 7.47 (d, 2H); 7.28 (d, 2H); 4.46 (br s, 2H); 4.28 (br s, 2H); 2.57 (s, 3H); 2.39 (s, 3H); 2.08 (s, 3H). [ES MS] m/z: 451 (MH+).

¹ PCT WO 2006/094799 A2
¹ PCT WO 2006/094799 A2

### Example 24

### 3-Chloro-2,6-dimethyl-5-{4-[(4-{4-[(trifluoromethyl)oxy]phenyl}-1-piperidinyl)methyl]-phenyl}-4(1H)-pyridinone hydrochloride

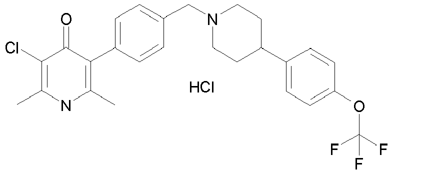

Example 24 was prepared by a method analogous to the described to Example 23 replacing Intermediate 32 with Intermediate 36.
¹H NMR (δ, ppm, DMSO-d₆-D₂O): 7.55 (d, 2H); 7.37-7.27 (m, 6H); 4.33 (br s, 2H); 3.48 (d, 2H); 3.14-3.06 (m, 2H); 2.92-2.84 (m, 1H); 2.38 (s, 3H); 2.08 (s, 3H); 2.03-1.89 (m, 4H). [ES MS] m/z: 491 (MH+).

### Example 25

### 3-Chloro-2,6-dimethyl-5-(3-{[methyl({4-[(trifluoromethyl)oxy]phenyl}methyl)amino]-methyl}phenyl)-4(1H)-pyridinone hydrochloride

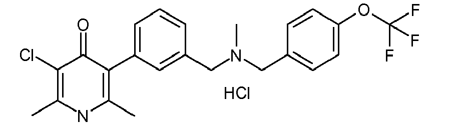

In a microwave reactor were placed 77.4 mg of Intermediate 33, 118 mg of K₂CO₃, 3.1 mg of *n*-Bu₄NBr and 1 ml of EtOH, then were added 54 mg of 3-chloro-5-iodo-2,6-dimethyl-4(1*H*)-pyridinone¹ and 3.2 mg of Pd(OAc)₂. The resulting mixture was deoxygenated bubbling N₂ for 5 min and heated to 100°C for 10 min. After cooling, the resulting mixture was filtered, concentrated to dryness and the residue was purified by chromatography on silica gel eluting with MeOH-CH₂Cl₂ 1:99 to 6:94 to afford the crude amine that was dissolved in MeOH, treated with 0.05 ml of 4.0 N HCl in dioxane and concentrated to dryness to afford 40 mg (43%) of the title compound as a colourless oil, which yield a pale yellow solid after adding CH₂Cl₂ and Et₂O successively followed by evaporation of the solvent.
¹H NMR (δ, ppm, DMSO-d₆-D₂O): 7.70 (d, 2H); 7.49-7.7.41 (m, 5H); 7.30-7.27 (m, 1H); 4.44 (br s, 2H); 4.26 (br s, 2H); 2.56 (s, 3H); 2.40 (s, 3H); 2.11 (s, 3H). [ES MS] m/z: 451 (MH+).

Examples 26-30 were prepared by a method analogous to that described for Example 25 replacing Intermediate 33 with the appropriate boronic acid, as shown in Table 9.
Examples 26, 28 and 30 were isolated as trifluoroacetate salt by HPLC purification (phase mobile: CH₃CN-H₂O with 0.1 % of TFA) instead of hydrochloride salt.

**Table 9**

| | | | | | | | |
|---|---|---|---|---|---|---|---|
| 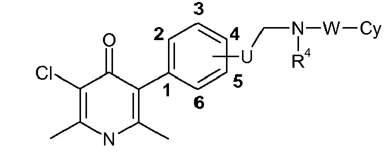 | | | | | | | |

| **Ex.** | **Pos** | **U** | -**W-Cy** | **R⁴** | **Start. Int.** | **Chemical name** | **Physical Data** |
|---|---|---|---|---|---|---|---|
| 26 | 4 | 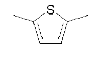 | 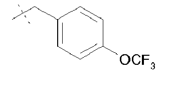 | Me | 34 | 3-Chloro-2,6- dimethyl-5-[4-(5-{[methyl({4-[(trifluoromethy l)oxy]phenyl}m ethyl)amino]-methyl}-2-thienyl)phenyl] -4(1*H*)-pyridinone trifluoroacetate | ¹H NMR (δ, ppm, DMSO-d₆): 11.68 (s, 1H); 10.10 (s, 1H); 7.68 (d, 4H); 7.57 (d, 1H); 7.50 (d, 2H); 7.36 (d, 1H); 7.26 (d, 2H); 4.68 (br s, 2H); 4.55 (br s, 2H); 2.63 (s, 3H); 2.39 (s, 3H); 2.11 (s, 3H). [ES MS] m/z: 533 (MH+). |
| 27^{a} | 4 | 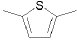 | 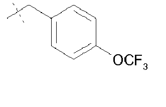 | Me | 34 | 3-Chloro-2,6-dimethyl-5-[4-(5-{[methyl({4-[(trifluoromethy I)oxy]phenyl}m ethyl)amino]-methyl}-2-thienyl)phenyl] -4(1*H*)-pyridinone hydrochloride. | ¹H NMR (δ, ppm, DMSO-d₆): 12.10 (s, 1H); 11.03 (s, 1H); 7.76 (d, 2H); 7.68 (d, 2H); 7.56 (d, 1H); 7.47 (d, 2H); 7.43 (d, 1H); 7.26 (d, 2H); 4.22-4.55 (m, 4H), 2.59 (s, 3H); 2.42 (s, 3H); 2.13 (s, 3H) . [ES MS] m/z: 533 (MH+). |
| 28 | 4 | 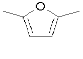 | 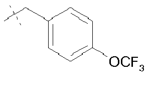 | Me | 35 | 3-Chloro-2,6-dimethyl-5-[4-(5-{[methyl({4-[(trifluoromethy I)oxy]phenyl}m ethyl)amino]-methyl}-2-furanyl)phenyl] -4(1*H*)-pyridinone trifluoroacetate | ¹H NMR (δ, ppm, DMSO-d₆): 11.67 (s, 1H); 10.23 (s, 1H); 7.77 (d, 2H); 7.69 (d, 2H); 7.49 (d, 2H); 7.28 (d, 2H); 7.06 (d, 1H); 6.86 (d, 1H); 4.68 (br s, 2H); 4.50 (br d, 2H); 4.31 (br s, 2H); 2.67 (s, 3H); 2.38 (s, 3H); 2.09 (s, 3H). [ES MS] m/z: 517 (MH+). |
| 29 | 4 | 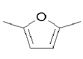 | 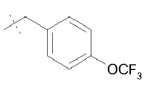 | Me | 35 | 3-Chloro-2,6-dimethyl-5-[4-(5-{[methyl({4-[(trifluoromethy I)oxy]phenyl}m ethyl)amino]-methyl}-2-furanyl)phenyl] -4(1*H*)-pyridinone hydrochloride. | ¹H NMR (δ, ppm, DMSO-d₆): 12.17 (br s, 1H); 11.13 (br s, 1H); 7.77 (d, 4H); 7.47 (d, 2H); 7.28 (d, 2H); 7.05 (d, 1H); 6.87 (d, 1H); 4.26-4.52 (m, 4H); 2.64 (br s, 3H); 2.43 (s, 3H); 2.13 (s, 3H). [ES MS] m/z: 517 (MH+). |
| 30 | 3 | 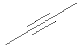 | 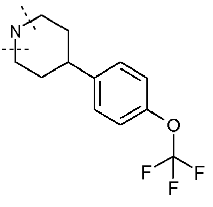 | | 37 | 3-Chloro-2,6-dimethyl-5-{3-[3-(4-{4-[(trifluoromethy l)oxy]phenyl}-1-piperidinyl)-1-propyn-1-yl]phenyl}-4(1*H*)-pyridinone trifluoroacetate | ¹H NMR (δ, ppm, DMSO-d₆-D₂O): 7.62-7.25 (m, 8H); 4.39 (s, 2H); 3.69 (d, 2H); 3.23 (m, 2H); 2.96-2.87 (m, 1H); 2.37 (s, 3H); 2-10-2.04 (m, 2H); 2.06 (s, 3H); 1.91-1.82 (m, 2H). [ES MS] m/z: 515 (MH+). |

| | | | | | | | |
|---|---|---|---|---|---|---|---|
| ^{a} Heated to 110 °C for 30 min under microwave radiation. | | | | | | | |

### Example 31

### 3-Chloro-2,6-dimethyl-5-(2-{[methyl({4-[(trifluoromethyl)oxy]phenyl}methyl)amino]methyl}-1-benzothien-5-yl)-4(1H)-pyridinone hydrochloride

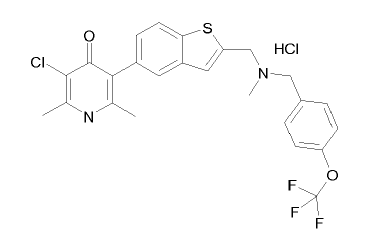

In a microwave reactor were placed 62 mg of 3-chloro-5-iodo-2,6-dimethyl-4(1*H*)-pyridinone¹ in 2.0 ml of EtOH, 130 mg of Intermediate 38, 3.5 mg of *n*-Bu₄NBr and 136 mg of K₂CO₃. This mixture was deoxygenated by bubbling N₂ for 5 min, then was Pd(OAc)₂ (3.7 mg) was added and the reaction mixture heated at 100°C under for 30 min. After cooling, the resulting mixture was filtered through a pad of celite, concentrated to dryness and the residue purified by chromatography on silica gel eluting with MeOH-CH₂Cl₂ 1:99 to 5:95 to afford 37 mg (67%) of the crude amine that was dissolved in 2.0 ml of MeOH and treated with 0.1 ml of 4.0 N HCl in dioxane. The solvents were removed to dryness to afford 39 mg of the title compound as a white solid.
¹H NMR (δ, ppm, DMSO-d₆): 12.18 (s, 1H), 11.17 (s, 1H), 8.04 (d, 1H), 7.77 (d, 2H), 7.74 (s, 2H), 7.47 (d, 2H), 7.23 (dd, 1H), 4.74-4.57 (m, 4H), 2.62 (s, 3H), 2.44 (s, 3H), 2.12 (s, 3H). [ES MS] m/z: 507 (MH+).

¹ PCT WO 2006/094799 A2

### Example 32

### 3-Chloro-2,6-dimethyl-5-{4-[1-({4-[(trifluoromethyl)oxy]phenyl}methyl)-1,2,3,6-tetrahydro-4-pyridinyl]phenyl}-4(1H)-pyridinone hydrochloride

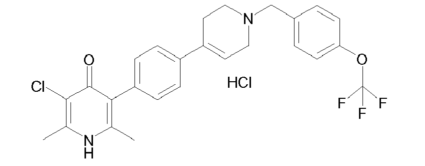

In a round bottom flask under argon atmosphere were placed 208 mg of Intermediate 39 in 15 ml of DMF, 152 mg of K₂CO₃, 104 mg of 3-chloro-5-iodo-2,6-dimethyl-4(1*H*)-pyridinone¹ and 26 mg of Pd(OAc)₂. The reaction mixture was deoxygenated by bubbling argon for 5 min, then was heated at 100°C for 15 h. After cooling, the mixture was diluted with EtOAc, washed three times with saturated solution of NH₄Cl and then with brine, dried over Na₂SO₄, filtered and concentrated to dryness. The residue was triturated with CH₂Cl₂ and the solid obtained filtered and dried under vacuum. The solid was treated with 5 ml of 1 N HCl solution, stirred for 10 minutes, filtered and dried under vacuum to afford 50 mg (27%) of the title compound as a white solid.
¹H NMR (δ, ppm, DMSO-d₆): 7.67-764 (d, 2H); 7.52-7.50 (d, 2H); 7.37-7.35 (d, 2H); 7.22-7.19 (d, 2H); 6.17 (br s, 1H); 4.20 (br s, 2H); 3.63 (br-s, 2H); 2.82 (br s, 2H); 2.50 (s, 3H); 2.16 (s, 3H). [ES MS] m/z: 489 (MH+).

### Example 33

### 3-Chloro-2,6-dimethyl-5-{4'-[(4-{4-[(trifluoromethyl)oxy]phenyl}-1-piperidinyl)methyl]-4-biphenylyl}-4(1H)-pyridinone hydrochloride

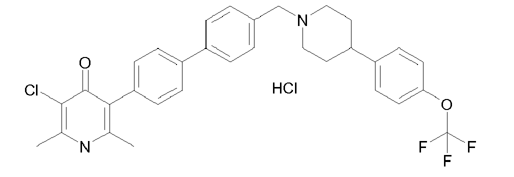

### METHOD A:

In a microwave reactor were placed 166 mg of Intermediate 36 in 3 ml of EtOH, 105 mg of Intermediate 51, 5.0 mg of *n*-Bu₄NBr, 182 mg of K₂CO₃ and 5.0 mg of Pd(OAc)₂. The resulting mixture was deoxygenated bubbling argon for 15 min, stirred at room temperature for 30 min and heated to 100°C for 30 min. The mixture was diluted with EtOH, filtered, concentrated to dryness and the residue purified by chromatography on silica gel eluting with MeOH-CH₂Cl₂ 0:100 to 10:90 to afford the free amine, to which 4.0 N HCl in dioxane was added. The mixture was concentrated to dryness to afford 96 mg of crude hydrochloride, which was triturated with CH₃CN to afford 77 mg of the title compound as a white solid.

### METHOD B:

A solution of Intermediate 54 (10.7 g) in a mixture MeOH (25 ml) and HCl 2N (100 ml) was refluxed for 6 h. After cooling to room temperature, a white solid precipitated. This solid was collected by filtration, washed with Et₂O and dried under vacuum. 7.77 g of the title compound were obtained as a white powder.
¹H NMR (δ, ppm, DMSO-d₆): 12.17 (s, 1H); 10.74 (s, 1H); 7.82 (d, 2H); 7.74-7.71 (m, 4H); 7.37-7.29 (m, 6H); 4.37 (s, 2H); 3.47 (d, 2H); 3.11-3.00 (m, 2H); 2.91-2.83 (m, 1H); 2.43 (s, 3H); 2.15 (s, 3H); 2.09-1.95 (m, 4H). [ES MS] m/z: 567 (MH+).

Examples 34-36 were prepared by a method analogous to method A described for Example 33 replacing Intermediate 36 with the appropriate boronic acid, as shown in Table 10

**Table 10**

| | | | | | | |
|---|---|---|---|---|---|---|
| 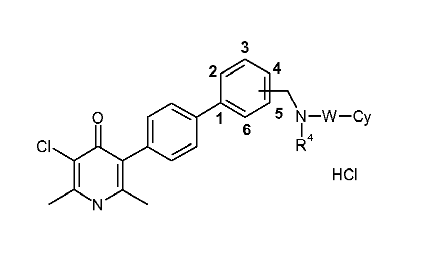 | | | | | | |

| **Ex.** | **Pos** | **-W-Cy** | **R₄** | **Star. Int.** | **Chemical name** | **Physical Data** |
|---|---|---|---|---|---|---|
| 34 | 3 | 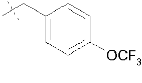 | Me | 33 | 3-Chloro-2,6-dimethyl-5-(3'-{[methyl({4-[(trifluoromethyl)oxy] phenyl}methyl)amino ]methyl}-4-biphenylyl)-4(1*H*)-pyridinone hydrochloride | ¹H NMR (δ, ppm, DMSO-d₆-D₂O): 11.97 (s, 1H); 10.75 (s, 1H); 7.98 (s, 1H); 7.82-7.79 (m, 1H); 7.76 (d, 2H); 7.74 (d, 2H); 7.56 (d, 2H); 7.48 (d, 2H); 7.31 (d, 2H); 7.52 (d, 2H); 4.34-4.25 (m, 2H); 2.53 (s, 3H); 2.42 (s, 3H); 2.14 (s, 3H). [ES MS] m/z: 527 (MH+). |
| 35 | 4 | 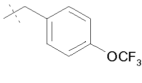 | Me | 32 | 3-Chloro-2,6-dimethyl-5-(4'-{[methyl({4-[(trifluoromethyl)oxy] phenyl}methyl)amino ]methyl}-4-biphenylyl)-4(1*H*)-pyridinone hydrochloride. | ¹H NMR (δ, ppm, DMSO-d₆): 12.30 (s, 1H); 11.02 (s, 1H); 7.77 (t, 4H); 7.74 (t, 4H); 7.47 (d, 2H); 7.31 (d, 2H); 4.48 (br t, 2H); 4.31 (br s, 2H); 2.54 (d, 3H); 2.44 (s, 3H); 2.16 (s, 3H). [ES MS] m/z: 527 (MH+). |
| 36^{a,b} | 4 | 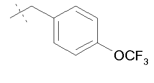 | Me | 32 | 3-Chloro-2,6-dimethyl-5-(4'-{[methyl({4-[(trifluoromethyl)oxy] phenyl}methyl)amino ]methyl}-4-biphenylyl)-4(1*H*)-pyridinone trifluoroacetate. | ¹H NMR (δ, ppm, DMSO-d₆): 11.66 (br s, 1H); 9.89 (br s, 1H); 7.83 (d, 2H); 7.48-7.73 (m, 8H); 7.29 (d, 2H); 4.50 (m, 2H); 4.29 (m, 2H); 2.58 (br s, 3H); 2.39 (s, 3H); 2.12 (s, 3H). [ES MS] m/z: 527 (MH+). |

| | | | | | | |
|---|---|---|---|---|---|---|
| ^{a} Heated at 100°C under microwave radiation for 30 min. ^{b} The product was isolated as trifluoroacetate salt after purification by HPLC (mobile phase: CH₃CN/H₂O with 0.1% of TFA). Acidic treatment (HCl in dioxane) was not carried out. | | | | | | |

### Example 37

### 3-Chloro-2,6-dimethyl-5-(4-{3-[(2-methyl)({4-[(trifluoromethyl)oxy]phenyl}methyl)amino]-1-propyn-1-yl}phenyl)-4(1H)-pyridinone hydrochloride

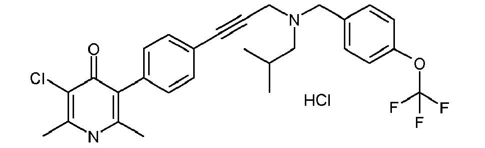

To a solution of 105 mg of Example 8 in 2.0 ml of MeOH and 0.5 ml of DCE under argon atmosphere were added 0.027 ml of 2-methylpropanal and 25 mg of NaBH₃CN consecutively. The mixture was stirred overnight and additional 0.027 ml of 2-methylpropanal and 25 mg of NaBH₃CN were added. The resulting reaction mixture was stirred overnight and the mixture concentrated to dryness. The residue was treated with EtOAc, the organic solution dried over MgSO₄ and concentrated to dryness. The crude was purified by chromatography on silica gel eluting with MeOH-CH₂Cl₂ 1:99 to 3:97 to afford 84 mg (72%) of the of the crude amine that was dissolved in MeOH, treated with 0.1 ml of 4.0 N HCl in dioxane and concentrated to dryness to give 91 mg of the title compound as a pale brown solid.
¹H NMR (δ, ppm, DMSO-d₆-D₂O): 7.76 (d, 2H); 7.57 (d, 2H); 7.49 (d, 2H); 7.26 (d, 2H); 4.48 (d, 2H); 4.28 (d, 2H); 3.06 (d, 2H); 2.80 (s, 3H); 2.39 (s, 3H); 2.14-2.05 (m, 1H); 2.08 (s, 3H); 0.94 (d, 6H). [ES MS] m/z: 517 (MH+).

### Example 38

### 3-Chloro-5-(4-{3-[(cyclopentylmethyl)({4-[(trifluoromethyl)oxy]phenyl}methyl) aminol-1-propyn-1-yl}phenyl)-2,6-dimethy)-4(1H)-pyridinone hydrochloride

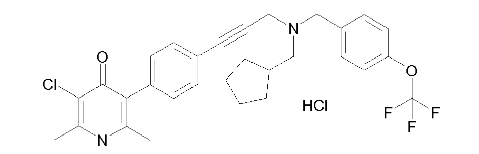

Example 38 was prepared by a method analogous to that described for Example 37 replacing 2-methylpropanal with 2- cyclopentanecarbaldehyde.
¹H NMR (δ, ppm, DMSO-d₆-D₂O): 7.75 (d, 2H); 7.56 (d, 2H); 7.50 (d, 2H); 7.26 (d, 2H); 4.48 (s, 2H); 4.26 (br s, 2H); 3.21 (s, 1H); 2.80 (s, 3H); 2.38 (s, 3H); 2.34-2.27 (m, 1H); 2.08 (s, 3H); 1.87-1.80 (m, 2H); 1.58-1.50 (m, 4H); 1.22 (s, 2H). [ES MS] m/z: 543 (MH+).

### Example 39

### 3-Chloro-2,6-dimethyl-5-[4-(3-{methyl[(5-methyl-2-thienyl)methyl]amino}-1-propyn-1-yl)phenyl]-4(1H)-pyridinone hydrochloride

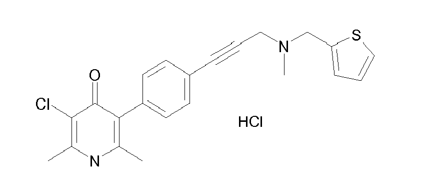

To a solution of Intermediate 50 (150 mg) in 3 ml of MeOH and 1 ml of CH₂Cl₂ were added 2-thiophenecarboxaldehyde (ALDRICH, 112 mg), NaBH₃CN (ALDRICH, 78 mg) and AcOH (0.086 ml) consecutively. The mixture was stirred at room temperature overnight, then the precipitated solid was filtered, washed with CH₂Cl₂/MeOH (95:5) and dried over vacuum to give 94 mg of a white solid. The solid was suspended in MeOH and 0.089 ml of 4M HCl in dioxane was added. After 1 h the mixture was concentrated under reduced pressure and Et₂O/Hex/ CH₂Cl₂ was added to give a precipitate which was filtered and dried to give 101 mg of the title compound as a white solid.
¹H NMR (δ, ppm, DMSO-d₆): 11.97 (bs, 1H); 11.20 (bs, 1H); 7.73 (d, 1H); 7.57 (d, 2H); 7.42 (m, 1H); 7.26 (d, 2H); 7.16 (m, 1H); 4.65 (bs, 2H); 4.24 (m, 2H); 2.83 (s, 3H); 2.40 (s, 3H); 2.10 (s, 3H).

Examples 40-50 were prepared by a method analogous to that described for Example 39, replacing 2-thiophenecarboxaldehyde with the appropriate aldehyde, as indicated in Table 11.

**Table 11**

| | | | | |
|---|---|---|---|---|
| 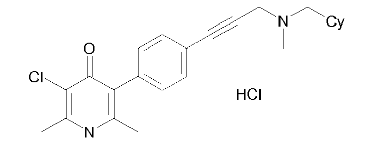 | | | | |

| **Ex.** | **Cy** | **Starting aldehyde** | **Chemical name** | **Physical Data** |
|---|---|---|---|---|
| 40^{a} | 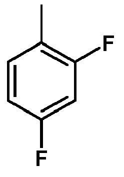 | 2,4-difluorobenz aldehyde | 3-Chloro-5-(4-{3-[[(2,4-difluorophenyl)methyl](m ethyl)amino]-1-propyn-1-yl}phenyl)-2,6-dimethyl-4(1*H*)-pyridinone hydrochloride | ¹H NMR (δ, ppm, DMSO-d₆): 11.91 (bs, 1H); 11.15 (bs, 1H); 7.80 (m, 1H); 7.54 (d, 2H); 7.41 (m, 1H); 7.26 (d, 2H); 7.25 (m, 1H); 4.45 (m, 2H); 4.35 (s, 2H); 2.82 (s, 3H); 2.40 (s, 3H); 2.09 (s, 3H). |
| 41 | 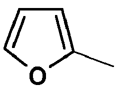 | 2-furaldehyde | 3-Ch loro-5-(4-{3-[(2-furanylmethyl)(methyl)a mino]-1-propyn-1-yl}phenyl)-2,6-dimethyl-4(1*H*)-pyridinone hydrochloride | ¹H NMR (δ, ppm, DMSO-d₆): 12.14 (bs, 1H); 11.46 (bs, 1H); 7.84 (s, 1H); 7.55 (d, 2H); 7.25 (d, 2H); 6.79 (m, 1H); 6.58 (m, 1H); 4.50 (bs, 2H); 4.28 (bs, 2H); 2.80 (s, 3H); 2.41 (s, 3H); 2.10 (s, 3H). |
| 42 | 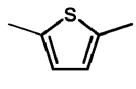 | 5-methylthioph ene-2-carboxaldeh yde | 3-Chloro-2,6-dimethyl-5-[4-(3-{methyl[(5-methyl-2-thienyl)methyl]amino}-1-propyn-1-yl)phenyl]-4(1*H*)-pyridinone hydrochloride | ¹H NMR (δ, ppm, DMSO-d₆): 11.98 (bs, 1H); 11.17 (bs, 1H); 7.57 (d, 1H); 7.26 (d, 2H); 7.19 (m, 1H); 6.83 (m, 1H); 4.55 (bs, 2H); 4.24 (m, 2H); 2.82 (s, 3H); 2.46 (s, 3H); 2.40 (s, 3H); 2.10 (s, 3H). |
| 43^{a} | 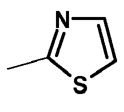 | 2-thiazolecarb oxaldehyde | 3-Chloro-2,6-dimethyl-5-(4-{3-[methyl(1,3-thiazol-2-ylmethyl)amino]-1-propyn-1-yl}phenyl)-4(1*H*)-pyridinone hydrochloride | ¹H NMR (δ, ppm, DMSO-d₆): 12.06 (bs, 1H); 7.95 (m, 2H); 7.54 (d, 2H); 7.24 (d, 2H); 4.77 (bs, 2H); 4.34 (bs, 2H); 2.87 (s, 3H); 2.40 (s, 3H); 2.09 (s, 3H). |
| 44^{a} | 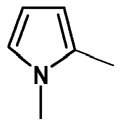 | 1-methyl-2-pyrrolecarbo xaldehyde | 3-Chloro-2,6-dimethyl-5-[4-(3-{methyl[(1-methyl-1H-pyrrol-2-yl)methyl]amino}-1-propyn-1-yl)phenyl]-4(1*H*)-pyridinone hydrochloride | ¹H NMR (δ, ppm, DMSO-d₆): 12.03 (bs, 1H); 11.03 (bs, 1H); 7.56 (d, 2H); 7.26 (d, 2H); 6.88 (m, 1H); 6.38 (m,1H); 6.06 (m,1H); 4.40 (m, 2H); 4.28 (m, 2H); 3.73 (s, 3H); 2.80 (s, 3H); 2.40 (s, 3H); 2.10 (s, 3H). |
| 45 | 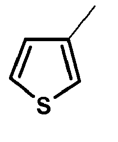 | 3-thiophenecar boxaldehyde | 3-Chloro-2,6-dimethyl-5-(4-{3-[methyl(3-thienylmethyl)amino]-1-propyn-1-yl}phenyl)-4(1*H*)-pyridinone hydrochloride | ¹H NMR (δ, ppm, DMSO-d₆): 12.04 (bs, 1H); 11.39 (bs, 1H); 7.84 (s, 1H); 7.68 (m, 1H); 7.58 (d, 2H); 7.38 (m, 1H); 7.26 (d, 2H); 4.43 (m, 2H); 4.26 (m, 2H); 2.78 (s, 3H); 2.41 (s, 3H); 2.10 (s, 3H). |
| 46 | 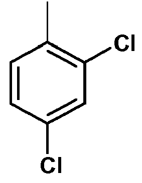 | 2,4-dichlorobenz aldehyde | 3-Chloro-5-(4-{3-[[(2,4-dichlorophenyl)methyl](m ethyl)amino]-1-propyn-1-yl}phenyl)-2 ,6-dimethyl-4(1*H*)-pyridinone hydrochloride | ¹H NMR (δ, ppm, DMSO-d₆): 12.10 (bs, 1H); 7.91 (d,1H); 7.78 (m, 1H); 7.58 (m, 1H); 7.53 (d, 2H); 7.26 (d, 2H); 4.56 (m, 2H); 4.39 (bs, 2H); 2.82 (s, 3H); 2.41 (s, 3H); 2.10 (s, 3H). |
| 47 | 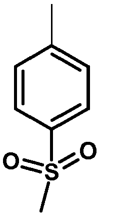 | 4-methylsulpho nylbenzaldeh yde | 3-Chloro-2,6-dimethyl-5-{4-[3-(methyl{[4-(methylsulfonyl)phenyl]m ethyl}amino)-1-propyn-1-yl]phenyl}-4(1*H*)-pyridinone hydrochloride | ¹H NMR (δ, ppm, DMSO-d₆): 11.91 (bs, 1H); 8.03 (d, 2H); 7.91 (d, 2H); 7.57 (d, 2H); 7.26 (d, 2H); 4.52 (m, 2H); 4.27 (m, 2H); 3.24 (s, 3H); 2.82 (s, 3H); 2.40 (s, 3H); 2.09 (s, 3H). |
| 48 | 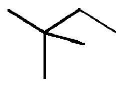 | 3,3-dimethylbuty raldehyde | 3-Chloro-5-(4-{3-[(3, 3-dimethylbutyl)(methyl)am ino]-1-propyn-1-yl}phenyl)-2 ,6-dimethyl-4(1*H*)-pyridinone hydrochloride | ¹H NMR (δ, ppm, DMSO-d₆): 11.89 (bs, 1H); 7.50 (d, 2H); 7.24 (d, 2H); 4.22 (m, 2H); 2.76 (m, 2H); 2.49 (s, 3H); 2.39 (s, 3H); 2.06 (s, 3H); 1.58 (m, 2H); 0.92 (s, 9H). |
| 49 | 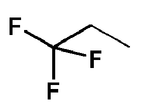 | 3,3,3-trifluoropropa nal | 3-Chloro-2,6-dimethyl-5-(4-{3-[methyl(3,3,3-trifluoropropyl)amino]-1-propyn-1-yl}phenyl)-4(1*H*)-pyridinone hydrochloride | ¹H NMR (δ, ppm, DMSO-d₆): 11.95 (bs, 1H); 7.52 (d, 2H); 7.24 (d, 2H); 4.37 (m, 2H); 2.90 (m, 2H); 2.86 (m, 2H); 2.49 (s, 3H); 2.39 (s, 3H); 2.08 (s, 3H). |
| 50^{b} | 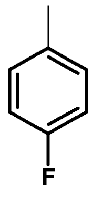 | 4-fluorobenzal dehyde | 3-Chloro-5-(4-{3-[[(4-fluorophenyl)methyl](met hyl)amino]-1-propyn-1-yl}phenyl)-2,6-dimethyl-4(1*H*)-pyridinone | ¹H NMR (δ, ppm, DMSO-d₆): 11.56 (bs, 1H); 7.45 (d, 2H); 7.36 (m, 2H); 7.18 (d, 2H); 7.15 (m, 2H); 3.57 (bs, 2H); 3.51 (bs, 2H); 2.37 (s, 3H); 2.27 (s, 3H); 2.07 (s, 3H). |

| | | | | |
|---|---|---|---|---|
| ^{a} 1 equivalent more of NaBH₃CN and aldehyde and 2 equivalent more of AcOH were added to complete the reaction. ^{b} the product was isolated as neutral amine form without addition of HCl. | | | | |

### Example 51

### 3-Chloro-2,6-dimethyl-5-(4-{3-[methyl({4-[(trifluoromethyl)oxyl]phenyl}methyl)amino]propyl}phenyl)-4(1H)-pyridinone

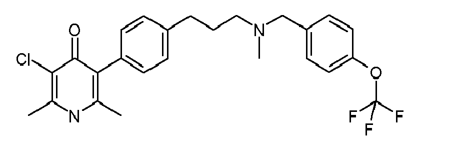

To a solution of Example 1 (50 mg) in MeOH were added one drop of 1H HCl and a pinch of palladium 10% w/w on activated charcoal. The mixture was hydrogenated at 20 psi of pressure for 1 h. The catalyst was removed by filtration and the solvent evaporated to dryness under vacuum to afford 43 mg of the title compound.
¹H NMR (δ, ppm, DMSO): 11.56 (s, 1H); 7.44-7.28 (m, 4H); 7.18-7.03 (m, 4H); 3.49 (s, 2H); 2.62-2.57 (m, 2H); 2.37 (s, 5H); 2.12 (s, 3H); 2.04 (s, 3H); 1.80-1.76 (m, 2H). [ES MS] m/z: 477.10 (MH+).

### Example 52

### 3-Chloro-2,6-dimethyl-5-{4-[3-(4-{4-[(trifluoromethyl)oxy]phenyl}-1-piperidinyl)propyl]phenyl}-4(1H)-pvridinone hydrochloride

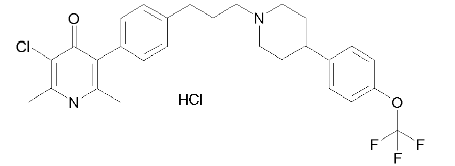

The crude Example 11 was dissolved in 5 ml of MeOH and 0.5 ml of 1 N HCl, then were added 6.0 mg of palladium 10% w/w on active charcoal. The mixture was hydrogenated at 3 bars overnight. The catalyst was removed by filtration, the solvent concentrated under vacuum and the residue, purified by column chromatography on silica gel, eluting with MeOH-CH₂Cl₂-Et₃N 1:98:1 to 10:89:1 to afford a crude free amine. It was suspended in CH₂Cl₂ and washed with 1 N Na₂CO₃. The layers were separated and the organic one dried over Na₂SO₄, filtered and concentrated to dryness to give 26 mg of material, which was dissolved in MeOH, treated with 0.1 ml of 4.0 N HCl in dioxane and concentrated to dryness to afford 27 mg (49%) of the title compound as a pale yellow solid.
¹H NMR (δ, ppm, DMSO-d₆): 12.00 (s, 1H); 10.22 (s, 1H); 7.37 (d, 2H); 7.33 (d, 2H); 7.37 (d, 2H); 7.13 (d, 2H); 3.58 (d, 2H); 3.14-3.00 (m, 4H); 2.93-2.82 (m, 1H); 2.68 (t, 2H); 2.41 (s, 3H); 2.09 (s, 3H); 2.06-1.97 (m, 4H). [ES MS] m/z: 519 (MH+).

### Example 53

### 3-Chloro-2,6-dimethyl-5-(4-{3-[methyl({4-[(trifluoromethyl)oxy]phenyl}methyl) amino]-1-propyn-1-yl}phenyl)-4-pyridinyl methyl[2-(methylamino)ethyl]carbamatehydrochloride

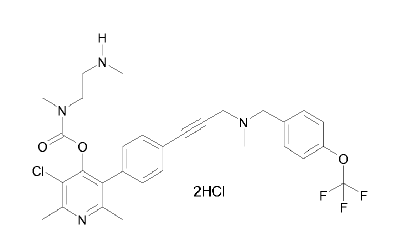

To a suspension of 3-chloro-2,6-dimethyl-5-(4-{3-[methyl({4-[(trifluoromethyl)oxy]phenyl} methyl)amino]-1-propyn-1-yl}phenyl)-4(1*H*)-pyridinone hydrochloride (1g, a synthesis of which is described as Example 19) and 1,1-dimethylethyl {2-[(chlorocarbonyl)(methyl) amino]ethyl}carbamate (0.637 g, a synthesis of which is described as Intermediate 55) in dry pyridine (40 ml) was added DIPEA (1.74 ml) and the mixture heated to 100 °C. After 1h of heating, the dark solution was allowed to cool to room temperature, then concentrated to dryness under vacuum, the resulting dark green residue treated with tert-butyl-methyl-ether and the resulting crystalline solid discharged. The solution was concentrated again and the same process repeated to give a new solid which was discharged and an organic solution which was concentrated to dryness to afford 1.03g of an orangish oily material which was used for the next step without further purification.

The above described oily material was dissolved in dry methanol (5 ml) to give a solution to which was added carefully at 4°C (ice-water bath) acetyl chloride (3.5 ml) dropwise. Once the addition concluded, the mixture was allowed to warm to room temperature then stirred overnight. Removal of the solvent under vacuum gave a foamy material which was dissolved in a small amount of 2-propanol and added dropwise onto 100 ml of tert-butylmethyl-ether with vigorous stirring. The white solid precipitated was filtered and dried under nitrogen to afford 0.9g of the title compound as an off-white powder.
¹H NMR (δ, ppm, DMSO-d₆): 11.82 (bs, 1H); 9.05 (2bs, 2H); 7.81(d, 2H); 7.68(bs, 2H); 7.49(d, 2H); 7.34(bs, 2H); 4.45 (bd, 2H); 4.26 (bd, 2H); 3.39 (bs, 2H); 2.80 (bs, 8H); 2.60 (s, 3H); 2.26 (s, 3H); [ES MS] m/z: 589 (MH+).

### Example 54

### 3-Chloro-2,6-dimethyl-5-{4'-[(4-{4-[(trifluoromethyl)oxy]phenyl}-1-piperidinyl) methyl]-4-biphenylyl}-4-pyridinyl [2-({[(1,1-dimethylethyl)oxy]carbonyl} amino)ethyl]methylcarbamate

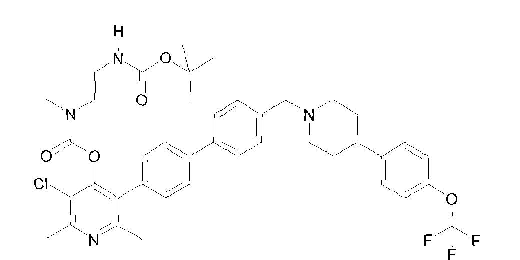

3-Chloro-2,6-dimethyl-5-{4'-[(4-{4-[(trifluoromethyl)oxy]phenyl}-1-piperidinyl)methyl]-4-biphenylyl}-4(1*H*)-pyridinone hydrochloride (0.1 g, a synthesis of which is described as Example 33) and 1,1-dimethylethyl {2-[(chlorocarbonyl)(methyl)amino]ethyl}carbamate (0.045g, a synthesis of which is described as Intermediate 55) were dissolved in a mixture of dry pyridine (5 ml) and DIPEA (0.140 ml). After stirring at room temperature for 1 minute, it was heated to reflux. The yellow solution thus formed was heated for 2h, then concentrated to dryness. The residue was dissolved in ethyl acetate (30 ml) and washed with 1 N HCl (2x30 ml), water (30 ml) and brine (30 ml), then dried over Na₂SO₄. Elimination of the solvent gave a yellowish oil which was purified by preparative TCL, eluting with dichloromethane/methanol v/v 10:1. The product was then extracted from the gel by using ethyl acetate and filtered, then concentrated to dryness. 0.0771 g of the title compound were obtained as a yellowish foam.
¹H NMR (δ, ppm, CDCl₃): 7.6 (m, 4H); 7.4 (bd, 2H); 7.2 (m, 4H); 7.1 (bd, 2H); 3.55 (bs, 2H); 3.4-2.9 (m, 8H); 2.8 and 2.7 (2bs, 3H); 2.65 (s, 1H); 2.60(m, 3H); 2.45(m, 1H); 2.3(m, 3H); 2.1(m, 2H); 1.75(m, 3H); 1.6(m, 3H); 1.4(m, 9H).

### Example 55

### 3-Chloro-2,6-dimethyl-5-{4'-[(4-{4-[(trifluoromethyl)oxy]phenyl}-1-piperidinyl)methyl]-4-biphenylyl}-4-pyridinyl methyl[2-(methylamino) ethyl]carbamate

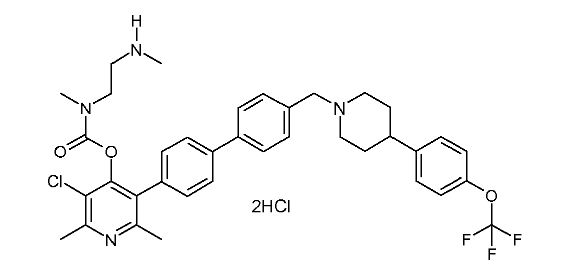

3-Chloro-2,6-dimethyl-5-{4'-[(4-{4-[(trifluoromethyl)oxy]phenyl}-1-piperidinyl)methyl]-4-biphenylyl}-4-pyridinyl [2-({[(1,1-dimethylethyl)oxy]carbonyl}amino)ethyl]
methylcarbamate (0.077g, a synthesis of which is described as Example 54) was dissolved in dry methanol (4 ml) and cooled in an ice-water bath. To this solution was added carefully acetyl chloride (0.5 ml) and the mixture stirred for 1 h. An additional amount of acetyl chloride (0.5 ml) was added and the mixture stirred for 2h, then concentrated to dryness under vacuum. To the residue obtained were added successively small amounts of tert-butylmethyl ether, methanol and acetone in order to made it solid, but it remained as a semisolid material. Elimination of the solvents to dryness gave a solid which was vacuum dried to afford 63mg of the title compound as a beige powder.
¹H NMR (δ, ppm, DMSO-d₆): 11.21 (bs, 1H); 9.05 and 8.98 (2bs, 2H); 7.9-7.7(2m, 6H); 7.35(m, 6H); 4.35 (bd, 2H); 3.41 (m, 4H); 2.9-2.7 (m, 6H); 2.62 (s, 3H); 2.33 (s, 6H); [ES MS] m/z: 681 (MH+).

### Example 56

### 3-Chloro-2,6-dimethyl-5-{4'-[(4-{4-[(trifluoromethyl)oxy]phenyl}-1-piperidinyl)methyl]-4-biphenylyl}-4-pyridinyl acetate hydrochloride

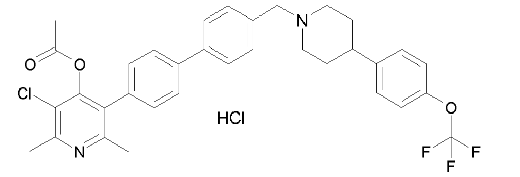

To a solution of 3-chloro-2,6-dimethyl-5-{4'-[(4-{4-[(trifluoromethyl)oxy]phenyl}-1-piperidinyl)methyl]-4-biphenylyl}-4(1*H*)-pyridinone hydrochloride (110 mg, a synthesis of which is described as Example 33) in dry pyridine (4 ml) was added acetic anhydride (0.517 ml) and the mixture heated under reflux (∼90°C) for 30 minutes. The crude mixture was diluted with dichloromethane and washed with 1 N HCl (x3) and brine (x1). The organic layer was dried over sodium sulphate and evaporation of the solvent gave the title compound (60 mg).
¹H-NMR(δ, ppm, DMSO-d₆): 10.34 (s, 1H); 7.86 (m, 4H); 7.7 (m, 2H); 7.34 (m, 6H); 4.38 (m, 2H); 3.47 (m, 3H); 3.07 (m, 2H); 2.86 (m, 1H); 2.60 (s, 3H); 2.30 (s, 3H); 2.04 (s, 3H); 2.0 (m, 3H); [ES+ MS] m/z 609 (MH⁺). 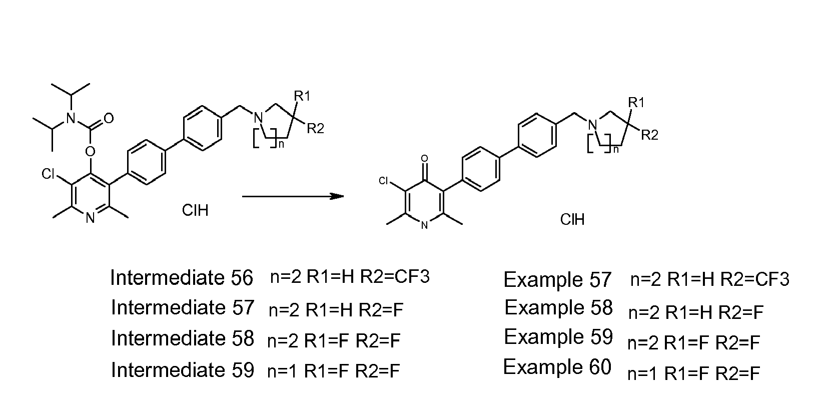 Examples 57-60 were prepared by an analogous method to Method B described for Example 33, replacing Intermediate 54 with the appropriate carbamate shown in Table 12. After cooling to room temperature, solvent was removed under reduced pressure and crude was triturated with acetonitrile.

**Table 12**

| **Example** | **R1** | **R2** | **n** | **Starting Carbamate** | **Chemical name** | **Physical Data** |
|---|---|---|---|---|---|---|
| 57 | H | CF3 | 2 | Intermediate 56 | 3-chloro-2,6-dimethyl-5-(4'-{[4-(trifluoromethyl) -1-piperidinyl]meth yl}-4-biphenylyl)-4(1*H*)-pyridinone hydrochloride | ¹H NMR (δ, ppm, DMSO-d₆): 12.15 (s, 1H); 11.02 (s, 1H); 7.82-7.79 (m, 2H); 7.72-7.69 (m, 4H); 7.30 (d, 2H); 4.31 (m, 2H); 3.46 (d, 2H); 3.03-2.90 (m, 2H); 2.73-2.58 (m, 1H); 2.43 (s, 3H); 2.15 (s, 3H); 2.03-1.91 (m, 4H). [ES MS] m/z: 475 (MH+). |
| 58 | H | F | 2 | Intermediate 57 | 3-chloro-5-{4'-[(4-fluoro-1-piperidinyl)meth yl]-4-biphenylyl}-2,6-dimethyl-4(1*H*)-pyridinone hydrochloride | ¹H NMR (δ, ppm, DMSO-d₆): 12.57 (s, 1H); 11.16 (s, 1H); 7.81-7.78 (d, 2H); 7.74-7.72 (d, 4H); 7.33-7.30 (d, 2H); 5.08-4.92 (d, 1H); 3.4-2.9 (m, 3H); 2.18 (s, 3H) [ES MS] m/z: 462 (MH+). |
| 59 | F | F | 2 | Intermediate 58 | 3-chloro-5-(4'-{[4-(1,1-dimethylethyl)-1-piperidinyl]meth yl}-4-biphenylyl)-2,6-dimethyl-4(1*H*)-pyridinone hydrochloride | ¹H NMR (δ, ppm, DMSO-d₆): 12.31 (br s, 1H); 11.50 (br s, 1H); 7.82-7.79 (m, 2H); 7.74-7.70 (m, 4H); 7.30 (d, 2H); 3.48-3.44 (m, 2H); 3.18-3.14 (m, 2H); 2.45 (s, 3H); 2.40-2.30 (m, 4H); 2.16 (s, 3H). [ES MS] m/z: 443 (MH+). |
| 60 | F | F | 1 | Intermediate 59 | 3-chloro-5-{4'-[(3,3-difluoro-1-pyrrolidinyl)met hyl]-4-biphenylyl}-2,6-dimethyl-4(1*H*)-pyridinone hydrochloride | ¹H NMR (δ, ppm, DMSO-d₆): 12.31 (br, 2H); 7.82-7.79 (m, 2H); 7.74-7.69 (m, 4H); 7.30 (d, 2H); 4.49 (s, 2H); 3.83 (m, 2H); 3.56 (m, 2H); 2.62 (m, 2H); 2.44 (s, 3H); 2.15 (s, 3H). [ES MS] m/z: 429(MH+). |

### Example 61

### 3-chloro-6-(hydroxymethyl)-2-methyl-5-{4'-[(4-{4-[(trifluoromethyl)oxy]phenyl}-1-piperidinyl)methyl]-4-biphenylyl}-4(1H)-pyridinone

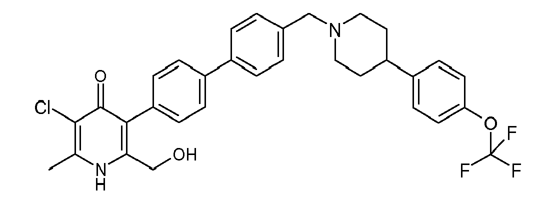

To a solution of 4-{4-[(trifluoromethyl)oxy]phenyllpiperidine (51.3 mg) in a mixture of dichloroethane (2ml) and tetrahydrofurane (2ml) under argon atmosphere, was added a solution of Intermediate 64 (61.7 mg) in tetrahydrofurane (4ml). The mixture was stirred 15 minutes and then triacetoxyborohydride (44 mg) was added portionwise at room temperature. As the reaction was not completed after 12, 24 and 36 hours 3x18 mg of triacetoxyborohydride were added consecutively. The mixture was concentrated to dryness under vacuum. The resulting residue was treated with water (20ml) and extracted with diethylacetate (3x20ml). The organic phase was washed with brine (10 ml), dried over Na₂SO₄, filtered and concentrated to afford 72 mg of a crude product that was purified by column chromatography on silica gel eluting with mixtures dichloromethane/methanol. 18 mg of the title compound were obtained as a white solid.
¹H-NMR(δ, ppm, DMSO-d₆): 11.53(bd, 1H); 7.66(d, 4H); 7.40(m, 4H); 7.27(m, 4H); 5.57(bs, 1H); 4.26(s, 2H); 3.54(s,2H); 2.93(d,2H); 2.46(s, 3H); 2.07(m, 2H); 1.73(m, 4H). [ES⁺ MS] m/z 583 (MH⁺)

### Example 62

### 3-chloro-6-(hydroxymethyl)-2-methyl-5-{4'-[(4-{4-[(trifluoromethyl)oxy]phenyl}-1-piperidinyl)methyl]-4-biphenylyl}-4(1H)-pyridinone hydrochloride.

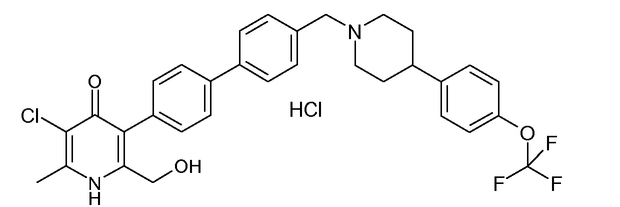

A solution of Intermediate 69 (290 mg) in a mixture methanol (20ml) and HCl 2N (10ml) was refluxed for 3 hours. After cooling to room temperature and dried under vacuum. the title compound was obtained as a white powder impurified with a 5% of diisopropilamine hydrochloride. The crude product was dissolved in NaOH 1 N (15 ml) under stirring. The free base was extracted with dichloromethane (4x30 ml), the organic layer was washed with water (2x50 ml) and brine (2x10 ml), dried over MgSO₄ and the solvent eliminated to dryness (diisopropilamine was eliminated in this process).
200 mg of the free base were dissolved in methanol (15 ml), HCl 2N (6ml). After 1h at room temperature, the solvents were eliminated to dryness giving 220 mg of the title compound as a pure white solid.
¹H-NMR(δ, ppm, DMSO-d₆): 11.74(bs, 1H); 10.64(bs, 1H); 7.81(m, 2H); 7.71(m, 4H); 7.34(m, 6H); 4.37(bs, 2H); 4.27(s, 2H); 3.47(m, 2H); 3.06(m, 2H); 2.87(m,1H); 2.48(s, 3H); 1.99(m, 4H).
[ES⁺ MS] m/z 583 (MH⁺)

### Example 63

### 3-chloro-2-(hydroxymethyl)-6-methyl-5-{4'-[(4-{4-[(trifluoromethyl)oxy]phenyl}-1-piperidinyl)methyl]-4-biphenylyl}-4(1H)-pyridinone hydrochloride

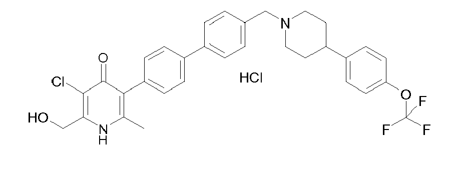

A solution of Intermediate 70 (245 mg) in a mixture methanol (25ml) and HCl 2N (12ml) was refluxed for 3 hours. After cooling to room temperature and dried under vacuum. the title compound was obtained as a white powder impurified with a 5% of diisopropilamine hydrochloride. The crude product was dissolved in NaOH 1 N (5 ml) under stirring. The free base was extracted with dichloromethane (3x20 ml), the organic layer was washed with water (30 ml) and brine (30 ml), dried over MgSO₄ and the solvent eliminated to dryness (diisopropilamine was eliminated in this process).
110 mg of the free base were dissolved in methanol (10 ml), HCl 2N (10ml). After 1h at room temperature, the solvents were eliminated to dryness giving 120 mg of the title compound as a pure white solid.
¹H-NMR(δ, ppm, DMSO-d₆): 11.51(bs, 1H); 10.58(bs, 1H); 7.82(m, 2H); 7.71(m, 4H); 7.34(m, 6H); 4.61 (s, 2H); 4.37(bs, 2H); 3.48(m, 2H); 3.06(m, 2H); 2.87(m,1H); 2.19(s, 3H); 1.99(m, 4H).
[ES⁺ MS] m/z 583 (MH⁺)

### Example 64

### 5-chloro-2,6-dimethyl-6'-{4-[(4-{4-[(trifluoromethyl)oxy]phenyl}-1-piperidinyl)methyl]phenyl}-3,3'-bipyridin-4(1H)-one hydrochloride

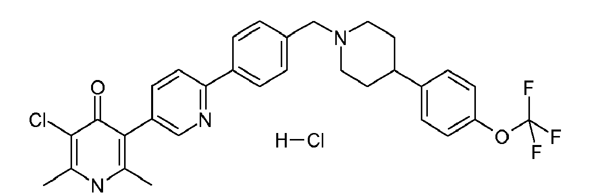

To a solution of 50 mg of Intermediate 75 and 54.3 mg of 4-{4-[(trifluoromethyl)oxy]phenyl}piperidine in a mixture of DCM:MeOH (5ml:5ml) was added 50 µL of acetic acid. To this mixture sodium triacetoxyborohydride was added portionwise until the reaction was completed. The reaction mixture was concentrated and the solid was partitioned between DCM and water, the organic layer was washed with 1 N NaOH, dried over Na₂SO₄, and evaporated in vacuo to give a yellow solid. This solid was dissolved in 10ml of DCM, 100 µL of hydrogen chloride (dioxane solution 4.0M) was added, it was stirred overnight. The reaction mixture was concentrated; the residue was triturated with ethyl ether and DCM to afford 11 mg of the title compound as a yellow solid.
¹H NMR (δ, ppm, DMSO-d₆): 12.01 (s, 1H); 10.92 (s, 1H); 8.50 (s, 1H); 8.21 (d, 2H); 8.05 (d, 1H); 7.76(m, 3H); 7.34 (m, 4H); 4.37 (m, 2H); 3.50-1.85 (m, 15H). [ES MS] m/z: 568 (MH+).

### Example 65

### 5-chloro-2,6-dimethyl-6'-(4-{[4-(trifluoromethyl)-1-piperidinyl]methyl}phenyl)-3,3'-bipyridin-4(1H)-one

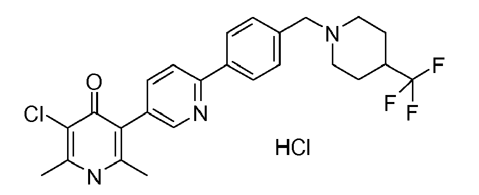

In a round-bottomed flask, 4-(trifluoromethyl)piperidine hydrochloride (73.9 mg, 0.390 mmol) and DIPEA (67 µl, 0.391 mmol) in tetrahydrofuran (THF) (1 ml) was stirred for 30min under nitrogen atmosphere, then, a solution of intermediate 4 (110mg) in tetrahydrofurane (1ml) and Dichloroethane (4ml) was added. 2 hours later, SODIUM TRIACETOXYBOROHYDRIDE (83mg) was added and the reaction mixture was stirred overnight under nitrogen atmosphere. Then LCMS showed reaction wasn't complete. 1.2 eq of amine (4-(trifluoromethyl)piperidine Hydrochloride) and 1.2eq of DIPEA were dissolved in 1ml of anh.THF, the mixture was stirred for 1h and then added to the reaction. 1h later 1.2eq of NaBH(OAc)3 were added and the reaction was stirred overnight. LCMS showed reaction was almost complete. The reaction was quenched with 6 drops of water, evaporated in vacuo to give a crude that was triturated with ACN, filtered and collected as a beige solid.
Analysis by 1H NMR in DMSO-D6 is consistent with desired product. ¹H-NMR (δ, ppm, DMSO-d₆)
The compound was dissolved in MeOH and an excess of HCl 2N to form the hydrochloride salt .It was triturated with ACN and then with DCM, and filtered affording the title compound.
¹H-N MR (δ, ppm, DMSO-d₆): 12.29 (s, 1H); 11.32(s, 1H); 8.58-8.57 (d, 1H); 8.22-8.19(d, 2H); 8.16-8.13 (d, 1H); 7.95-7.92 (m, 1H); 7.80-7.78 (d, 2H); 4.36-4.34 (d, 2H); 3.46-3.43 (d, 2H); 2.98 (m, 2H); 2.53 (s, 6H); 2.44 (s, 3H); 1.99 (m, 4H)
[ES MS] m/z: 512 (MH+).

### Example 66

### 5-chloro-6'-{4-[(4,4-difluoro-1-piperidinyl)methyl]phenyl}-2,6-dimethyl-3,3'-bipyridin-4(1H)-one

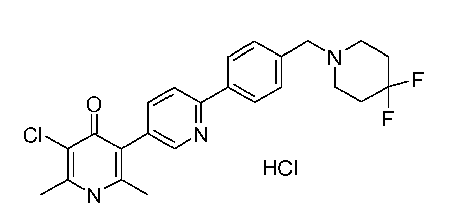

This compound was prepared using an analogous procedure to that described for Example 65, replacing 4-(trifluoromethyl)piperidine hydrochloride with 4,4-difluoropiperidine hydrochloride as the amine hydrochloride salt.
¹H-NMR (δ, ppm, DMSO-d₆): 12.67(s, 1H); 12.11 (s, 1H); 8.63(d, 2H); 8.23-8.20 (m, 3H); 8.10-8.07(m, 1H); 7.86-7.84(d, 2H); 2.74-2.53(m, 2H); 2.47(s, 3H); 2.40-2.29(m, 2H) [ES MS] m/z: 480 (MH+).

### Example 67

### 5-chloro-6'-{1,1-difluoro-2-[4-(trifluoromethyl)-1-piperidinyl]ethyl}-2,6-dimethyl-3,3'-bipyridin-4(1H)-one

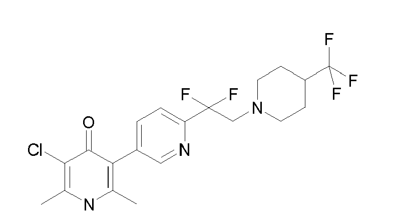

Intermediate 81 (11 mg, 0.019 mmol) was dissolved in 1ml MeOH and 1ml 1 N HCl was added. The solution was stirred at 80°C overnight. Solvent was removed under reduced pressure to yield green oil. Cooled acetonitrile was added, but the product precipitated together with the impurity. Acetonitrile was removed under reduced pressure. The crude was dissolved in CH₂Cl₂ and filtered through a 200mg silica gel cartridge eluting with CH₂Cl₂: MeOH. Product was dissolved in CH₂Cl₂: MeOH (1ml) and washed with saturated NaHCO₃ (2ml) in a separator tube. Layers were separated and organic layer was again back-extracted with CH₂Cl₂: MeOH (4x3ml). Combined organic layers were dried over anhydrous sodium sulphate and concentrated to yield 5 mg of the title compound as a white solid.
¹H-NMR (δ, ppm, CD₃OD): 8.50 (s, 1H); 7.87-7.83 (m, 1H); 7.78-7.75 (m, 1H); 3.23-3.18 (m, 2H); 2.98-2.94 (m, 2H); 2.51 (s, 3H); 2.31 (m, 2H); 2.20 (s, 3H); 1.74-1.70 (m, 2H); 1.50-1.35 (m, 2H); 0.87 (m, 2H). [ES MS] m/z: 450 (MH+).

### Example 68

### 5-chloro-2,6-dimethyl-6'-({2-[4-(trifluoromethyl)-1-piperidinyl]ethyl}oxy)-3,3'-bipyridin-4(1H)-one hydrochloride

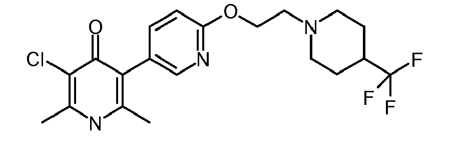

4-(trifluoromethyl)piperidine hydrchloride (ALDRICH, 88 mg, 0.574 mmol) was dissolved in 2ml anhydrous DCE and DIPEA (FLUKA, 74.2 mg, 0.574 mmol) was added. The free base was then added to a stirring solution of [(5'-chloro-2',6'-dimethyl-4'-oxo-1',4'-dihydro-3,3'-bipyridin-6-yl)oxy]acetaldehyde (Intermediate 85, 140 mg, 0.478 mmol) in 5ml anhydrous DCE under nitrogen atmosphere at 0°C. Finally, sodium cyanoborohydride (ALDRICH, 36.1 mg, 0.574 mmol) was added and the mixture was allowed to warm to room temperature overnight. Solvent was removed, the crude was dissolved in CH₂Cl₂ and washed with saturated NH₄Cl and saturated NaCl. Organic layer was dried over anhydrous sodium sulphate, filtered and concentrated. The crude was purified by flash-master chromatography using Dichloromethane/Methanol as eluent. The obtained solid was dissolved in MeOH and 1ml 1 N HCl was added. The mixture was stirred for 5 hours to get the corresponding Hydrochloride. Solvent was removed and cold acetonitrile was added. The white precipitated was filtered to yield 60 mg of the title compound.
¹H-NMR (δ, ppm, DMSO-d₆): 12.47 (br s, 1H); 11.13 (br s, 1H); 8.00 (s, 1H); 7.61 (dd, 1H); 6.92 (d, 1H); 4.69 (m, 2H); 3.70-3.51 (m , 4H); 2.65 (m, 1H); 2.44 (s, 3H); 2.16 (s, 3H); 2.00 (m, 4H). [ES MS] m/z: 430 (MH+).

### Biological Assays

The compounds of this invention may be tested in one of several biological assays to determine the concentration of compound which is required to have a given pharmacological effect.
In vitro anti-malarial activity against *Plasmodium falciparum.*
IC₅₀ assay / [³H]-Hypoxanthine assay

### I. Materials

### Parasite.

### Plasmodium falciparum strains.

### Culture medium.

The culture medium comprised RPMI 1640 with 25mM HEPES, sodium bicarbonate and glutamine (GIBCO^{™} *cat. ref*.: 52400), supplemented with 10% of pooled human sera AB (Irvine Scientific, *cat. ref.:* 5009) and HT supplement (0.15 mM hypoxanthine and 24 µM thymidine), (GIBCO^{™} *cat. ref.:* 41065). Human sera were decomplemented 30 min. at 56°C, aliquoted and stored frozen at -20°C until use in this culture medium.

This culture medium ("complete medium") was usually prepared fresh just before use and pre-warmed to 37°C.

### Red Blood Cells.

Red blood cells O+ stock suspensions were prepared from whole blood bags coming from incomplete blood donation, provided by the Spanish Red Cross (<25 days after sampling). This "whole blood" was aliquoted and stored at 4°C.

To prepare red blood cells for the assay, the whole blood was centrifuged and washed 3 times with RPMI without serum. The upper phase, containing white blood cells was removed. The washed red blood cells were kept as a 50 % suspension in complete medium. The prepared cells were stored at 4°C and were employed in the assay at any time up to 4 days after preparation.

### II. Compounds.

### Compound Preparation

Test compounds were dissolved at 2 mg/ml in 100% DMSO on the day of the assay. If necessary, complete dissolution was achieved by gentle heating (the mixture was heated at a temperature <37°C) and sonication (sonication bath).

Before test compounds were added to the parasites, the percentage of DMSO in the compound solution was reduced by further dilutions of the solution with culture medium prepared in the same way as described above for complete medium, but which did not contain hypoxanthine. The final concentration of DMSO in the assay plates was not permitted to exceed 0.2%, so that it did not produce any detectable undesired effects on the development of the parasite. For IC₅₀ determinations, 10 serial 2-fold dilutions were prepared in complete medium in the presence of a constant amount of DMSO. Any obvious signs of insolubility of the stock solutions in 100% DMSO or precipitation when these solutions were diluted in assay media, were recorded.

### III. Plasmodium falciparum culture (parasite)

*Plasmodium falciparum* strains were maintained in complete medium at an hematocrit of 5% in continuous culture using a method adapted from that published by Trager and Jensen(1) and Traeger(2).

The parasitemia was calculated by counting the percentage of parasitized erythrocytes by optical microscopy. Thin films of blood were made every day from each culture flask, fixed with methanol and stained for 10 min. in Giemsa (Merck, *cat. ref.:* 1.09204) at 10 % in buffered water pH 7.2. The glass slides were observed and counted with an optical microscope (Nikon, Eclipse E200) equipped with a 100 X immersion oil objective.

The culture was maintained at an hematocrit of 5%, with a daily change of medium and was diluted when parasitemia had reached about 5%. The parasite population was asynchronous, composed of a stable proportion (≅ 70%) of young trophozoites (ring forms) and showed a regular rate of growth of 3 to 3.5 times the initial number of parasites daily.

Growth was achieved in culture flasks (canted neck, Corning) incubated at 37°C under low oxygen atmosphere (5 % CO₂, 5% O₂, 95 % N₂).

### IV- IC₅₀ Assay

[³H] Hypoxanthine incorporation assay was conducted using a method adapted from Desjardins et al. (3). The assays were performed in 96 wells flat bottom microplates.
1. Serial dilutions of the test compounds (50µl of a 5X solution / well) were deposited in duplicate. Compounds of the invention were tested in this assay. Chloroquine and Atovaquone were used as control compounds for each assay.
2. The inoculum was prepared as a suspension of parasitized red blood cells (PRBCs) at 2.5% of hematocrit and 0.5% of parasitemia in culture medium prepared in the same way as described above for complete medium, but which did not contain hypoxanthine.
3. [³H]-Hypoxanthine (Amersham Biosciences, *cat. ref.:* TRK74) was added extemporaneously to the inoculum suspension at a concentration of 1 µCi/ml (equating to 0.25 µCi /well). 200 µl of the resulting suspension was distributed into each well (other than the control well H12 described below) leading to a final volume of 250 µl per well, at 2% of hematocrit and 0.5 % of parasitemia / well.
4. In each plate, 2 columns were reserved for control wells :
   - Column 11: ***Positive control wells:*** PRBCs with 0.2% DMSO - (i) to determine DMSO solvent effect on parasite growth (at a final concentration of 0.2%) and (ii) to compare with cultures treated with test compounds.
   - Column 12 (comprising wells A12-H12):
      A12-D12- ***Background value wells:*** Uninfected RBCs - blank control to obtain the background reading from RBCs without parasites.
      E12-G12- ***Solvent effect wells:*** PRBCs without DMSO - to determine DMSO solvent effect on PRBCs by comparing these wells with column 11 wells.
      H12-***Non-radioactive well:*** PRBCs with cold hypoxanthine - (i) to carry out a thin blood film to determine parasitemia value after incubation by microscopy and (ii) to ensure that the parasites have grown properly during the assay. (200 µl of inoculum suspension was prepared as described above (Items 2 and 3) but with non-tritiated hypoxanthine instead of [³H]-hypoxanthine, then added to this well to a final volume of 250 µl).
5. The plates were incubated for 48 hours at 37°C under low oxygen atmosphere. At the end of the assay, a thin film was made with the non-radioactive sample (well H12) for a visual control of the development of the parasites. Incorporation was stopped by freezing the plates overnight at -80°C.
6. The growth was quantified by measuring the level of incorporation of [³H]-hypoxanthine into the nucleic acids of the parasite. After thawing the plates, the content of the wells was harvested on glass fibre filters (Wallac, *cat. ref.:* 1450-421) with a semi-automated cell-harvester (Harvester 96, TOMTEC). The filters were dried and treated with a Melt-on scintillator (Meltilex^{®} A, PerkinElmer *cat. ref.:* 1450-441). Incorporation of radioactivity was measured with a β-counter (Wallac Microbeta, PerkinElmer).

The assays were repeated at least three independent times.

### V. Analysis of the data

The value of each well was corrected by subtracting the background value from the absolute value. Background was calculated for each plate as the average value in cpm of the uninfected control wells.

For each concentration of a given test compound, the average (mean) value of the duplicate samples was calculated.

For each concentration of each test compound, the percentage of inhibition was then calculated by comparison with the value obtained from a control well containing PRBCs which was diluted with DMSO to achieve the same well volume but in the absence of test compound. The control well used here is the column 11 well described above (taking a mean value over all column 11 wells).

Analysis of the data were performed using Excel and GraphPad Prism 3.0 software.
Results were expressed as the average ± standard deviation of at least 3 independent experiments performed on different days.

### VI. Bibliography

1. Trager W, Jensen JB. Human malaria parasites in continuous culture. Science. 1976 Aug 20;193(4254):673-5.
2. Trager W. Cultivation of malaria parasites. Methods Cell Biol. 1994;45:7-26.
3. Desjardins RE, Canfield CJ, Haynes JD, Chulay JD. Quantitative assessment of antimalarial activity in vitro by a semiautomated microdilution technique. Antimicrob Agents Chemother 1979 Dec;16(6):710-8.

### Results of in vitro activity assay

### In vitro assay

Examples 1-52 and 56-67 of the present invention described hereinabove were found to have an IC₅₀ value of less than 125 ng/ml. Example 68 was found to have an IC₅₀ value of 210 ng/ml. Examples 53-55 were not stable in the assay conditions.

## Claims

1. A compound of Formula I: 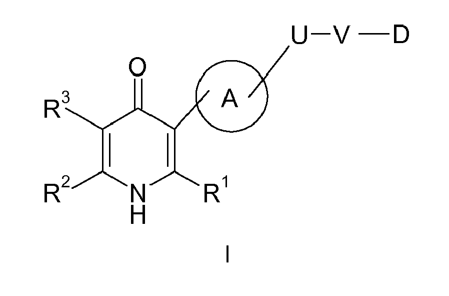 wherein:
R¹ and R² independently represent C₁₋₄alkyl or C₁₋₄alkylOH;
R³ represents halo;
A represents phenyl; pyridinyl; pyrimidinyl; or benzothienyl; wherein A is optionally substituted with halo;
U represents a bond; -C₂alkynyl-; phenyl; furanyl; or thienyl; wherein U is optionally substituted with halo or C₁₋₆alkyl;
V represents C₀₋₄alkylene or a C₁₋₄alkoxy linker group, either of which is optionally substituted with halo;
Either
i) D represents an 8-12-membered fused bicyclic ring system XY, wherein one fused ring X is linked to V and is either saturated or partially saturated and the other ring Y is either aromatic, saturated or partially saturated and wherein ring X contains one or two nitrogen atoms; and wherein D is optionally substituted with halo, CF₃, OCF₃, C₁₋₄alkyl or -SO₂C₁₋₄alkyl;
or
ii) D represents -E-W-Cy, wherein
E represents -N(R⁴)- or a 5-7-membered saturated or partially saturated heterocyclic ring containing one or two nitrogen atoms, wherein any nitrogen atom in the heterocyclic ring is a tertiary nitrogen, and wherein the heterocyclic ring is optionally substituted with with halo, or with one or more C₁₋₄alkyl groups;
R⁴ represents hydrogen, optionally substituted C₃₋₇cycloalkyl wherein C₃₋₇cycloalkyl is optionally substituted with one or more C₁₋₄alkyl groups, or optionally substituted C₁₋₆alkyl, wherein C₁₋₆alkyl is optionally substituted with a group selected from C₃-₇cycloalkyl; phenyl and C₁₋₄alkoxy;
W represents C₀₋₄alkylene; and
Cy represents phenyl; furanyl; thienyl; thiazolyl; pyrrolyl; pyridinyl or C₁₋₆alkyl; each of which is optionally substituted with halo, CF₃, OCF₃, C₁₋₄alkyl or -SO₂C₁₋₄alkyl, or Cy represents halo;
or a pharmaceutically acceptable salt, solvate, ester, carbamate or ether thereof; provided that when Cy represents C₁₋₆alkyl, U represents -C₂alkynyl- and V represents C₁₋₄alkylene or a C₁₋₄alkoxy linker group, either of which is optionally substituted with halo.

2. A compound according to claim 1 wherein R¹ represents methyl.

3. A compound according to claim 1 or claim 2 wherein R² represents methyl.

4. A compound according to any preceding claim wherein R³ represents chloro.

5. A compound according to any preceding claim wherein U represents a bond; -C₂alkynyl-; -phenyl attached to the pyridine ring and the group U in a para- or meta-orientation; -2,5-furanyl; or -2,5-thienyl.

6. A compound according to any preceding claim wherein V represents C₁₋₄alkylene or a C₁₋₄alkoxy linker group, either of which is optionally substituted with halo.

7. A compound according to any preceding claim wherein D represents 6-(trifluoromethyl)-3,4-dihydro-2(1H)-isoquinolinyl or 8-(trifluoromethyl)-3,4-dihydro-2(1H)-isoquinolinyl.

8. A compound according to any of claims 1 to 6 wherein D represents -E-C₀₋₄alkylene-Cy.

9. A compound according to any preceding claim wherein E represents -N(R⁴)-wherein R⁴ represents hydrogen, methyl, propyl, 2-methylpropyl, cyclopropyl, phenylmethyl, cyclopentylmethyl or 2-(methyloxy)ethyl.

10. A compound according to any of claims 1 to 8 wherein E represents E represents piperidinyl, 1,2,3,6-tetrahydro-4-pyridinyl or pyrrolidinyl.

11. A compound according to any preceding claim wherein W represents C₀alkylene, -CH₂- or -CH₂CH₂-.

12. A compound according to any preceding claim wherein Cy represents unsubstituted phenyl, 4-(methylsulfonyl)phenyl, fluorophenyl, chlorophenyl, (4-trifluoromethyl)oxyphenyl, (trifluoromethyl)phenyl, furanyl, thienyl thiazolyl, pyrrolyl, C₁₋₆alkyl or halo.

13. A compound according to claim 1, or a pharmaceutically acceptable derivative thereof, wherein the compound is selected from the group consisting of:
3-chloro-2,6-dimethyl-5-(4-{3-[methyl({4-[(trifluoromethyl)oxy]phenyl}methyl)amino]-1-propyn-1-yl}phenyl)-4(1H)-pyridinone;
3-chloro-2,6-dimethyl-5-{4-[3-(methyl{[4-(trifluoromethyl)phenyl]methyl}amino)-1-propyn-1-yl]phenyl}-4(1H)-pyridinone;
3-chloro-2,6-dimethyl-5-{4-[3-(methyl{[2-(trifluoromethyl)phenyl]methyl}amino)-1-propyn-1-yl]phenyl}-4(1H)-pyridinone;
3-(4-{3-[{[2,4-bis(trifluoromethyl)phenyl]methyl}(methyl)amino]-1-propyn-1-yl}phenyl)-5-chloro-2,6-dimethyl-4(1H)-pyridinone;
3-chloro-2,6-dimethyl-5-(4-{3-[methyl(phenylmethyl)amino]-1-propyn-1-yl}phenyl)-4(H)-pyridinone;
3-chloro-2,6-dimethyl-5-(4-{3-[propyl({4-[(trifluoromethyl)oxy]phenyl}methyl)amino]-1-propyn-1-yl}phenyl)-4(1H)-pyridinonetrifluoroacetate;
3-chloro-5-(4-{3-[cyclopropyl({4-[(trifluoromethyl)oxy]phenyl}methyl)amino]-1-propyn-1-yl}phenyl)-2,6-dimethyl-4(1H)-pyridinone;
3-chloro-2,6-dimethyl-5-(4-{3-[({4-[(trifluoromethyl)oxy]phenyl}methyl)amino]-1-propyn-1-yl}phenyl)-4(1H)-pyridinone;
3-chloro-2,6-dimethyl-5-(4-{3-[[2-(methyloxy)ethyl]({4-[(trifluoromethyl)oxy]phenyl}methyl)amino]-1-propyn-1-yl}phenyl)-4(1H)-pyridinone;
3-chloro-2,6-dimethyl-5-{4-[3-(4-phenyl-1-piperidinyl)-1-propyn-1-yl]phenyl}-4(1H)-pyridinone;
3-chloro-2,6-dimethyl-5-{4-[3-(4-{4-[(trifluoromethyl)oxy]phenyl}-1-piperidinyl)-1-propyn-1-yl]phenyl}-4(1H)-pyridinone hydrochloride;
3-(4-{3-[bis(phenylmethyl)amino]-1-propyn-1-yl}phenyl)-5-chloro-2,6-dimethyl-4(1H)-pyridinone hydrochloride;
3-chloro-2,6-dimethyl-5-{4-[3-(2-phenyl-1-piperidinyl)-1-propyn-1-yl]phenyl}-4(1H)-pyridinone hydrochloride;
3-chloro-2,6-dimethyl-5-(4-{3-[6-(trifluoromethyl)-3,4-dihydro-2(1H)-isoquinolinyl]-1-propyn-1-yl}phenyl)-4(1H)-pyridinone hydrochloride;
3-chloro-2,6-dimethyl-5-(4-{3-[8-(trifluoromethyl)-3,4-dihydro-2(1H)-isoquinolinyl]-1-propyn-1-yl}phenyl)-4(1H)-pyridinone hydrochloride;
3-chloro-2,6-dimethyl-5-{4-[3-methyl-3-(4-phenyl-1-piperidinyl)-1-butyn-1-yl]phenyl}-4(1H)-pyridinone;
3-chloro-2,6-dimethyl-5-(4-{3-[methyl(2-{4-[(trifluoromethyl)oxy]phenyl}ethyl)amino]-1-propyn-1-yl}phenyl)-4(1H)-pyridinone hydrochloride;
3-chloro-2,6-dimethyl-5-(4-{4-[methyl({4-[(trifluoromethyl)oxy]phenyl}methyl)amino]-1-butyn-1-yl}phenyl)-4(1H)-pyridinone hydrochloride;
3-chloro-2,6-dimethyl-5-(4-{3-[methyl({4-[(trifluoromethyl)oxy]phenyl}methyl)amino]-1-propyn-1-yl}phenyl)-4(1H)-pyridinone hydrochloride;
3-chloro-2,6-dimethyl-5-(4-{3-[propyl({4-[(trifluoromethyl)oxy]phenyl}methyl)amino]-1-propyn-1-yl}phenyl)-4(1H)-pyridinone;
3-chloro-2,6-dimethyl-5-(4-{3-[propyl({4-[(trifluoromethyl)oxy]phenyl}methyl)amino]-1-propyn-1-yl}phenyl)-4(1H)-pyridinone hydrochloride;
3-chloro-2,6-dimethyl-5-{4-[3-(4-{4-[(trifluoromethyl)oxy]phenyl}-1-piperidinyl)-1-propyn-1-yl]phenyl}-4(1H)-pyridinone trifluoroacetate;
3-chloro-2,6-dimethyl-5-(4-{[methyl({4-[(trifluoromethyl)oxy]phenyl}methyl)amino]methyl}phenyl)-4(1H)-pyridinone hydrochloride;
3-chloro-2,6-dimethyl-5-{4-[(4-{4-[(trifluoromethyl)oxy]phenyl}-1-piperidinyl)methyl]phenyl}-4(1H)-pyridinone hydrochloride;
3-chloro-2,6-dimethyl-5-(3-{[methyl({4-[(trifluoromethyl)oxy]phenyl}methyl)amino]methyl}phenyl)-4(1H)-pyridinone hydrochloride;
3-chloro-2,6-dimethyl-5-[4-(5-{[methyl({4-[(trifluoromethyl)oxy]phenyl}methyl)amino]methyl}-2-thienyl)phenyl]-4(1H)-pyridinone trifluoroacetate;
3-chloro-2,6-dimethyl-5-[4-(5-{[methyl({4-[(trifluoromethyl)oxy]phenyl}methyl)amino]methyl}-2-thienyl)phenyl]-4(1H)-pyridinone hydrochloride;
3-chloro-2,6-dimethyl-5-[4-(5-{[methyl({4-[(trifluoromethyl)oxy]phenyl}methyl)amino]methyl}-2-furanyl)phenyl]-4(1H)-pyridinone trifluoroacetate;
3-chloro-2,6-dimethyl-5-[4-(5-{[methyl({4-[(trifluoromethyl)oxy]phenyl}methyl)amino]methyl}-2-furanyl)phenyl]-4(1H)-pyridinone hydrochloride;
3-chloro-2,6-dimethyl-5-{3-[3-(4-{4-[(trifluoromethyl)oxy]phenyl}-1-piperidinyl)-1-propyn-1-yl]phenyl}-4(1H)-pyridinone trifluoroacetate;
3-chloro-2,6-dimethyl-5-(2-{[methyl({4-[(trifluoromethyl)oxy]phenyl}methyl)amino]methyl}-1-benzothien-5-yl)-4(1H)-pyridinone hydrochloride;
3-chloro-2,6-dimethyl-5-{4-[1-({4-[(trifluoromethyl)oxy]phenyl}methyl)-1,2,3,6-tetrahydro-4-pyridinyl]phenyl}-4(1H)-pyridinone hydrochloride;
3-chloro-2,6-dimethyl-5-{4'-[(4-{4-[(trifluoromethyl)oxy]phenyl}-1-piperidinyl)methyl]-4-biphenylyl}-4(1H)-pyridinone hydrochloride;
3-chloro-2,6-dimethyl-5-(3'-{[methyl({4-[(trifluoromethyl)oxy]phenyl}methyl)amino]methyl}-4-biphenylyl)-4(1H)-pyridinone hydrochloride;
3-chloro-2,6-dimethyl-5-(4'-{[methyl({4-[(trifluoromethyl)oxy]phenyl}methyl)amino]methyl}-4-biphenylyl)-4(1H)-pyridinone hydrochloride;
3-chloro-2,6-dimethyl-5-(4'-{[methyl({4-[(trifluoromethyl)oxy]phenyl}methyl)amino]methyl}-4-biphenylyl)-4(1H)-pyridinone trifluoroacetate;
3-chloro-2,6-dimethyl-5-(4-{3-[(2-methylpropyl)({4-[(trifluoromethyl)oxy]phenyl}methyl) amino]-1-propyn-1-yl}phenyl)-4(1H)-pyridinone hydrochloride;
3-chloro-5-(4-{3-[(cyclopentylmethyl)({4-[(trifluoromethyl)oxy]phenyl}methyl)amino]-1-propyn-1-yl}phenyl)-2 ,6-dimethyl-4(1H)-pyridinone hydrochloride;
3-chloro-2,6-dimethyl-5-(4-{3-[methyl(2-thienylmethyl)amino]-1-propyn-1-yl}phenyl)-4(1H)-pyridinone hydrochloride;
3-chloro-5-(4-{3-[[(2,4-difluorophenyl)methyl](methyl)amino]-1-propyn-1-yl}phenyl)-2,6-dimethyl-4(1H)-pyridinone hydrochloride;
3-chloro-5-(4-{3-[(2-furanylmethyl)(methyl)amino]-1-propyn-1-yl}phenyl)-2,6-dimethyl-4(1H)-pyridinone hydrochloride;
3-chloro-2,6-dimethyl-5-[4-(3-{methyl[(5-methyl-2-thienyl)methyl]amino}-1-propyn-1-yl)phenyl]-4(1H)-pyridinone hydrochloride;
3-chloro-2,6-dimethyl-5-(4-{3-[methyl(1,3-thiazol-2-ylmethyl)amino]-1-propyn-1-yl}phenyl)-4(1H)-pyridinone hydrochloride;
3-chloro-2,6-dimethyl-5-[4-(3-{methyl[(1-methyl-1H-pyrrol-2-yl)methyl]amino}-1-propyn-1-yl)phenyl]-4(1H)-pyridinone hydrochloride;
3-chloro-2,6-dimethyl-5-(4-{3-[methyl(3-thienylmethyl)amino]-1-propyn-1-yl}phenyl)-4(1H)-pyridinone hydrochloride;
3-chloro-5-(4-{3-[[(2,4-dichlorophenyl)methyl](methyl)amino]-1-propyn-1-yl}phenyl)-2,6-dimethyl-4(1H)-pyridinone hydrochloride;
3-chloro-2,6-dimethyl-5-{4-[3-(methyl{[4-(methylsulfonyl)phenyl]methyl}amino)-1-propyn-1-yl]phenyl}-4(1H)-pyridinone hydrochloride;
3-chloro-5-(4-{3-[(3,3-dimethylbutyl)(methyl)amino]-1-propyn-1-yl}phenyl)-2,6-dimethyl-4(1H)-pyridinone hydrochloride;
3-chloro-2,6-dimethyl-5-(4-{3-[methyl(3,3,3-trifluoropropyl)amino]-1-propyn-1-yl}phenyl)-4(1H)-pyridinone hydrochloride;
3-chloro-5-(4-{3-[[(4-fluorophenyl)methyl](methyl)amino]-1-propyn-1-yl}phenyl)-2,6-dimethyl-4(1H)-pyridinone;
3-chloro-2,6-dimethyl-5-(4-{3-[methyl({4-[(trifluoromethyl)oxy]phenyl}methyl)amino] propyl}phenyl)-4(1H)-pyridinone;
3-chloro-2,6-dimethyl-5-{4-[3-(4-{4-[(trifluoromethyl)oxy]phenyl}-1-piperidinyl)propyl]phenyl}-4(1H)-pyridinone hydrochloride;
3-chloro-2,6-dimethyl-5-(4-{3-[methyl({4-[(trifluoromethyl)oxy]phenyl}methyl)amino]-1-propyn-1-yl}phenyl)-4-pyridinyl methyl[2-(methylamino)ethyl]carbamate hydrochloride;
3-chloro-2,6-dimethyl-5-{4'-[(4-{4-[(trifluoromethyl)oxy]phenyl}-1-piperidinyl)methyl]-4-biphenylyl}-4-pyridinyl [2-({[(1,1-dimethylethyl)oxy]carbonyl}amino)ethyl]methylcarbamate;
3-chloro-2,6-dimethyl-5-{4'-[(4-{4-[(trifluoromethyl)oxy]phenyl}-1-piperidinyl)methyl]-4-biphenylyl}-4-pyridinyl methyl[2-(methylamino)ethyl]carbamate;
3-chloro-2,6-dimethyl-5-{4'-[(4-{4-[(trifluoromethyl)oxy]phenyl}-1-piperidinyl)methyl]-4-biphenylyl}-4-pyridinyl acetate hydrochloride;
3-chloro-2,6-dimethyl-5-(4'-{[4-(trifluoromethyl)-1-piperidinyl]methyl}-4-biphenylyl)-4(1H)-pyridinone hydrochloride;
3-chloro-5-{4'-[(4-fluoro-1-piperidinyl)methyl]-4-biphenylyl}-2,6-dimethyl-4(1H)-pyridinone hydrochloride;
3-chloro-5-(4'-{[4-(1,1-dimethylethyl)-1-piperidinyl]methyl}-4-biphenylyl)-2,6-dimethyl-4(1H)-pyridinone hydrochloride;
3-chloro-5-{4'-[(3,3-difluoro-1-pyrrolidinyl)methyl]-4-biphenylyl}-2,6-dimethyl-4(1H)-pyridinone hydrochloride;
3-chloro-6-(hydroxymethyl)-2-methyl-5-{4'-[(4-{4-[(trifluoromethyl)oxy]phenyl}-1-piperidinyl)methyl]-4-biphenylyl}-4(1H)-pyridinone;
3-chloro-6-(hydroxymethyl)-2-methyl-5-{4'-[(4-{4-[(trifluoromethyl)oxy]phenyl}-1-piperidinyl)methyl]-4-biphenylyl}-4(1H)-pyridinone hydrochloride;
3-chloro-2-(hydroxymethyl)-6-methyl-5-{4'-[(4-{4-[(trifluoromethyl)oxy]phenyl}-1-piperidinyl)methyl]-4-biphenylyl}-4(1H)-pyridinone hydrochloride;
5-chloro-2,6-dimethyl-6'-{4-[(4-{4-[(trifluoromethyl)oxy]phenyl}-1-piperidinyl)methyl] phenyl}-3,3'-bipyridin-4(1H)-one hydrochloride;
5-chloro-2,6-dimethyl-6'-(4-{[4-(trifluoromethyl)-1-piperidinyl]methyl}phenyl)-3,3'-bipyridin-4(1H)-one;
5-chloro-6'-{4-[(4,4-difluoro-1-piperidinyl)methyl]phenyl}-2,6-dimethyl-3,3'-bipyridin-4(1H)-one;
5-chloro-6'-{1,1-difluoro-2-[4-(trifluoromethyl)-1-piperidinyl]ethyl}-2,6-dimethyl-3,3'-bipyridin-4(1H)-one; and
5-chloro-2,6-dimethyl-6'-({2-[4-(trifluoromethyl)-1-piperidinyl]ethyl}oxy)-3,3'-bipyridin-4(1H)-one hydrochloride.

14. A compound which is:
3-Chloro-2,6-dimethyl-5-{4'-[(4-{4-[(trifluoromethyl)oxy]phenyl}-1-piperidinyl)methyl]-4-biphenylyl}-4(1*H*)-pyridinone.

15. A compound or a pharmaceutically acceptable derivative thereof according to any one of claims 1-14 for use in medical therapy.

16. A compound or a pharmaceutically acceptable derivative thereof according to any one of claims 1-14 for use in the treatment of malaria.

17. Use of a compound or a pharmaceutically acceptable derivative thereof according to any one of claims 1-14 in the manufacture of a medicament for the treatment of malaria.

18. A compound according to claim 15 or 16, or use as according to claim 17, wherein malaria is caused by infection with *Plasmodium falciparum.*

19. A method for the treatment of a human or animal subject suffering from malaria comprising administering to said human or animal subject an effective amount of a compound or a pharmaceutically acceptable derivative thereof according to any one of claims 1-14.

20. A method according to claim 19 wherein malaria is caused by infection with *Plasmodium falciparum.*

21. A pharmaceutical composition comprising a compound or a pharmaceutically acceptable derivative thereof according to any one of claims 1-14 and one or more pharmaceutically acceptable carriers and/or excipients.
